# EUROPEAN PATENT APPLICATION

(11) **EP 4 814 007 A1**
(43) Date of publication of application: **30.09.2026**
(21) Application number: 25166668.1
(22) Date of filing: 27.03.2025
(51) Int. Cl.: C07K 14/735, C07K 16/46

(54) **ENGINEERED ANTIBODY CH3 DOMAINS**

(71) Applicant: Merck Patent GmbH, 64293 Darmstadt (DE)
(72) Inventor: WOZNIAK-KNOPP, Gordana, 1180 Vienna (AT); NATALE, Veronica, 1020 Vienna (AT); SIEGMUND, Vanessa, 64293 Darmstadt (DE); PEKAR, Lukas, 64293 Darmstadt (DE); ZIELONKA, Stefan, 64293 Darmstadt (DE); TOLEIKIS, Lars, 67259 Kleinniedesheim (DE); BECKER, Stefan, 64293 Darmstadt (DE)
(74) Representative: Redl, Gerda

(57) **Abstract**

A heterodimeric CH3 assembly of a first and second CH3 domain of the IgG-type, wherein each of the CH3 domains comprises a dimerization sheet, each dimerization sheet being engineered to comprise alternating IgA and IgG segments, wherein the segments of the first CH3 domain are dimerized to the respective segments of the second CH3 domain, wherein either one or both of the CH3 domains is further engineered to incorporate a CD89 binding site.

## Description

### FIELD OF THE INVENTION

The invention relates to engineered CH3 domains which favor a heterodimeric assembly and which confer certain effector functions to binding molecules comprising the CH3 assembly.

### BACKGROUND OF THE INVENTION

Antibody-based therapies that employ cell-mediated attack of tumor cells have proven to be one of the most efficient strategies in recent decades for combating cancer disease. Initially, these methods have relied on activation of natural killer (NK) cells via FcγRIII binding by IgG1-based therapeutics, but the advent of bispecific antibodies has propelled potent T-cell activation as a mode of action into several clinical settings. Consequently, the majority of bispecific antibodies approved for human use today display engagement of T cells and simultaneous binding of a tumor-associated antigen as their main mode of action. Nevertheless, these therapies are limited by the ability of the patient to respond either because of low numbers of T cells in the tumor microenvironment or T-cell exhaustion. At the same time, severe side effects have been reported in many cases, most commonly cytokine release syndrome ranging in symptoms from shivers and mild diarrhoea to systemic inflammation and death, and diverse neurological symptoms.

Neutrophils have long been recognized as a cell group that can be activated by bispecific antibodies to attack cancer cells. In contrast to T cells, they appear in high numbers and are present in the bloodstream as well as in the tissues. As they are a part of the innate immune system, their concentration and activity are less affected by tumor evasion mechanisms as discovered for T cells. Most of the described neutrophil-activating bispecific antibodies mediate the formation of an immunological synapse between the tumor cell and neutrophils via the interaction with the receptor FcαR (CD89), expressed on their surface, and can incite potent tumoricidal activity via different mechanisms, including degranulation, neutrophil extracellular trap release, apoptosis, phagocytosis and trogoptosis. There are no deficiencies described in the expression of CD89, in strong contrast with Fcγ receptors. The potency of the killing activity has been assigned to possible concurrent activation of neutrophils via CD89 and activating Fcγ receptors: combinations of target-specific antibodies of IgA and IgG class mediated more potent killing by polymorphonuclear cells (PMNs), and IgG-type antibodies binding to tumor antigen and CD89 could engage NK cells, macrophages, as well as neutrophils, in the killing of cancer cells. Tandem constructs of such type could cause more potent PMN-mediated killing than either of the parental IgG1 or IgA2 building blocks, used for construction, and exhibited good pharmacokinetic properties in mice (Borrow et al, 2015). In addition to fusions, "cross-isotype" IgG/IgA antibodies were constructed that could react with two activating Fcγ receptors as well as engaged FcαR, and could incite more potent antibody dependent cellular cytotoxicity (ADCC) and antibody dependent cellular phagocytosis (ADCP) than the antibodies of IgG class (Kelton et al, 2014). Generally, it is believed that for potent cellular activation via CD89, cross-linking via bivalent interaction of the receptor and the IgA-Fc is required.

In the last three decades, bispecific antibodies have not only scored as effector-cell groups engaging molecules, but also demonstrated superior selectivity and the ability to overcome redundant signalling pathways, and thus enabled more efficient intervention than antibodies of single specificity by addressing the same targets. As a result, there are many different formats available, and one of most diverse classes are asymmetrical heterodimeric bispecific antibodies, where targeting of two different antigens commonly relies on the two antibody Fab arms and the heterodimerization property of the Ig-CH3 domains is steered towards pairing of complementary Fc-chains.

This has been achieved, for example, with a strand-exchanged-engineered domain (SEED) format, where the CH3 domains are comprised in two heterologous chains named AG and GA, which CH3 domains comprise interchanging amino acid stretches of IgG and IgA (Davis, et al. 2010). Such bispecific antibodies have been efficiently used for targeting cancer cells with enhanced selectivity, shown enhanced activity resulting from biparatopic antigen binding, and were able to activate T cells and NK cells upon the contact with tumor cells (Kelton, et al 2014).

Typically, the paratopes within the SEED antibody are composed of a Fab fragment and a single-chain Fv (scFv) fragment, to avoid possible promiscuous pairing of the light chain, but this issue has been addressed either with exploiting a common light chain or other technologies for preferential cognate heavy and light chain pairing.

Immunoglobulin (Ig) A has raised a lot of interest as therapeutic agent due to its ability to engage cell groups different from traditionally used IgG scaffold and proving potent in tumor eradication. Further, its multimeric forms enable increased flexibility in the design of available paratopes.

Kelton et al (2012) disclose an IgGA antibody which is a "cross-type" engineered human Fc antibody comprising engineered IgG1-CH2 and IgG1-CH3 antibody domains, which displays both IgG-like and IgA-like effector functions.

Heinkel et al (2022) describes a stable heterodimeric IgA for the development of multispecific therapeutics. Variants containing mutations in the IgA CH3:CH3 interface were engineered to drive heterodimerization of the IgA-Fc via steric designs.

### SUMMARY OF THE INVENTION

It is the objective to provide improved antibody Fc domains which favor heterodimerization and provide IgA-like effector functions. It is a further objective to engineer binding molecules, in particular multispecific binders such as bispecific antibodies, which comprise IgA-like effector functions.

The objective is solved by the subject matter as claimed and as further described herein.

According to the invention, there is provided a heterodimeric CH3 assembly of a first and second CH3 domain of the IgG-type, wherein each of the CH3 domains comprises a dimerization sheet, each dimerization sheet being engineered to comprise alternating IgA and IgG segments, wherein the segments of the first CH3 domain are dimerized to the respective segments of the second CH3 domain, wherein either one or both of the CH3 domains is further engineered to incorporate a CD89 binding site.

According to a specific aspect, each (*i.e.,* both) of the first and second CH3 domains is engineered to incorporate the CD89 binding site.

According to another specific aspect, only one of the CH3 domains is engineered to incorporate the CD89 binding site.

Specifically, the dimerization sheets of the CH3 domains are positioned at the assembly interface.

Specifically, the alternating IgA and IgG segments are segments of IgA- and IgG-CH3 domains, preferably IgA and IgG segments originating from about the respective positions or regions of a dimerization sheet of the IgA- and IgG-CH3 domains, respectively.

Specifically, the alternating IgA and IgG segments are intertwined segments of IgA and IgG, preferably respective segments of the dimerization sheet of IgA and IgG CH3 antibodies.

According to a specific aspect, the first and second CH3 domains are strand-exchanged-engineered (SEED) CH3 domains, where the CH3 domains comprise two heterologous chains named AG (as a first CH3 domain) and GA (as a second CH3 domain), which are composed of interchanging amino acid stretches of IgG and IgA (such as described by Davis, et al. 2010).

According to a specific example, both CH3 antibodies are SEED CH3 domains engineered to incorporate the CD89 binding site.

According to another specific example, both CH3 antibodies are SEED CH3 domains, and only one of them is engineered to incorporate the CD89 binding site.

According to a specific aspect, the first and second CH3 domains of the heterodimeric CH3 assembly described herein form heterodimers preferentially over forming homodimers. Specifically, the first and second CH3 domains of the heterodimeric CH3 assembly described herein have an increased affinity to assemble to each other relative to CH3 domains of the same type. In particular, the first CH3 domains preferentially assembles with the second CH3 domains, thereby obtaining heterodimerization. In other words, assembly of CH3 domains comprising the structure (or sequence) of the first CH3 domain occurs only to a lesser extent, and likewise, assembly of CH3 domains comprising the structure (or sequence) of the second CH3 domain occurs only to a lesser extent.

Therefore, it is possible that binding constructs which comprise two polypeptide chains can be heterodimerized by incorporating the heterodimeric CH3 assembly described herein, while reducing homodimerization.

According to a specific aspect, each of the CH3 domains of the CH3 assembly is of the IgG type, which is engineered to obtain a strand-exchange by incorporating at least one or at least two beta strand IgA segments, each of at least 2 amino acids length. Specifically, the strand-exchanged CH3 domains comprised in the CH3 assembly described herein may comprise alternating segments of IgA and IgG amino acid sequences, *e.g.* incorporating at least 1, 2, 3, 4, or 5 different IgA segments, each located at different positions and separated from each other by a non-IgA segment, *e.g.* IgG segments. Specifically, the heterodimeric CH3 assembly described herein comprises a cognate pair of CH3 domains through pairing of at least one IgA segment of the first CH3 domain with at least one corresponding IgA segment of the second CH3 domain.

By engineering the CH3 domains as described herein, production of a heterodimeric CH3 assembly which comprises two different CH3 domains, or a heterodimeric Fc of a heterodimeric antibody heavy chain, which comprises two different Fc chains and antibody heavy chains, respectively, which preferably pair with each other, is possible, while substantially reducing the tendency of producing a respective homodimer, *i.e.* a dimer of the same sequence.

According to a specific aspect, the alternating IgA and IgG segments of the dimerization sheets originate from corresponding segments of naturally-occurring dimerization sheets of respective IgA and IgG CH3 domains. Specifically, the naturally-occurring dimerization sheets are of human CH3 IgA and IgG domains. For example, the alternating IgA and IgG segments are segments of naturally occurring IgA (e.g., IgA1 or IgA2) CH3 domains, and segments of naturally occurring IgG (*e.g.*, IgG3, IgG1 or IgG2) CH3 domains, respectively, in particular wherein the CH3 domains are of human origin.

Specifically, the CH3 domains of the heterodimeric CH3 assembly can be of a mammalian species origin *e.g.*, human, mouse, rabbit, goat, camelid, llama, cow or horse, or of an avian species, *e.g.*, hen.

Specifically, any one or both of the CH3 domains can be wild-type CH3 domains consisting of an amino acid sequence that is naturally-occurring besides the engineered dimerization sheet and the novel CD89 binding site incorporated into the CH3 domain, thereby obtaining an artificial product.

According to a specific aspect, the CH3 domains are of human IgG origin, in particular of IgG3, IgG1, IgG2 or IgG4 antibodies. Specifically, the CH3 domains are of human IgG3, IgG1, IgG2 or IgG4.

According to a specific aspect, the first CH3 domain comprises or consists of the amino acid sequence of any one of SEQ ID NO:1 to 24, and the second CH3 domain comprises or consists of the amino acid sequence of SEQ ID NO:25 to 36.

Specifically, each of SEQ ID NO:1 to 36 comprises point mutations to incorporate a CD89 binding site.

According to a specific aspect, only one of the CH3 domains comprises point mutations to incorporate the CD89 binding site, and the other one is a SEED CH3 domain without such point mutations e.g., a SEED CH3 domain of the IgA1 or IgA2 type.

According to a specific example, the first CH3 domain is a SEED CH3 domain which comprises point mutations to incorporate a CD89 binding site, such as comprising or consisting of any one of SEQ ID NO:1 to 24, and the second CH3 domain is a SEED CH3 domain which is not engineered to comprise the CD89 binding site, such as comprising or consisting of any one of SEQ ID NO:38 or 40.

According to another specific example, the second CH3 domain is a SEED CH3 domain which comprises point mutations to incorporate a CD89 binding site, such as comprising or consisting of any one of SEQ ID NO:25 to 36, and the second CH3 domain is a SEED CH3 domain which is not engineered to comprise the CD89 binding site, such as comprising or consisting of any one of SEQ ID NO:37 or 39.

According to a specific aspect, one or both of the CH3 domains of the heterodimeric CH3 assembly comprise a CD89 binding site. Specifically, the CD89 binding site allows the interaction with CD89. While the SEED CH3 domains without further engineering to incorporate a novel CD89 binding site do not interact with CD89, engineering a number of point mutations in one or both of the first CH3 domain (the AG domain of the SEED CH3) and the second CH3 domain (the GA domain of the SEED CH3) produced CH3 assemblies which when comprised in an Fc or binding molecule (*e.g.*, an antibody) produced respective CD89 binders with respective effector function such as to induce neutrophil-mediated specific target cell killing.

With the mutagenesis strategy described herein, model bispecific SEED antibodies did not lose the ability to recognize its target antigens. As the CD89 interaction is based on a novel binding site in the Fc region, this method is considered applicable with any target specific binders of choice, which comprise the engineered heterodimeric CH3 assembly or respective Fc as described herein.

Specifically, the binding CH3 assembly comprises one or two CD89 binding sites, in particular wherein the first and/or second CH3 domain comprises a CD89 binding site.

According to a specific aspect, the CD89 binding site is a functional binding site as determined in a suitable binding assay, such as *e.g.*, ELISA, surface plasmon resonance, biolayer interferometry or flow cytometry, FACS-based assay, thermal shift assay.

Specifically, said one or two CD89 binding sites of the CH3 assembly described herein are functional in an Fc described herein or a binding construct described herein. In particular, the respective binding construct may have antibody-dependent cytotoxicity (ADCC) using neutrophils as effector cells. In particular, the binding construct which specifically binds to a target on target cells has ADCC activity by neutrophil-mediated specific target cell killing. Specifically, the ADCC is at least 10% or at least 15% of target cell killing compared to untreated control.

Specifically, the CD89 binding site allows an affinity of binding of the respective CH3 domain or CH3 assembly to CD89 (FcαR) with a KD of less than any of 10⁻⁶M, 10⁻⁷M, 10⁻⁸M, 10⁻⁹M, or 10⁻¹⁰M.

Specifically, the CD89 binding site is a novel binding site introduced into a CH3 domain (*e.g.*, a CH3 domain of the IgG type, such as a human IgG CH3, in particular a human IgG3 or IgG1 CH3 domain) by a number of point mutations which is at least 5, 6, 7, 8, 9, 10, 11 or 12 point mutations such as selected from the point mutations further described herein.

In particular, the number of point mutations in the first CH3 domain (AG-CH3 domain) is at least 5, 6, 7, 8, 9, 10, 11, or 12; and the number of point mutations in the second first CH3 domain (GA-CH3 domain) is at least 3, 4, 5, 6, 7 or 8.

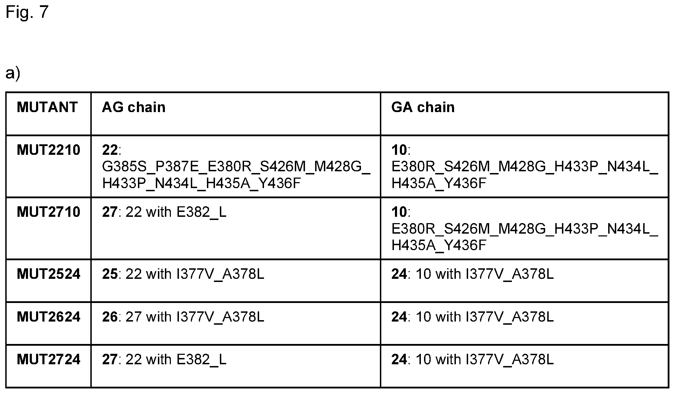

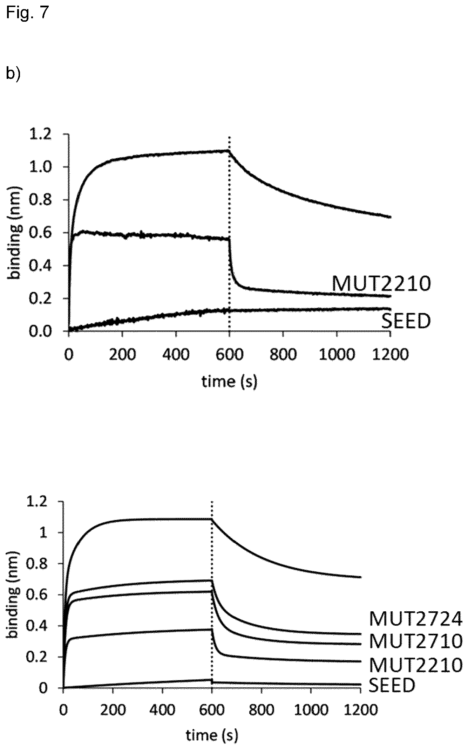

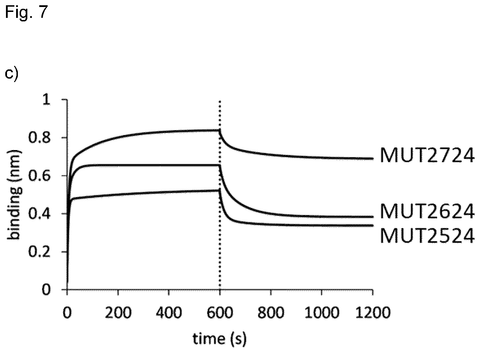

Exemplary point mutations are shown in the Table of Figure 7 a) (numbering according to Davis et al, 2010).

According to a specific aspect, either of the first and second CH3 domains of the heterodimeric CH3 assembly can be independently selected from the IgA1 or IgA2 type, in particular of the SEED-IgA1 or SEED IgA2-Type.

According to a specific aspect, both, the first and second CH3 domains are of the IgA1 type, in particular of the SEED-IgA1 type.

Specifically, the point mutations in the first CH3 domain (AG-CH3 domain) are at least E40R, G45S, P47E, S89M and M91G in the SEED-IgA1 AG CH3 domain (numbering of the SEED-IgA1 AG CH3 domain, SEQ ID NO:37; *i.e.,* E380R, G385S, P387E, S426M and M428G, EU numbering). Optionally, the first CH3 domain further includes at least the four point mutations H96P, N97L, H98A, Y99F (numbering of the SEED-IgA1 AG CH3 domain, SEQ ID NO:37; *i.e.* H433P, N434L, H435A, Y436F EU numbering). These four point mutations introduce the "PLAF" (SEQ ID NO:89) motif which obliterates an FcRn binding site, which could overlap with the CD89 binding site.

According to a specific example, the point mutations in the first CH3 domain are at least E40R, G45S, P47E, S89M, M91G, and optionally E42L (numbering of the SEED-IgA1 AG CH3 domain, SEQ ID NO:37; *i.e.,* E380R, G385S, P387E, S426M, M428G, and optionally E382L, EU numbering).

According to a specific example, the point mutations in the first CH3 domain are at least E40R, G45S, P47E, S89M, M91G, and optionally one or both of I37V and A38L (numbering of the SEED-IgA1 AG CH3 domain, SEQ ID NO:37; *i.e.,* E380R, G385S, P387E, S426M, M428G, and optionally one or both of I377V and A378L, EU numbering).

According to a specific example, the point mutations in the first CH3 domain are at least E40R, G45S, P47E, S89M, M91G, E42L, and one or both of I37V and A38L (numbering of the SEED-IgA1 AG CH3 domain, SEQ ID NO:37; *i.e.,* E380R, G385S, P387E, S426M, M428G, E382L, and optionally one or both of I377V and A378L, EU numbering).

According to a specific aspect, both, the first and second CH3 domains are of the IgA2 type, in particular of the SEED-IgA2 type.

Specifically, the point mutations in the first CH3 domain (AG-CH3 domain) are at least E40R, G45S, P47E, S89M and M91G in the SEED-IgA2 AG CH3 domain (numbering of the SEED-IgA2 AG CH3 domain, SEQ ID NO:39; *i.e.* E380R, G385S, P387E, S426M and M428G, EU numbering). Optionally, the first CH3 domain further includes at least the four point mutations H96P, N97L, H98A, Y99F (numbering of the SEED-IgA2 AG CH3 domain, SEQ ID NO:39; *i.e.* H433P, N434L, H435A, Y436F EU numbering). These four point mutations introduce the "PLAF" (SEQ ID NO:89) motif which obliterates an FcRn binding site, which could overlap with the CD89 binding site.

According to a specific example, the point mutations in the first CH3 domain are at least E40R, G45S, P47E, S89M, M91G, and optionally E42L (numbering of the SEED-IgA2 AG CH3 domain, SEQ ID NO:39; *i.e.,* E380R, G385S, P387E, S426M, M428G, and optionally E382L, EU numbering).

According to a specific example, the point mutations in the first CH3 domain are at least E40R, G45S, P47E, S89M, M91G, and optionally one or both of I37V and A38L (numbering of the SEED-IgA2 AG CH3 domain, SEQ ID NO:39; *i.e.,* E380R, G385S, P387E, S426M, M428G, and optionally E382L, EU numbering).

According to a specific example, the point mutations in the first CH3 domain are at least E40R, G45S, P47E, S89M, M91G, E42L, and one or both of I37V and A38L (numbering of the SEED-IgA2 AG CH3 domain, SEQ ID NO:39; *i.e.,* E380R, G385S, P387E, S426M, M428G, E382L, and optionally one or both of I377V and A378L, EU numbering).Specifically, the point mutations in the second CH3 domain (GA-CH3 domain) are at least E40R, S89M, and M91G in the SEED-IgA1 GA CH3 domain (SEQ ID NO:38; *i.e.,* E380E, S426M, and M428G, EU numbering). Optionally, the second CH3 domain further includes at least the four point mutations H96P, N97L, H98A, Y99F (H433P, N434L, H435A, Y436F, EU numbering). These four point mutations introduce the "PLAF" (SEQ ID NO:89) motif which obliterates an FcRn binding site, which could overlap with the CD89 binding site.

According to a specific example, the point mutations in the second CH3 domain are at least E40R, S89M, and M91G, and optionally one or both of I37V and A38L (numbering of the SEED-IgA1 GA CH3 domain, SEQ ID NO:38; *i.e.,* E380R, S426M, and M428G, and optionally one or both of I377V and A378L, EU numbering).

According to a specific example, the point mutations in the second CH3 domain are at least E40R, S89M, M91G, and both of I37V and A38L (numbering of the SEED-IgA1 GA CH3 domain, SEQ ID NO:38, *i.e.,* E380R, S426M, and M428G, and both of I377V and A378L, EU numbering).

Specifically, the point mutations in the second CH3 domain (GA-CH3 domain) are at least E40R, S89M, and M91G in the SEED-IgA2 GA CH3 domain (numbering of the SEED-IgA2 GA CH3 domain, SEQ ID NO:40; *i.e.,* E380E, S426M, and M428G, EU numbering). Optionally, the second CH3 domain further includes at least the four point mutations H96P, N97L, H98A, Y99F (H433P, N434L, H435A, Y436F, EU numbering). These four point mutations introduce the "PLAF" (SEQ ID NO:89) motif which obliterates an FcRn binding site, which could overlap with the CD89 binding site.

According to a specific example, the point mutations in the second CH3 domain are at least E40R, S89M, and M91G, and optionally one or both of I37V and A38L (numbering of the SEED-IgA2 GA CH3 domain, SEQ ID NO:40; *i.e.,* E380R, S426M, and M428G, and optionally one or both of I377V and A378L, EU numbering).

According to a specific example, the point mutations in the second CH3 domain are at least E40R, S89M, M91G, and both of I37V and A38L (numbering of the SEED-IgA2 GA CH3 domain, SEQ ID NO:40; *i.e.,* E380R, S426M, and M428G, and both of I377V and A378L, EU numbering).

According to a specific aspect,
a) the first CH3 domain comprises or consists of SEQ ID NO:37 or SEQ ID NO:39, which is engineered to comprise the point mutations
   i) E40R, G45S, P47E, S89M, M91G, and
   ii) optionally E42L, and/or one or both of I37V and A38L; and
   iii) optionally H96P, N97L, H98A, Y99F;
      and
b) the second CH3 domain comprises or consists of SEQ ID NO:38 or SEQ ID NO:40, which is engineered to comprise the point mutations
   i) E40R, S89M, M91G, and
   ii) optionally one or both of I37V and A38L; and
   iii) optionally H96P, N97L, H98A, Y99F.

Specifically, the first and second CH3 domains may comprise at least 125 amino acids, such as in SEQ ID NO:1, 3, 5, 7, 9, 11, 13, 15, 17, 19, 21, 23, 25, 27, 29, 31, or 35. Any of such sequences comprises the full-length of a CH3 domain.

Specifically, the first and second CH3 domains may comprise less than 125 amino acids, e.g., at least 99 amino acids, such as in SEQ ID NO:2, 4, 6, 8, 10, 12, 14, 16, 18, 20, 22, 24, 26, 28, 20, 30, 32, 34, or 36. Any of such sequences comprise a truncated CH3 domain, which comprises the PLAF motif (SEQ ID NO:89) motif.

According to a specific aspect, the CH3 domains of the heterodimeric CH3 assembly described herein do not comprise a neonatal Fc receptor (FcRn) binding site. Binding the FcRn receptor could sterically hinder CD89 binding. By the introduction of the PLAF motif as described herein SEQ ID NO:89), binding of CD89 by the CH3 domain can be enhanced.

Specifically, the heterodimeric CH3 assembly may comprise one CH3 domain comprising the PLAF motif such as to introduce one CD89 binding site, and another CH3 domain which does not comprise the PLAF motif, such as to confer or retain FcRn binding. Therefore, the present disclosure also refers to any such AG-CH3 domains and GA-CH3 domains with and without the substitutions H96P, N97L, H98A, and Y99F to introduce the PLAF motif.

According to a specific example, one or both of the CH3 domains of the heterodimeric CH3 assembly may comprise a "PLAF" motif (SEQ ID NO:89).

Specifically, only one of the CH3 domains of the heterodimeric CH3 assembly comprises a "PLAF" motif (SEQ ID NO:89).

Specifically, only one of the CH3 domains of the heterodimeric CH3 assembly comprises a "PLAF" motif (SEQ ID NO:89), and the other one is further engineered to comprise the P96H, L97N, A98H, and F99Y substitutions in the respective sequences SEQ ID NO:1-36, thereby obtaining the respective CH3 domain without the PLAF motif.

Specifically, the present disclosure also refers to any such AG-CH3 domains and GA-CH3 domains of SEQ ID NO: 1-36, which is further engineered to comprise the P96H, L97N, A98H, and F99Y substitutions, thereby obtaining the respective CH3 domain without the PLAF motif.

A preferred first CH3 domain comprises or consists of any one of SEQ ID NO:1-24.

A preferred second CH3 domain comprises or consists of any one of SEQ ID NO: 25-36.

A preferred combination of a first and a second CH3 domain is any one of the following:
a) the first CH3 domain comprises or consists of SEQ ID NO:2; and the second CH3 domain comprises or consists of SEQ ID NO: 26;
b) the first CH3 domain comprises or consists of SEQ ID NO:14; and the second CH3 domain comprises or consists of SEQ ID NO:32;
c) the first CH3 domain comprises or consists of SEQ ID NO:4; and the second CH3 domain comprises or consists of SEQ ID NO: 26;
d) the first CH3 domain comprises or consists of SEQ ID NO:16; and the second CH3 domain comprises or consists of SEQ ID NO: 32;
e) the first CH3 domain comprises or consists of SEQ ID NO:6; and the second CH3 domain comprises or consists of SEQ ID NO: 28;
f) the first CH3 domain comprises or consists of SEQ ID NO:18; and the second CH3 domain comprises or consists of SEQ ID NO: 34;
g) the first CH3 domain comprises or consists of SEQ ID NO:6; and the second CH3 domain comprises or consists of SEQ ID NO: 28;
h) the first CH3 domain comprises or consists of SEQ ID NO:18; and the second CH3 domain comprises or consists of SEQ ID NO: 36;
i) the first CH3 domain comprises or consists of SEQ ID NO:10; and the second CH3 domain comprises or consists of SEQ ID NO: 28;
j) the first CH3 domain comprises or consists of SEQ ID NO:22; and the second CH3 domain comprises or consists of SEQ ID NO: 34;
k) the first CH3 domain comprises or consists of SEQ ID NO:12; and the second CH3 domain comprises or consists of SEQ ID NO: 28;
l) the first CH3 domain comprises or consists of SEQ ID NO:24; and the second CH3 domain comprises or consists of SEQ ID NO: 34;
m) the first CH3 domain comprises or consists of SEQ ID NO:8; and the second CH3 domain comprises or consists of SEQ ID NO: 30;
n) the first CH3 domain comprises or consists of SEQ ID NO:20; and the second CH3 domain comprises or consists of SEQ ID NO: 36;
o) or a respective combination of any one of a) to n) above, wherein the first and/or second CH3 domains comprise or consist of respective truncated CH3 sequences comprising at least the N-terminal 98 amino acids, which truncated CH3 sequences comprise the PLAF motif (SEQ ID NO:89);
p) or a respective combination of any one of a) to o) above, wherein one (in particular, only one) of said first and second CH3 domains of said combination comprises the sequence described herein for said combination, which is further engineered to comprise the P96H, L97N, A98H, and F99Y substitutions.

According to a specific aspect, preferred combinations of a first and a second CH3 domain are exemplified herein. Specifically, a heterodimeric CH3 assembly and respective heterodimeric Fc or heterodimeric antibody heavy chains as described herein may comprise or consist of the combination of sequences described herein or in the Examples section. Further preferred combinations of sequences of heterodimerized Fc or heterodimerized antibody heavy chains are described in the Examples section.

Specifically, the CH3 domains of the heterodimeric CH3 assembly may comprise an amino acid sequence which comprises a naturally occurring (*i.e.,* wild-type) amino acid sequence besides the mutations to engineer the dimerization sheet and the CD89 binding site, or may comprise one or more further mutations such as to modify the CH3 function or to engineer CH3 derivatives.

According to a specific aspect, the CH3 domains of the heterodimeric CH3 assembly described herein may be further modified to enhance characteristics of the CH3 domains or of an Fc or a construct comprising the CH3 domains, such as increased thermostability, effector function, half-life, heterodimerization, *etc.*

Specifically, the CH3 assembly can be further modified by incorporating *de novo* (*i.e.* additional) disulfide bridges, or linkers for drug conjugation.

Specifically, the CH3 assembly can be further modified by incorporating one or more modifications which enhance heterodimer formation of the CH3 domains, such as one or more of the following:
a) one or more knob or hole mutations, preferably wherein the one or more knob or hole mutations is any of:
   T366Y/Y407'T,
   F405A/T394'W,
   T366Y:F405A/T394'W:Y 407'T,
   T366W/Y407'A, or
   S354C:T366W/Y349'C:T366'S:L368'A:Y407'V,
   wherein numbering is according to EU numbering;
b) a cysteine residue in the first CH3 domain that is covalently linked to a cysteine residue in the second CH3 domain, thereby introducing an interdomain disulfide bridge, preferably wherein the cysteine residue in the first CH3 domain that is covalently linked to a cysteine residue in the second CH3 domain links the C-terminus of both CH3 domains;
c) one or more mutations where repulsive charge suppresses homodimer formation, preferably wherein the one or more mutations where repulsive charge suppresses homodimer formation is any of:
   K409D/D399'K,
   K409D/D399'R,
   K409E/D399'K,
   K409E/D399'R,
   K409D:K392D/D399'K:D360'K, or
   K409D:K392D:K30D/D399'K:D360'K:E361'K
   wherein numbering is according to EU numbering;
      and/or
d) one or more mutations selected for heterodimer formation and/or thermostability, preferably wherein the one or more mutations selected for heterodimer formation and/or thermostability is any of:
   T350V:L351Y:F405A:Y407V/T350'V:T366'L:K392'L:T394'W,
   T350V:L351Y:F405A:Y407V/T350'V:T366'L:K392'M:T394'W,
   L351Y:F405A:Y407V/T366'L:K392'M:T394'W,
   F405A:Y407V/T366L:K392'M:T394'W, or
   F405A:Y407V/T336'L:T394'W
   wherein numbering is according to EU numbering.

In the specification of the CH3 point mutations described herein, the "slash" differentiates the point mutations on one chain or one domain from the point mutations from the other chain or other domain of the respective pair (CH3 assembly); the "indent" in the amino acid position numbering signifies the second chain or dimer of the heterodimer. The "colon" identifies the combination of point mutations on one of the chains or domains, respectively.

Functional variants of a CH3 domain described herein are particularly characterized by a certain degree of sequence identity, comparing the amino acid sequence besides the engineered dimerization sheet and the CD89 binding site to the respective wild-type sequence, such as *e.g.* at least 90% or at least any one of 95%, 96%, 97%, 98%, or 99% sequence identity to the respective naturally-occurring CH3 domain sequence. In particular, a functional variant is characterized by the beta-barrel structure of the antibody domain which resembles the structure of respective domains in the respective human IgG, IgA, IgM or IgE structure, in particular a respective human IgA or IgG structure.

Specifically, a functionally active variant of a CH3 domain can be used comprising one or more point mutations in the naturally-occurring sequence besides the engineered dimerization sheet and the CD89 binding site, preferably up to 10 point mutations, in particular any of 1, 2, 3, 4, 5, 6, 7, 8, 9, or 10 point mutations.

The invention further provides for a heterodimeric Fc comprising dimerized Fc chains of CH2 and CH3 domains, wherein the CH3 domains comprise the CH3 assembly described herein. Specifically, the heterodimeric Fc comprises or consists of a dimer of Fc chains. The heterodimeric Fc is specifically characterized by a dimer of Fc chains each characterized by comprising the chain of CH2-CH3 antibody domains, which dimer is a heterodimer, *e.g.* wherein a first Fc chain differs from a second Fc chain in at least the different mutations in the first and second CH3 domains of the heterodimeric CH3 assembly. According to a specific aspect, the CH2 domains can be of any immunoglobulin type, in particular CH2 domains of human immunoglobulins. Specifically, the CH2 domain is of an immunoglobulin of any one of the IgG, IgA, IgM, or IgE isotype, particularly any of an IgG1, IgG2, IgG3, IgG4, IgA1, IgA2, IgD, IgE, or IgM antibody, preferably of a human antibody, such as a human IgG or IgA.

Specifically, the CH2 domain can be of the IgA type, preferably IgA2 or IgA1, of the IgG type, preferably IgG3, IgG1, IgG2, or IgG4, or of the IgE type. Preferably, the CH2 domains comprise or consist of an amino acid sequence independently selected from an amino acid sequence comprising at least 90% sequence identity to any one of SEQ ID NO:43 to 48.

Specifically, the CH2 domain comprises or consists of the amino acid sequence which is any one of SEQ ID NO:43-48, or an amino acid sequence with at least 90%, 95%, 96%, 97%, 98%, or 99% sequence identity to any of SEQ ID NO:43-48.

Specifically, the CH2 domain can be of a mammalian species *e.g.*, human, mouse, rabbit, goat, camelid, llama, cow or horse, or of an avian species, *e.g.*, hen.

Specifically, the CH2 domain can be a wild-type CH2 domain consisting of an amino acid sequence that is naturally-occurring, or a functional variant thereof which is an artificial CH2 domain *e.g.*, a CH2 domain which comprises one or more point mutations such as to modify Fc function or to engineer Fc derivatives.

Functional variants of a CH2 domain are particularly characterized by a certain degree of sequence identity, such as *e.g*. at least 90% or at least any one of 95%, 96%, 97%, 98%, or 99% sequence identity to the naturally-occurring CH2 domain sequence. In particular, a functional variant is characterized by the beta-barrel structure of the antibody domain which resembles the structure of respective domains in the respective human IgG, IgA, IgM or IgE structure, in particular a respective human IgA or IgG structure.

Specifically, a functionally active variant of a CH2 domain can be used comprising one or more point mutations in the naturally-occurring sequence, preferably up to 10 point mutations, in particular any of 1, 2, 3, 4, 5, 6, 7, 8, 9, or 10 point mutations.

Specifically, in an Fc chain described herein, the C-terminus of the CH2 domain is fused to the N-terminus of a CH3 domain of the heterodimeric CH3 assembly described herein.

Specifically, the heterodimeric Fc comprises or consists of heterodimeric antibody Fc heavy chains, wherein a first Fc chain comprises the first CH3 domain (AG-CH3 domain) described herein, and the Fc second chain comprises the second CH3 domain (GA-CH3 domain) described herein.

According to a specific aspect, either of the first and second Fc chains of the heterodimeric Fc can be independently selected from the IgA1 or IgA2 type, in particular of the SEED-IgA1 or SEED IgA2-type.

Specifically, both, the first and second Fc chains of the heterodimeric Fc are of the IgA1 type, in particular of the SEED-IgA1 type.

Specifically, both, the first and second Fc chains of the heterodimeric Fc are of the IgA2 type, in particular of the SEED-IgA1 type.

According to specific examples, the first Fc chain of the heterodimerized Fc comprises or consists of any one of SEQ ID NO:49-53.

According to specific examples, the second Fc chain of the heterodimerized Fc comprises or consists of any one of SEQ ID NO:54-56.

A preferred combination of a first and a second Fc chain of a heterodimeric Fc described herein is any one of the following:
a) the first Fc chain comprises or consists of SEQ ID NO:49; and the second Fc chain comprises or consists of SEQ ID NO: 54;
b) the first Fc chain comprises or consists of SEQ ID NO:49; and the second Fc chain comprises or consists of SEQ ID NO: 55;
c) the first Fc chain comprises or consists of SEQ ID NO:50; and the second Fc chain comprises or consists of SEQ ID NO: 54;
d) the first Fc chain comprises or consists of SEQ ID NO:52; and the second Fc chain comprises or consists of SEQ ID NO: 54;
e) the first Fc chain comprises or consists of SEQ ID NO:51; and the second Fc chain comprises or consists of SEQ ID NO: 55.

Specifically, the heterodimeric Fc comprises the structure of an Fc part of an antibody (herein referred to as "antibody Fc" or "Fc"), which is composed of two CH2 domains and two CH3 domains, wherein a first chain of a CH2 domain fused to a first CH3 domain of the heterodimeric CH3 assembly is forming a dimer with a second chain of a CH2 domain fused to a second CH3 domain heterodimeric CH3 assembly. The CH2 domains of the two Fc chains can be identical, but can also differ from each other. The CH2 domains of the Fc chains may be any naturally-occurring human CH2 domain, or a CH2 domain comprising or consisting of the amino acid sequence which is any one of SEQ ID NO:43-48, or an amino acid sequence with at least 90%, 95%, 96%, 97%, 98%, or 99% sequence identity to any of SEQ ID NO:43-48.

Specifically, the heterodimeric Fc may comprise a functional variant of an Fc region, such as a functional variant comprising the CH3 domains of the heterodimeric CH3 assembly as described herein, and a certain degree of sequence identity in the CH2 domain, such as e.g. at least 90% or at least 95%, 96%, 97%, 98% or 99%, to the naturally-occurring sequence. Specifically, the Fc region is characterized by the beta-barrel structure of the CH2 and CH3 antibody domains which resembles the structure of respective domains in the human IgG, IgA, IgM or IgE structure, in particular a human IgG1 structure.

Specifically, a functionally active variant of an Fc region can be used comprising one or more point mutations in the naturally-occurring sequence in one or both of the CH2 and CH3 domains comprised in the Fc region, preferably up to 10 point mutations, in particular any of 1, 2, 3, 4, 5, 6, 7, 8, 9, or 10 point mutations in one or both of the antibody domains, CH2 and CH3, preferably only in the CH2 domain.

The heterodimeric Fc described herein may or may not comprise one or more (in particular two) linkers or hinge regions (also referred to herein as "hinge").

Specifically, the Fc chains may further comprise a linker and/or hinge region, in particular wherein the linker and/or hinge extends the CH2 domain sequence at the N-terminus. The linkers or hinge regions as used in the dimer of the Fc chains can be identical or differ from each other. By a linker or hinge region, one or more further antibody domains or a binding moiety can be fused or bound to the Fc.

Binding of any such one or more further antibody domains or a binding moiety can be by recombinant fusion or chemical linkage. Specifically, the binding may be through linking the C-terminus of an antibody domain to the N-terminus of the CH2 domain.

When binding antibody domains to each other, one or more amino acid residues in the terminal regions can be deleted to shorten the domain size, or extended to increase flexibility of the domains.

Specifically, a shortened (or truncated) domain sequence can be used, optionally wherein the domain sequence comprises a deletion of the respective C-terminal and/or N-terminal region, such as to delete at least 1, 2, 3, 4, or 5, up to 6, 7, 8, 9, or 10 amino acids.

Specifically, a linking sequence may be used, such as a linker or a hinge region or at least part of the hinge region of an immunoglobulin e.g., a peptidic linker composed of an amino acid sequence *e.g.*, including at least 1, 2, 3, 4, or 5 amino acids, up to 10, 15, or 20 amino acids. A linking sequence is herein also referred to as "junction".

A domain may be extended by a linker *e.g.* through an amino acid sequence that originates from the N-, or C- terminal region of an antibody domain that would natively be positioned adjacent to the domain, such as to include the native junction between the domains. Alternatively, the linker may contain an amino acid sequence originating from the hinge region. However, the linker may as well be an artificial sequence, *e.g.* consisting of serial Gly and/or Ser amino acids, preferably with a length of 5 to 20 amino acids, preferably 8 to 15 amino acids.

Specifically, the hinge region can be any peptidic hinge region composed of an amino acid sequence, which is a hinge region of a naturally-occurring immunoglobulin. Specifically, the hinge region can be of a human immunoglobulin. According to specific examples, hinge regions of (or originating from) an IgG, IgA, IgM or IgE antibody can be used *e.g.*, of (or originating from) an IgG1, IgA1 or IgA2 antibody.

For example, the hinge region may comprise or consist of SEQ ID NO:57 or 58.

Where both Fc chains comprise a hinge region. it is preferable that the two hinge regions are connected to each other by one or more disulfide bonds between cysteine residues of the amino acid sequence of the hinge regions.

According to a specific aspect, the heterodimeric Fc comprises the novel CD89 (FcαR) binding site incorporated into one or both of the CH3 domains of the heterodimeric CH3 assembly described herein, and optionally one or more further Fc receptor binding sites (in particular human Fc receptor binding sites), such as to bind FcRn, FcγR (*e.g.*, FcγRI or FcγRII or FcγRIII), FcεR, in particular FcγRI (CD64), FcγRIIA (CD32), FcγRIIB1 (CD32), FcγRIIB2 (CD32), FcγRIIIA (CD16a), FcγRIIIB (CD16b), FcεRI, FcεRII (CD23), Fcα/µR (CD351), or Fcµ, or a complement (or complement component) binding site, such as to bind C1q (CD16a), in particular human C1q.

Specifically, the Fc provides for a respective effector-function mediated by FcαR and the respective other Fc receptor binding and optionally mediated by the C1q binding.

Specifically, the Fc receptor binding sites recognize the respective human receptors and optionally the human C1q.

Specifically, the heterodimeric Fc comprises the novel CD89 (FcαR) binding site and may additionally comprise an FcRn.

Specifically, the heterodimeric Fc comprises the novel CD89 (FcαR) binding site and may additionally comprise an FcRn and an FcγR binding site.

Specifically, the heterodimeric Fc comprises the novel CD89 (FcαR) binding site and may additionally comprise an FcRn, an FcγR binding site and/or a C1q binding site.

According to a specific aspect, the binding construct further described herein, which comprises the heterodimeric CH3 assembly or the heterodimeric Fc described herein, has antibody-dependent cytotoxicity (ADCC) using neutrophils as effector cells. In particular, the binding construct which specifically binds to a target on target has ADCC activity by neutrophil-mediated specific target cell killing.

Specifically, the ADCC using neutrophils as effector cells is mediated by the heterodimeric CH3 assembly and the heterodimeric Fc described herein, respectively. In particular, the ADCC effector function (using neutrophils as effector cells) is mediated by the novel CD89 binding site comprised in the heterodimeric CH3 assembly. ADCC mediated by CD89 binding can be determined by an ADCC assay using neutrophils as effector cells.

Specifically, the heterodimeric Fc described herein comprises cell-mediated cytotoxic effector functions such as an antibody dependent cellular cytotoxicity (ADCC) which is an FcαR mediated effector function, which requires binding neutrophils (as effector cells) to the FcαR (CD89).

According to a specific aspect, the heterodimeric Fc described herein may also comprise an FcγR binding site such as a FcγRI or FcγRII or FcγRIII binding site. ADCC and ADCP effector functions are typically FcγR mediated effector functions, which require binding innate effector cells to the FcγR. The heterodimeric Fc described herein may also comprise a C1q (CD16a) binding site to mediate complement-dependent cytotoxicity (CDC). CDC is mediated by binding the Fc to C1q (CD16a).

According to a specific aspect, the heterodimeric Fc described herein comprises cell-mediated cytotoxic effector functions, such as ADCC (FcαR-mediated ADCC, and optionally in addition FcγR-mediated ADCC), and optionally CDC. In addition, the Fc may also comprise FcγR-mediated antibody dependent cellular phagocytosis (ADCP) effector function.

Yet, according to a specific aspect, the heterodimeric Fc comprises the novel CD89 binding site, and no additional FcγR binding site and/or no C1q (CD16a) binding site. Such Fc provides for only the effector-function mediated by CD89 binding. Such Fc is considered deficient in binding to an FcγR and C1q (CD16a).

According to a specific aspect, the heterodimeric Fc described herein, when expressed in a mammalian expression system, is glycosylated. Specifically, the Fc comprises N-fucosylated glycans.

The invention further provides for a binding construct comprising one or more binding moieties and heterodimeric Fc described herein. Specifically, the binding construct is a heterodimeric binding construct.

Specifically, the binding construct comprises at least one hinge or linker, which links a binding moiety to the N-terminus of an Fc chain. The binding moiety can be bound to the Fc chain directly or indirectly *e.g.*, with one or more elements between the binding moiety and the Fc chain, such as one or more antibody domains (such as one or more constant domains), linkers and/or hinges. Preferably, the binding construct comprises at least one hinge which originates from an IgA or IgG antibody.

Specifically, the binding moiety is fused (or otherwise bound) to the heterodimeric Fc, in particular to the N-terminus of a CH2 domain of an Fc chain, or to a linker or hinge region linking the binding moiety to the CH2 domain.

According to a specific aspect, the binding construct comprises one binding moiety bound to the heterodimeric Fc.

According to a specific example, the binding construct comprises only one binding moiety, which is bound to either a first Fc chain that comprises the first CH3 domain of the heterodimeric CH3 assembly described herein, or to a second Fc chain that comprises the second CH3 domain of the heterodimeric CH3 assembly.

According to another specific aspect, the binding construct comprises more than one binding moiety bound to the heterodimeric Fc, in particular at least two binding moieties. Specifically, the binding construct may comprise at least two binding moieties with different binding specificities.

According to a preferred aspect, the binding construct comprises two binding moieties, e.g., two binding moieties with different binding specificities. Specifically, a first binding moiety can be bound to a one Fc chain, and a second binding moiety can be bound to the other Fc chain. Specifically, a first binding moiety can be bound to a first Fc chain that comprises the first CH3 domain of the heterodimeric CH3 assembly described herein, and a second binding moiety can be bound to a second Fc chain that comprises the second CH3 domain of the heterodimeric CH3 assembly.

According to a specific aspect, the binding construct comprises at least one binding moiety which comprises or consists of any one of an antigen-binding moiety, an enzyme, a substrate, a cytokine, a cytokine-binding moiety, a receptor, or a ligand.

According to specific examples,
a) the antigen-binding moiety comprises an antibody variable region; the antibody variable region may comprise or consist of one or more antibody domains *e.g.*, including at least one antibody variable domains, preferably any one or more of a VL domain, a VH domain, a VHH domain, Fv, scFv, diabody, Fab, a scFab, Fab', Fab₂, Fab₃, F(ab')₂; sdAb, diabody, triabody, tetrabody, minibody, nanobody, maxibody, tandab, DVD, BiTe, TandAb, or a combination of any of the foregoing, preferably a Fab, scFab, F(ab')₂, scFv, Fd, Fv, VH or VHH.

Specifically, the cytokine is any one of IL-2, IL-12, or II-18.

Specifically, the cytokine binding moiety is a cytokine receptor domain.

According to a specific aspect, the binding construct comprises at least one binding moiety which comprises or consists of an antigen-binding portion (or fragment) of an antibody, or the binding site of any one of an enzyme, an adhesion protein, a ligand or a ligand binding portion of a receptor, which binding site is capable of binding a cognate structure of a binding partner. Specifically, the binding moiety can be composed of the binding site of a naturally occurring receptor.

According to a specific aspect, the binding construct comprises at least one binding moiety which comprises or consists of an antigen-binding ("also referred to as "target-binding" or "target antigen-binding") moiety which comprises one or more antibody variable domains, in particular a VH and a VL domain, which associate to form a VH/VL binding site involving or composed of three VH-CDR regions and three VL-CDR regions.

Specifically, a binding construct can be an antibody or an antibody fragment e.g., a full-length antibody, such as a multispecific antibody or a multispecific antigen-binding fragment of an antibody.

According to a specific example, the binding construct can be a full-length antibody e.g., comprising the structure of any one of an IgG, IgA, IgM, or IgE antibody, wherein the naturally-occurring Fc is exchanged for a heterodimeric Fc described herein, or wherein the naturally-occurring CH3 assembly is exchanged for a heterodimeric CH3 assembly described herein.

According to a specific aspect, the binding construct may comprise two, three, or more binding moieties. Depending on the number of binding moieties, the binding construct can monovalently bind to a specific target (*i.e.,* where the binding construct comprises only one binding moiety specifically recognizing one target), or multivalently bind to a specific target (*i.e.,* where the binding construct comprises at least two binding moieties specifically recognizing the same one target).

According to a specific aspect, the binding construct comprises at least two binding moieties with different target binding specificities. Such binding construct comprising at least two different target binding specificities is herein also referred to as "multispecific". A binding construct comprising two different target binding specificities is herein also referred to as "bispecific".

Specifically, the binding construct may be a multispecific binding construct which comprises two, three, or more binding moieties, comprising target specificities to specifically recognize at least two, three or more different targets.

According to a specific aspect, the binding construct is a multispecific binding construct, comprising at least two binding moieties specifically recognizing two or more different targets. Such binding construct may be a monovalent or multivalent (*i.e.* where the binding is by at least two valencies such as bivalent binding) binder of at least two different targets.

Specifically, the binding construct can be a full-length monovalent or multivalent or bispecific antibody which comprises at least two antigen-binding moieties such as further described herein.

According to a specific example, the binding construct is a bispecific binding construct, comprising only two binding moieties specifically recognizing two different targets. Such binding construct may be a monovalent binder of two different targets. For example, such binding construct can be a full-length antibody.

According to another specific example, the binding construct is a monospecific binding construct, comprising one binding moiety specifically recognizing only one target. Such binding construct may bind a monovalent binder of the only one target. For example, such binding construct can be a one-armed antibody.

According to a specific aspect, the binding construct is a multispecific antibody, preferably a bispecific, trispecific, or tetraspecific antibody), or a one-armed antibody.

Specifically, the binding construct can be a multispecific antibody such as a bispecific antibody, specifically recognizing two or more different antigens, wherein a specific antigen is recognized by one, two or more antigen-binding moieties. Specifically, the binding construct can be bivalent or multivalent, wherein an antigen is specifically recognized by two or more antigen-binding moieties, respectively.

Specifically, the binding construct is a heterodimeric and bispecific antibody targeting two different antigens or two different epitopes of an antigen.

Specifically, the binding construct can be cross-reactive, wherein two or more targets (or antigens) are specifically recognized by one cross-specific binding site.

According to a specific aspect, the binding construct described herein is a heterodimeric antibody comprising two different heavy chains (HC), each comprising a CH2 and a CH3 domain, and optionally a CH4 domain, which HCs dimerize into a heterodimeric Fc as described herein.

According to a specific aspect, the binding construct can be an antibody or an antigen-binding fragment thereof *e.g.*, a monoclonal antibody or an antigen-binding fragment thereof.

Specifically, the binding construct can be a monoclonal antibody. Specifically, a preparation of a monoclonal antibody is provided which is obtained by cultivating a cell line of host cell that is engineered by recombinant techniques to express monoclonal antibodies.

Specifically, the binding construct is a human, humanized or chimeric antibody.

Specifically, the binding construct is an antibody (*e.g.*, a human, humanized or chimeric antibody), in particular a human IgG or IgA antibody, wherein the antibody is modified to incorporate the heterodimeric Fc described herein, or the heterodimeric CH3 assembly described herein, and optionally further engineered to incorporate at least two antigen-binding moieties such as described herein, preferably wherein the antigen-binding moieties comprise target specificities to specifically recognize at least two different targets.

According to a specific aspect, the binding moiety may be of an antibody or antigen-binding fragment thereof. Specifically, the binding moiety comprises a binding site of a full-length antibody or heavy-chain only antibody, which may *e.g.*, comprise a binding moiety composed of a single antibody variable domain (such as a single heavy chain variable domain *e.g.*, a VH or VHH), or a single chain of antibody domains which single chain comprises at least one antibody variable domain. Specifically, the binding moiety comprises one or more antibody domains which comprise at least one antibody binding site in a CDR. Specifically, the antigen-binding site can be comprised in 6 CDRs (as in an Fv), or in 3 CDRs (as in a single variable domain).

Specifically, the binding moiety comprises one or more antibody domains such as at least one antibody variable domain *e.g.*, a Fab, Fab', Fab'₂, Fab₂, Fab₃, F(ab')₂, Fd, Fv, sdAb, scFv, diabody, triabody, tetrabody, minibody, nanobody, maxibody, tandab, DVD, BiTe, TandAb, VH, VL, VHH, or a combination of any of the foregoing.

The binding moiety is preferably selected from the group consisting of a Fab, scFab, F(ab')₂, scFv, Fd, Fv, VH or VHH.

According to a specific aspect, the binding moiety used in the binding construct described herein is a Fab (also referred to as "Fab arm"), which is a dimer of a heavy chain (HC) consisting of a VH-CH1 domain sequence and a light chain (LC) consisting of a VL-CL (kappa or lambda) domain sequence, with or without any disulfide bridges, a hinge domain and/or linker sequences connecting antibody domains. A Fab arm is typically understood as a Fab fragment (or Fab part) when cleaved from an antibody. The Fab arm is specifically characterized by only one antigen-binding site formed by pairing the VH and VL domains, and is capable of binding the target only by monospecific and monovalent binding.

Specifically, the binding construct described herein comprises two Fv binding moieties within the same or different formats. An Fv is herein understood as an assembly of a VH and a VL which VH/VL assembly forms an antigen-binding site by the respective CDRs of the VH and VL.

In particular, the binding construct may comprise at least two Fvs in the form of a Fab, Fab', Fab'₂, Fab₂, Fab₃, F(ab')₂, Fd, Fv, sdAb, scFv, or diabody, wherein said at least two Fvs may be of the same or different format.

According to a specific example, the binding construct comprises two Fvs in the form of a Fab, or two Fvs in the form of an scFv.

According to a specific example, the binding construct described herein comprises two Fab arms, thereby providing two Fv structures, each with specific binding characteristics.

According to another specific example, the binding construct described herein comprises two different Fab arms, thereby providing two different Fv structures, wherein the two Fvs specifically recognize different targets, in particular different antigens or different epitopes of an antigen.

According to another specific example, the binding construct comprises one Fv in the form of a Fab, and another Fv in the form of an scFv.

According to a specific example, the binding construct described herein comprises one Fab arm and one scFv, thereby providing two Fv structures, each with specific binding characteristics.

According to a specific example, the binding construct described herein comprises one Fab arm and one scFv, thereby providing two Fv structures, wherein the two Fvs specifically recognize different targets, in particular different antigens or different epitopes of an antigen.

Specifically, the binding construct is a heterodimeric and bispecific antibody comprising a first and a second Fab arm recognizing different antigens or epitopes, such as a bispecific full-length antibody.

According to a specific aspect, the binding construct specifically recognizes a target expressed on the surface of a target cell, in particular through one or more binding moieties. Such targets (herein also referred to as "target antigen" or "surface antigens") are specifically on the surface of target cells e.g., mammalian cells, in particular human cells, which are targeted to react with the binding construct upon binding to the target.

Specifically, a target antigen is selected from cell surface antigens, including receptors, in particular from the group consisting of erbB receptor tyrosine kinases (such as EGFR, HER2 including Her2neu, HER3 and HER4). In addition further antigens may be targeted, e.g., molecules of the TNF-receptor superfamily, such as Apo-1 receptor, TNFR1, TNFR2, nerve growth factor receptor NGFR, CD40, CD40-Ligand, OX40, TACI, BCMA, BAFF-receptor, T-cell surface molecules, T-cell receptors, T-cell antigen, Apo-3, DR4, DR5, DR6, decoy receptors ,such as DcR1, DcR2, CAR1, HVEM, GITR, ZTNFR-5, NTR-1, TNFL1, IGFR-1, c-Met, but not limited to these molecules, B-cell surface antigens, such as CD10, CD19, CD20, CD21, CD22, DC-SIGN, antigens or markers of solid tumors or hematologic cancer cells, cells of lymphoma or leukaemia, other blood cells including blood platelets, but not limited to these molecules.

According to specific examples, the surface antigens are selected from the group consisting of receptor tyrosine kinases (ErbB family).

Specifically, the binding construct is internalizing upon binding to a target cell. According to specific examples, the internalizing binding construct specifically recognize antigens selected from the group consisting of receptor tyrosine kinases (ErbB family). Internalization of the binding construct upon binding to the target cell can be determined by standard techniques, including e.g., flow cytometry, radiolabel studies, image analysis, or cytotoxic assays.

Specifically, the binding construct comprises a functional antigen-binding site such as comprised in one or more variable antibody domains, or an Fv, which functional antigen-binding site is capable of binding a target with a high affinity e.g., with a KD of less than any of 10⁻⁶M, 10⁻⁷M, 10⁻⁸M, 10⁻⁹M, or 10⁻¹⁰M.

Specifically, the binding construct is a bispecific and heterodimeric antibody targeting two different antigens (or epitopes of an antigen), wherein each of the antigens (or epitopes) is recognized by the antibody with a KD of less than any of 10⁻⁶M, 10⁻⁷M, 10⁻⁸M, 10⁻⁹M, or 10⁻¹⁰M.

Specifically, the binding construct is a monospecific or bispecific antibody targeting at least EGFR.

Specifically, the binding construct is a bispecific or multispecific antibody, wherein a first target is any of CD3, CD16 or Her2neu, and a second target is EGFR. Preferred targets of a monospecific or multispecific antibody are selected from the group consisting of CD3, CD16, Her2neu, EGFR, CD28, CD2, NKp30, NKp45, MICA, CD137, cMet, ROR1, DLL3, PD-1, PD-L1, MUC-1, GD2, VEGF, and TROP-2.

The invention further provides for one or more nucleic acid molecules encoding the heterodimeric CH3 assembly described herein, or the heterodimeric Fc described herein, or the binding construct described herein. Specifically, the nucleic acid molecules are isolated nucleic acid molecules.

Specifically, a first nucleic acid molecule is provided which encodes the first CH3 domain of the heterodimeric CH3 assembly, or which encodes the Fc chain comprising the first CH3 domain of the heterodimeric CH3 assembly, or which encodes the respective chain of the binding construct which comprises such Fc chain.

Specifically, a second nucleic acid molecule is provided which encodes the second CH3 domain of the heterodimeric CH3 assembly, or which encodes the Fc chain comprising the second CH3 domain of the heterodimeric CH3 assembly, or which encodes the respective chain of the binding construct which comprises such Fc chain.

Specifically, a nucleic acid molecule or a set of nucleic acid molecules is provided, comprising both, the first and the second nucleic acid molecules described herein.

The invention further provides for an expression system comprising one or more of the nucleic acid molecules described herein. One or more coding nucleic acid molecules may be used in an expression system, which may include one or more expression cassettes such as comprised in one or more expression vectors.

Specifically, an expression cassette is incorporated in a vector (or plasmid) comprising or incorporating a nucleic acid described herein, which expression cassette optionally comprises further sequences to express the nucleic acid sequence, such as regulatory sequences.

The invention further provides for a host cell comprising said one or more nucleic acid molecules described herein or the expression system described herein. Specifically, the host cell is a production host cell comprising at least one expression cassette or a vector (or plasmid) comprising or incorporating one or more nucleic acid molecules described herein.

Specifically, the host cell transiently or stably expresses the proteins or polypeptides or respective polypeptide chains, which are encoded by said one or more nucleic acid molecules described herein.

According to specific examples, the host cell is a eukaryotic host cell, preferably any of mammalian cells or yeast.

The invention further provides for a method of producing a binding construct described herein, wherein a host cell described herein is cultured or maintained under conditions to produce said binding construct.

Specifically, the binding construct may be isolated and/or purified from the cell culture supernatant.

According to a specific example, the binding construct is a bispecific full-length antibody which is heterodimeric comprising two different HCs and two different LCs, and the binding construct comprises a correct pairing of the cognate HC/LC pairs. Specifically, the binding construct is produced by the host cell, wherein less than 10% of the antibodies produced are incorrectly paired, preferably less than 5%, as measured by mass spectrometry (LC-ESI-MS) comparing maximum peak intensity.

Specifically, the binding construct described herein is provided for medical, diagnostic or analytical use.

Specifically, the binding construct described herein is provided for use in the treatment of cancer, autoimmune disease or allergy, targeting at least one antigen which is relevant to the disease. Therefore, the invention further refers to a method for treating a subject suffering from cancer, autoimmune disease or allergy, by administering an effective amount of the binding construct described herein, wherein the binding construct is targeting at least one antigen or at least two antigens, which targeting is relevant to treatment of the disease.

Specifically, the target is a cancer-associated antigen, and the disease is a tumor disease or cancer.

Specifically, the disease is a tumor disease or cancer.

Specifically, the cancer is selected from the group consisting of breast cancer, prostate cancer, ovarian cancer, cervical cancer, skin cancer, pancreatic cancer, colorectal cancer, metastatic colorectal cancer (mCRC), non-resectable liver metastases, Squamous Cell Carcinoma of the Head and Neck, Non-Small Cell Lung Cancer (NSCLC), and Head and Neck Squamous Cell Carcinoma (HNSCC).

The invention further provides for a pharmaceutical preparation comprising the binding construct described herein, preferably in a parenteral or mucosal formulation, optionally containing a pharmaceutically acceptable carrier or excipient.

Specifically, the binding construct described herein is provided in a pharmaceutical preparation comprising a pharmaceutically acceptable carrier or excipient in a parenteral formulation.

Unless indicated otherwise, the positions are herein numbered according to the IMGT system (Lefranc et al., 1999, Nucleic Acids Res. 27: 209-212). Yet, in the Examples section, the numbering according to the EU index (herein referred to as "EU numbering") (Edelman, G.M. et al., Proc. Natl. Acad. USA, 63, 78-85 (1969)). The table of Figure 7a shows point mutations within a CH3 domain using the EU numbering.

### FIGURES

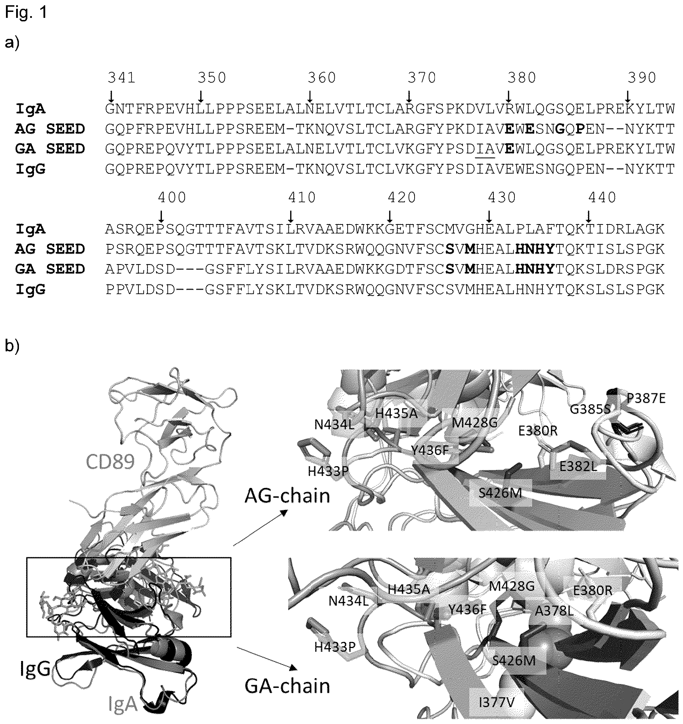

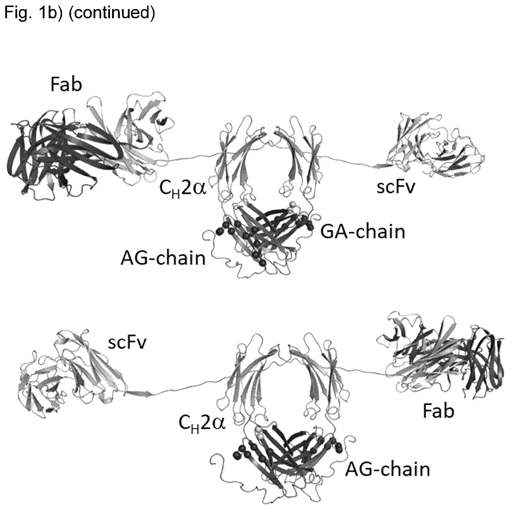

Figure 1: Molecular features of bispecific SEED-based antibodies with effector functions. (a). Sequence alignments of human CH3 domains of IgA1, IgG, SEED-AG chain and SEED-GA chain. Amino acid residues that were subject to modification to mediate CD89 binding are in bold and those altered to influence the thermostability are underlined.
   Sequences shown in the table of Fig. 1 a)
      SEQ ID NO:91: IgA: 
GNTFRPEVHLLPPPSEELALNELVTLTCLARGFSPKDVLVRWLQGSQELPREKYLTW
ASRQEPSQGTTTFAVTSILRVAAEDWKKGETFSCMVGHEALPLAFTQKTIDRLAGK
      SEQ ID NO:92: AG SEED 
GQPFRPEVHLLPPSREEMTKNQVSLTCLARGFYPKDIAVEWESNGQPENNYKTTPS
RQEPSQGTTTFAVTSKLTVDKSRWQQGNVFSCSVMHEALHNHYTQKTISLSPGK
      SEQ ID NO:93: GA SEED 
GQPREPQVYTLPPPSEELALNELVTLTCLVKGFYPSDIAVEWLQGSQELPREKYLTW
APVLDSDGSFFLYSILRVAAEDWKKGDTFSCSVMHEALHNHYTQKSLDRSPGK
      SEQ ID NO:94: IgG 
GQPREPQVYTLPPSREEMTKNQVSLTCLVKGFYPSDIAVEWESNGQPENNYKTTPP
VLDSDGSFFLYSKLTVDKSRWQQGNVFSCSVMHEALHNHYTQKSLSLSPGK
   (EU numbering)
   (b). Overlay of IgA1-Fc (dark gray) with bound CD89 (pale gray) (PDB:1OW0) and IgG-Fc (black) (PDB:1OQO, Herr et al. 2003, Nature 423:614-20) (left), with altered residues in the C_{H}3 domain highlighted (center), and cartoon diagram of mutated SEED construct (right) (PDB: 1IGA, Amore et al. 2001, J Am Soc Nephrol 12:1862-71). The Figure was prepared with PyMOL Molecular Graphics System, Version 2.4.0 Schrödinger, LLC, using PDB: 1IGA, and scFv was modeled with SWISS _MODEL.

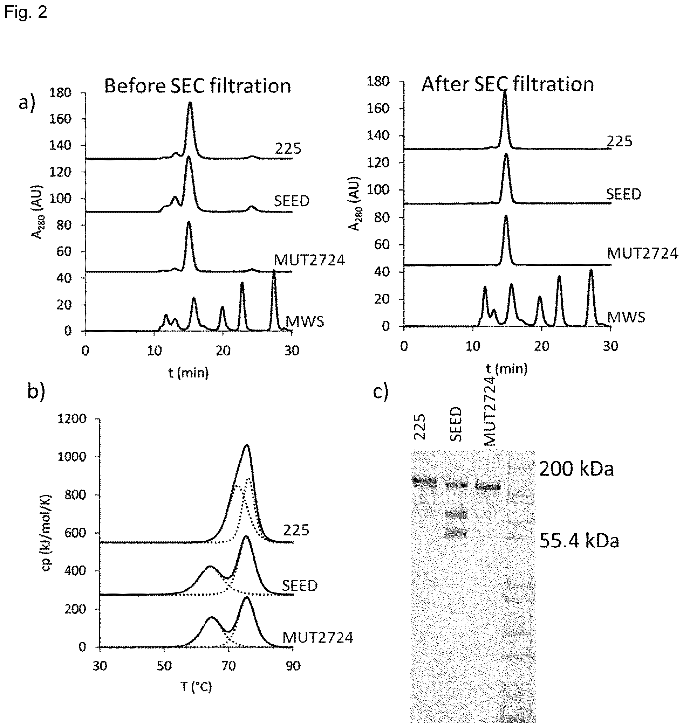

Figure 2: Biophysical characterization of IgA- and SEED-based constructs. (a) Analysis with size exclusion chromatography in native conditions before (left) and after (right) gel filtration (MWS: molecular weight standard). (b) Thermostability analysis using differential scanning calorimetry. (c) SDS-PAGE analysis (M: Mark 12 unstained marker).

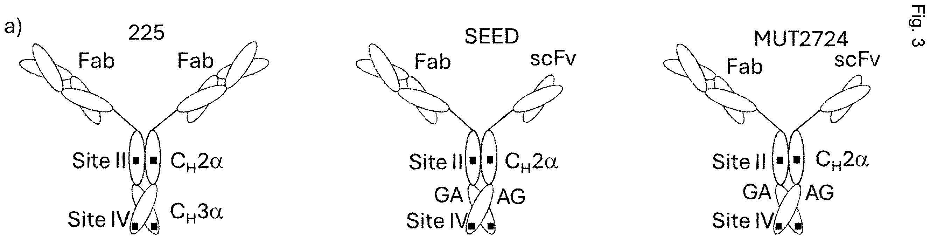

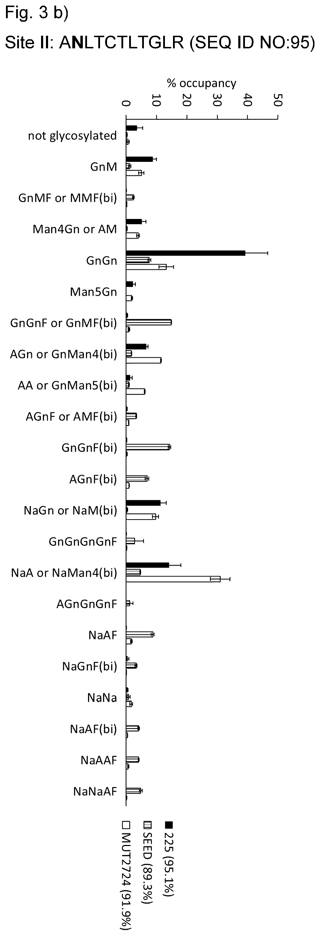

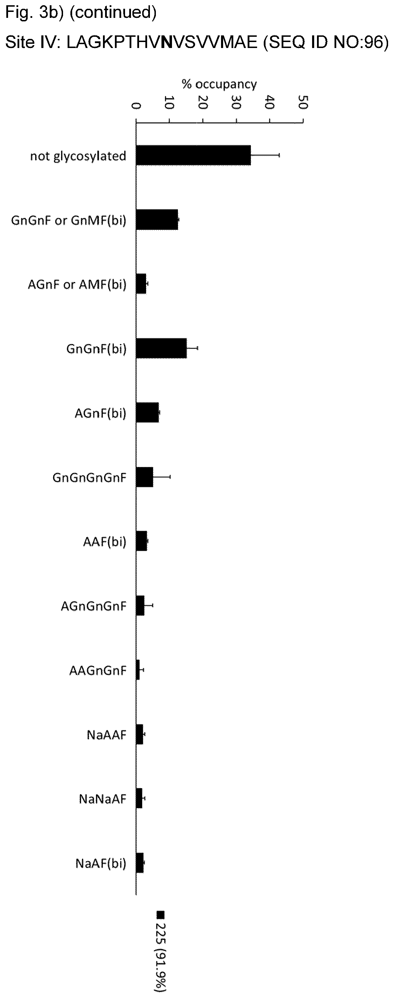

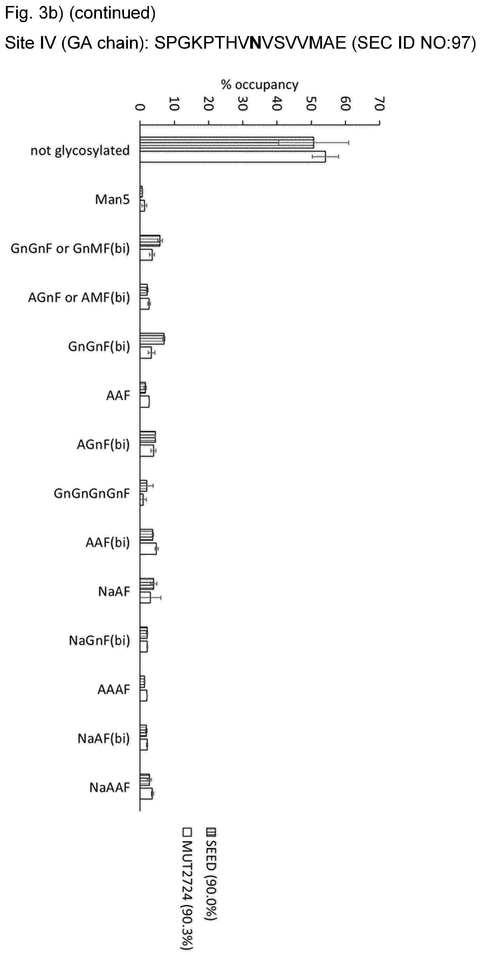

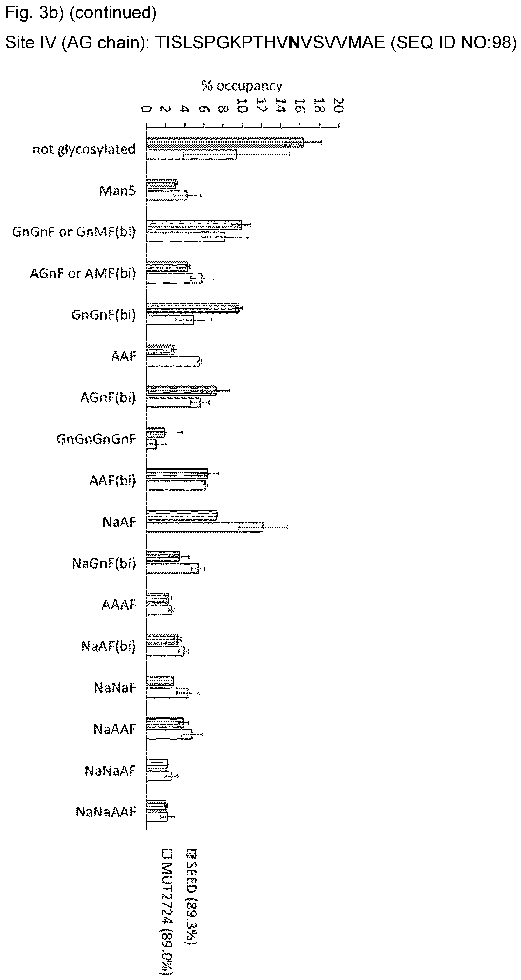

Figure 3: Analysis of glycans in IgA1, SEED-IgA1 and mutated SEED-IgA1 constructs. (a). Schemes of the individual constructs showing the analyzed glycosites (black dots). (b). Frequency of glycans found at individual glycosites (percentage in brackets indicates the coverage achieved after all glycoforms with frequency above 2% were included).

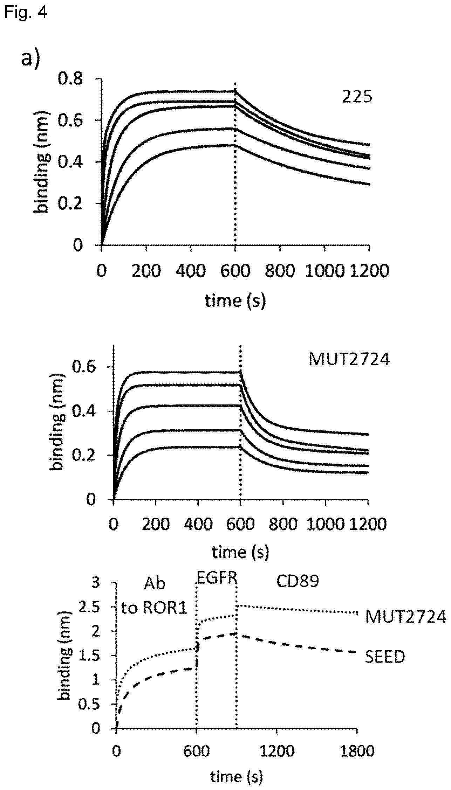

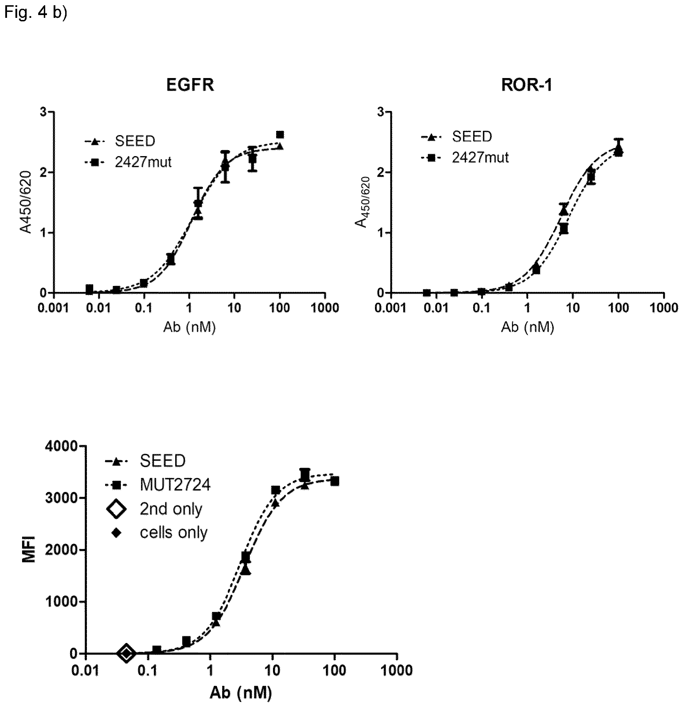

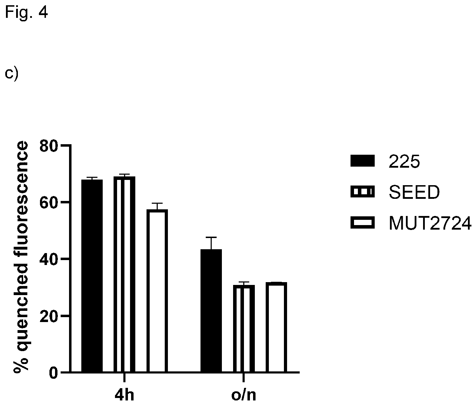

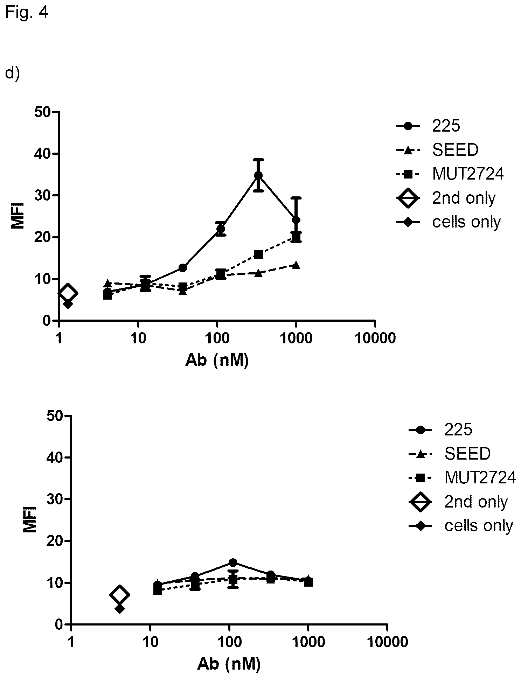

Figure 4: Antigen-binding properties of IgA1, SEED-IgA1 and mutated SEED-IgA1. (a) Biolayer interferometry experiment showing binding of 225-IgA (top) and mutated SEED-IgA1 (center) to immobilized CD89), and difference in binding to CD89 between the mutated and wild-type SEED-IgA1 after the reaction with ROR1 and EGFR (Ab: antibody) (bottom). (b). ELISA assay showing binding of SEED-IgA1 and mutated SEED-IgA1 to EGFR (right) and ROR1 (center) and FACS assay of binding to antigen-positive cell surface (left). (c). Internalization of the constructs into MDA-MB-468 cells monitored with percentage of quenched surface fluorescence. (d). Binding of the constructs to the surface of cell line HL-60 when induced for expression of CD89 and before induction (2nd only: secondary reagent only).

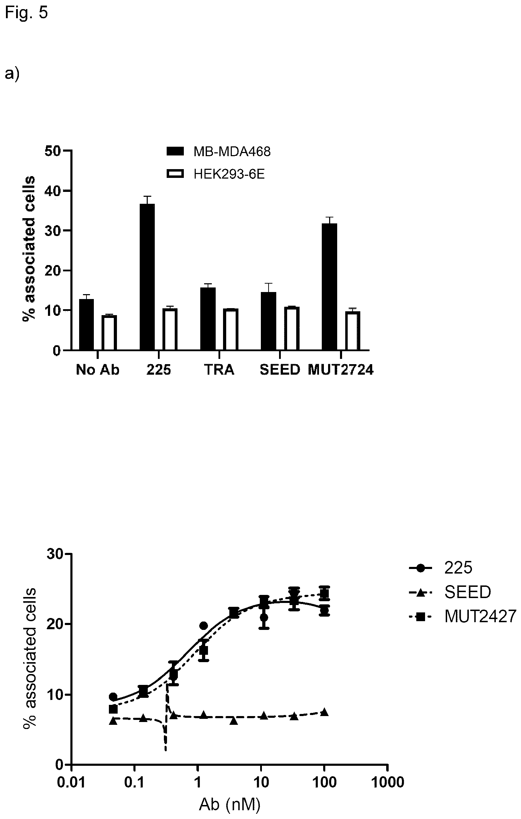

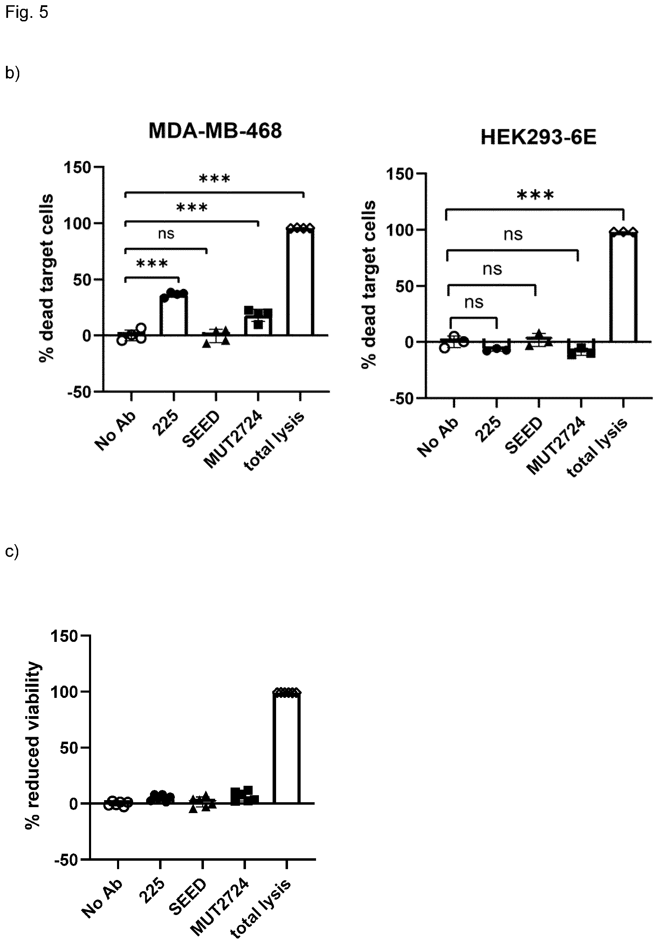

Figure 5: Biological activity of IgA1-based constructs. (a). Cell-cell interaction assay evaluated for the percentage complexed tumor target and induced HL-60 cells with or without addition of the antibodies (left) and the dose-dependency of antibody concentration (right). (b). Results of antibody-dependent cytotoxicity assay using neutrophils as effector cells, with antigen-positive MDA-MB-468 cells and control HEK293-6E cells. (c). Direct effect of antibodies on MDA-MB-468 cells (no effector cells). Significance was determined with 1-way-ANOVA analysis with GraphPad Prism 8.0.2, ns: not significant, P>0.05; *: 0.05>P>0.01; **: 0.01>P>0.001; ***: P<0.001.

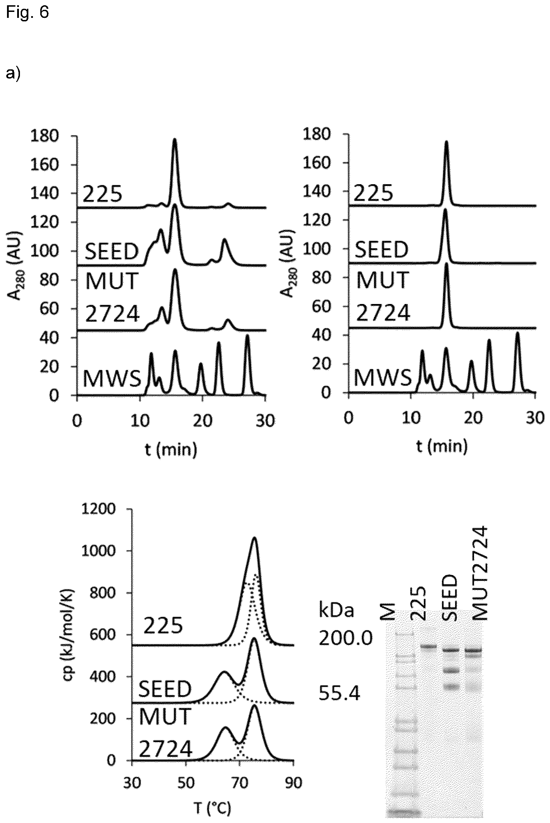

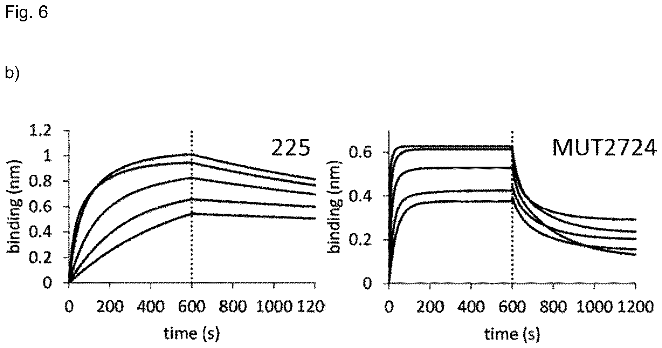

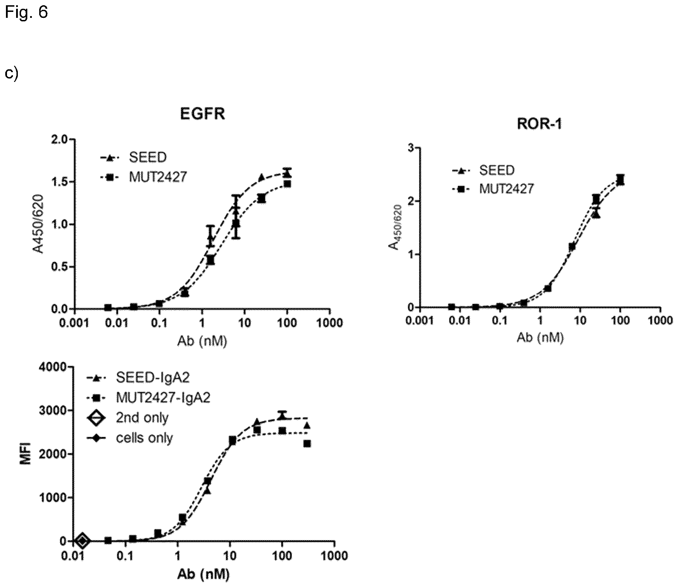

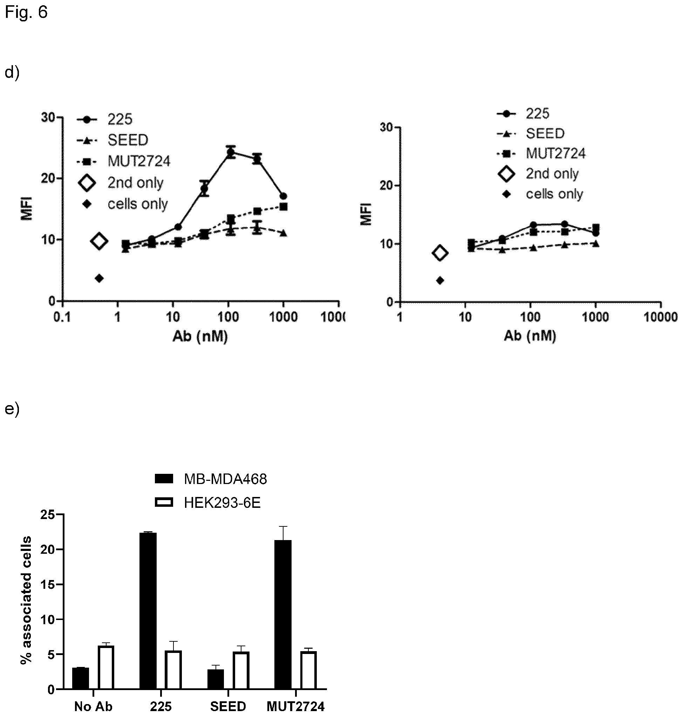

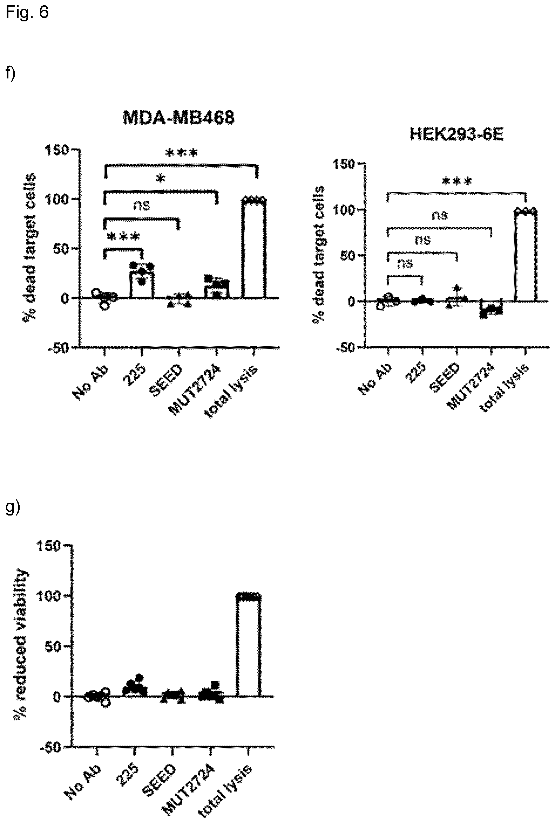

Figure 6: Characterization of antibodies in IgA2 scaffold. (a). Analysis with size exclusion chromatography in native conditions before and after gel filtration (left) (MWS: molecular weight standard), thermostability determined with differential scanning calorimetry (center), and SDS-PAGE analysis (M: Mark 12 Unstained marker). (b). Biolayer interferometry experiment showing binding to immobilized CD89 by 225-IgA2 and mutated SEED-IgA2. (c). Binding of SEED- and mutated SEED constructs to EGFR and ROR1 in ELISA and their reactivity with the MDA-MB-468 cell surface (2nd only: secondary reagent only). (d). Binding to cell surface of HL-60 cells after and before induction of CD89 expression (2nd only: secondary reagent only). (e). Cell-cell interaction assay evaluated for the percentage complexed tumor target and induced HL-60 cells with or without addition of the antibodies. (f). Activity of the antibodies in an ADCC assay using neutrophils as effector cells. (g). Direct effect of antibodies for MDA-MB-468 cells (no effector cells). Significance was determined with 1-way-ANOVA analysis with GraphPad Prism 8.0.2, ns: not significant, P>0.05; *: 0.05>P>0.01; **: 0.01>P>0.001; ***: P<0.001.
Figure 7: Biophysical characterization and antigen binding of different SEED-IgA1 mutants intended for CD89 engagement. (a) Chain composition and individual amino acid substitutions. (EU numbering)
(b) Biolayer interferometry experiment comparing binding of immobilized CD89 by 225-IgA1, SEED-IgA1 and mutant MUT2210 (left), further mutated variants MUT2710 and MUT2724 (center) and individual mutants with different variants of AG chain (MUT2524, MUT2624 and MUT2724) (right). (c) Size exclusion chromatography in native conditions of all presented mutants (MWS: molecular weight standard). Standard peaks present proteins of 670, 158, 44, 17 and 1.3 kDa. (d) Differential scanning calorimetry profiles showing the midpoints of transition (TMs) after deconvolution for SEED and CD89-binding mutants where residues have been modified to increase thermostability.
Figure 8: Induction of CD89 expression on the surface of HL-60 cells. (a) Expression of CD89 on the surface of induced and non-induced HL-60 cells measured with an anti-CD89 antibody (MFI: mean fluorescence units). (b) Determination of CD89 copy number using QIFlkit (open circles: standard bead populations, full diamond: induced HL-60 cells).
Figure 9: Characterization of isolated neutrophils. Percentage of positive cells for each surface marker is indicated.
Figure 10: Cartoon diagram of mutated SEED-IgA2 construct. The Figure was prepared with the PyMOL Molecular Graphics System, Version 2.4.0 Schrödinger, LLC, using PDB: 1R70 and scFv was modelled with SWISS_MODEL.
Figure 11: Analysis of glycans in IgA2, SEED-IgA2 and mutated SEED-IgA2 constructs. (a) Schemes of the individual constructs showing the analyzed glycosites (Black dot) and legend of symbols used for presentation of glycan patterns. (b) Frequency of glycans found at individual glycosites (percentage in brackets indicates the coverage achieved after all glycoforms with frequency above 2% were included).
Figure 12: Sequences as used herein.

### DETAILED DESCRIPTION OF THE INVENTION

Unless indicated or defined otherwise, all terms used herein have their usual meaning in the art, which will be clear to the skilled person. Reference is for example made to the standard handbooks, such as Sambrook et al., 2012, Molecular Cloning: A Laboratory Manual, volumes 1-4, Cold Spring Harbor Press, NY); Lewin, "Genes IV", Oxford University Press, New York, (1990), and Janeway et al., "Immunobiology" (5th Ed., or more recent editions), Garland Science, New York, 2001, Ausubel et al., Current Protocols in Molecular Biology, John Wiley and Sons, Baltimore, Md. (1989), Vega et al., Gene Targeting, CRC Press, Ann Arbor Mich. (1995), and Vectors: A Survey of Molecular Cloning Vectors and Their Uses, Butterworths, Boston Mass. (1988).

As used herein, the terms "a", "an" and "the" are used herein to refer to one or more than one *i.e.,* to at least one. The terms "comprise", "contain", "have" and "include" as used herein can be used synonymously and shall be understood as an open definition, allowing further members or parts or elements. "Consisting" is considered as a closest definition without further elements of the consisting definition feature. Thus "comprising" is broader and contains the "consisting" definition.

The term "about" or "around" as used herein refers to the same value or a value differing by +/-10% or +/-5% of the given value.

Specific terms as used throughout the specification have the following meaning.

Any one or more of the nucleic acids (or nucleic acid molecules), a heterodimeric CH3 assembly, the heterodimeric Fc, or the binding construct (*e.g.*, an antibody), as described herein, are herein also referred to as ""compound" or "compounds" described herein.

The term "antibody" as used herein shall refer to an antigen-binding compound such as an immunoglobulin or immunoglobulin-like molecule, or other proteins exhibiting modular antibody formats *e.g.*, comprising or being composed of one or more antibody domains and bearing antigen-binding properties similar to immunoglobulins. The terms "antibody" and "immunoglobulin" are herein used interchangeably.

An antibody is typically understood as a protein (or protein complex) that includes one or more polypeptides that are encoded by immunoglobulin genes or fragments of immunoglobulin genes. The recognized immunoglobulin genes include the kappa, lambda, alpha, gamma, delta, epsilon, and mu constant region genes, as well as immunoglobulin variable region genes. Light chains (LC) are classified as either kappa (including a VL and a Ckappa domain) or lambda (including a VL and a C lambda domain). Heavy chains (HC) are classified as gamma, mu, alpha, delta, or epsilon, which in turn define the immunoglobulin classes, IgG, IgM, IgA, IgD and IgE, respectively. Typically, an antibody comprises an antigen-binding site through a specific CDR structure of one or more antibody variable domains (*e.g.*, a pair of VH/VL domains) which specifically recognizes a target antigen through the respective CDR loops.

The term "antibody" as used herein specifically includes full-length antibodies, including antibodies of immunoglobulin-like structures. Specifically, an antibody can be a full-length antibody such as of an IgG type (*e.g.*, IgG1, IgG2, IgG3, or IgG4 subtype), IgA1, IgA2, IgD, IgE, or IgM antibody.

The term "full length antibody" is used herein to refer to any antibody molecule comprising an Fc region or at least most of the Fc part of an antibody, which specifically includes a dimer of heavy chains. A full-length antibody can be monospecific or multispecific e.g., bispecific. This term "full length antibody" is used herein to emphasize that a particular antibody molecule is not an antibody fragment.

The term "antibody" shall specifically include antibodies in the isolated form, which are substantially free of other antibodies such as directed against different target antigens and/or comprising a different structural arrangement of antibody domains. Still, an isolated antibody may be comprised in a combination preparation, containing a combination of the isolated antibody *e.g.*, with at least one other antibody, such as monoclonal antibodies or antibody fragments having different specificities.

The term "antibody" shall also apply to antibodies of animal origin, including human species, such as mammalian, including human, murine, rabbit, goat, camelid, llama, cow and horse, or avian, such as hen, which term shall particularly include recombinant antibodies which are based on a sequence of animal origin *e.g.*, human sequences.

The term "antibody" shall specifically apply to human antibodies.

The term "human" as used with respect to an antibody is understood to include antibodies having variable and constant regions derived from human germline immunoglobulin sequences. A human antibody may include amino acid residues not encoded by human germline immunoglobulin sequences (*e.g.*, mutations introduced by random or site-specific mutagenesis *in vitro* or by somatic mutation *in vivo*), for example in the CDRs. Human antibodies include antibodies isolated from human immunoglobulin libraries or from animals transgenic for one or more human immunoglobulin.

A human antibody is preferably selected or derived from the group consisting of IgA1, IgA2, IgD, IgE, IgG1, IgG2, IgG3, IgG4 and IgM.

A murine antibody is preferably selected or derived from the group consisting of IgA, IgD, IgE, IgG1, IgG2A, IgG2B, IgG2C, IgG3 and IgM.

The term "antibody" shall further apply to chimeric antibodies *e.g.*, chimeric antibodies, with sequences of origin of different species, such as sequences of murine and human origin.

The term "chimeric" as used with respect to one or more antibody domains comprised in a compound described herein such as a binding construct (*e.g.*, an antibody), refers to those molecules wherein one portion of each of the amino acid sequences of heavy and light chains is homologous to corresponding sequences in immunoglobulins derived from a particular species or belonging to a particular class, while the remaining segment of the chain is homologous to corresponding sequences in another species or class. Typically, the variable region of both light and heavy chains mimics the variable regions of immunoglobulins derived from one species of mammals, while the constant portions are homologous to sequences of immunoglobulins derived from another. For example, the variable region can be derived from presently known sources using readily available B-cells or hybridomas from non-human host organisms in combination with constant regions derived from, for example, human cell preparations.

The term "antibody" shall further apply to humanized antibodies.

The term "humanized" as used with respect to one or more antibody domains comprised in a compound described herein such as a binding construct (*e.g.*, an antibody), refers to a molecule having a binding moiety (*e.g.*, an antigen-binding site) that is substantially derived from an immunoglobulin from a non-human species, wherein the remaining immunoglobulin structure of the molecule is based upon the structure and/or sequence of a human immunoglobulin. The binding moiety (*e.g.*, antigen binding site) may either comprise complete variable domains fused onto constant domains or only the complementarity determining regions (CDR) grafted onto appropriate framework regions in the variable domains. Binding moieties (*e.g.*, antigen-binding sites) may be wild-type or modified *e.g.*, by one or more amino acid substitutions, preferably modified to resemble human immunoglobulins more closely. Some forms of humanized immunoglobulins preserve all CDR sequences (for example, a humanized mouse antibody which contains all six CDRs from the mouse antibody). Other forms have one or more CDRs which are altered with respect to the original antibody.

According to a specific embodiment, all antibody domains comprised in a compound described herein such as a heterodimeric CH3 assembly, heterodimeric Fc, or a binding construct (*e.g.*, an antibody), are of human origin or humanized, or a functionally active variant thereof with at least 60% sequence identity, or at least 70%, 80%, 90%, or 95% sequence identity to a respective human antibody sequence, preferably wherein the origin of the antibody domains can be selected from any one or more of IgG1, IgG2, IgG3, IgG4, IgA, IgM, or IgE antibodies. Specifically, all antibody domains originate from the same basic immunoglobulin fold, although b-sheet formats may differ, and connecting loops can certainly be variable (to specifically recognize an antigen of choice), especially in V domains.

The term "antibody" further applies to monoclonal or polyclonal antibodies, specifically a recombinant antibody, which term includes all antibodies and antibody structures that are prepared, expressed, created or isolated by recombinant means, such as antibodies originating from animals, *e.g.* mammalian species including humans, which comprise genes or sequences from different origin, *e.g.* chimeric, humanized antibodies, or hybridoma derived antibodies. Further examples refer to antibodies isolated from a host cell transformed to express the antibody, or antibodies isolated from a recombinant, combinatorial library of antibodies or antibody domains, or antibodies prepared, expressed, created or isolated by any other means that involve splicing of antibody gene sequences to other DNA sequences.

Antibody domains may be of native structure or can be modified by mutagenesis or derivatization *e.g.*, to modify the antigen binding properties or any other property, such as half-life, thermostability, effector function, or binding properties, such as binding to any one or more of Fc receptors *e.g.*, FcαR, FcγR, or FcRn. The term "antibody" further includes variants (or functionally active variants), derivatives, combinations or fusions of antibodies, antibody domains, or antibody fragments.

An antibody typically comprises or consists of antibody domains, which are constant or variable domains of the heavy or light chains of immunoglobulins, with one or more linkers (such as a linking sequence or a hinge sequence), or without a linker.

Antibodies are specifically understood to comprise or consist of combinations of variable and/or constant antibody domains with or without a linker or hinge, including pairs of variable antibody domains, such as one or two VH/VL pairs. Polypeptides are understood as antibody domains, if comprising a beta-barrel structure consisting of at least two beta-strands (also referred to as "beta-sheets") of an antibody domain structure connected by a loop sequence.

The Fv part of an antibody is typically understood as the pair of VL and VH domains that produces a heterodimer by connecting a binding surface involving the C, C' and F strands of each of the domains (the binding interface). By such contact of the beta-sheet region of the VL domain with the beta-sheet region of the VH domain, a dimer (designated as VL/VH) is produced.

A Fab arm is herein understood as the pair of a first and a second antibody chain, wherein the first chain comprises or consists of a VL domain and a CL domain, which is linked to the C-terminus of the VL domain (light chain, LC), and the second chain comprises or consists of a VH domain and a CH1 domain, which is linked to the C-terminus of the VH domain (heavy chain, HC), wherein the VL connects to (pairs with) the VH via the binding interface, and the CL connects to (pairs with) the CH1 via the binding interface, thereby producing a (hetero)dimer of the LC and HC (also designated LC/HC).

The Fc part of an antibody is herein understood as the pair of antibody chains, each comprising a CH2 domain and a CH3 domain, which is linked to the C-terminus of the CH2 domain (Fc chains), wherein the CH2 domains of each of the antibody chains connect to each other via the binding surface involving the A, B and/or E strands of each of the CH2 domains (the binding interface), and wherein the CH3 domains of each of the antibody chains connect to (pair with) each other, in particular via the binding surface involving the A, B and/or E strands of each of the CH3 domains (by contact of the dimerization sheets of the CH3 domains, specifically forming the assembly interface), thereby producing a dimer of Fc chains. The Fc described herein is a heterodimeric Fc, in particular because of the CH3 domains which are engineered to favor heterodimerization over homodimerization.

The term "CH3 domain" as used herein refers to a constant domain of an antibody heavy chain designated CH3. A CH3 domain comprise an immunoglobulin fold characterized by a beta-sheet sandwich structure with several beta-strands in each sheet, wherein said domain participates in the formation of dimeric interfaces between paired heavy chains. A CH3 domain comprises specific structural elements such as, beta strands that form the core immunoglobulin fold, interface-forming residues that mediate dimerization, loops that connect the beta-strands, optionally at least one disulfide bond, and residues that contribute to domain stability. Specifically, CH3 domains participate in heavy chain dimerization and contribute to effector functions resulting *e.g.*, from Fc receptor binding and/or complement activation. The term CH3 domain comprises naturally occurring CH3 domains from any immunoglobulin isotype, including but not limited to IgA, IgD, IgE, IgG, and IgM, mutant CH3 domains comprising one or more amino acid substitutions, deletions, or insertions relative to a naturally occurring CH3 domain, allotypic variants, engineered CH3 domains modified to promote heterodimeric assembly, and hybrid CH3 domains comprising sequences from different immunoglobulin isotypes or subtypes.

The term "CH3 assembly" as used herein refers to an assembly of two CH3 domains. CH3 assemblies are also called dimers or pairs of CH3 domains. CH3 domains of an assembled CH3 pair are herein also referred to as "first and second CH3 domains".

The assembly of CH3 domains is mainly determined by an assembly interface formation through beta-sheet interactions, in particular through the dimerization sheets of the two CH3 domains. Specifically, each of the CH3 domains in the CH3 assembly comprises a dimerization sheet, and the CH3 domains assemble by the interaction of the two dimerization sheets.

Regarding the beta-sheet interactions, specific beta-strands from each CH3 domain can be involved in an assembly interface formation, for example by the ABED sheet of a first CH3 domain interacting with the ABED sheet of a second CH3 domain, e.g., by conserved residues that form key contact points between the domains, and by precise alignment of the beta-strands.

The assembly process is typically as follows. Initially, complementary surfaces contact between complementary surfaces, key hydrophobic interactions are formed, hydrogen bonding networks are established, secondary interactions provide stabilization and finally, structural adjustments take place to achieve the optimal interface packing. The assembled structure is maintained by buried hydrophobic residues at the core of the interface, networks of hydrogen bonds, water-mediated interactions, electrostatic complementarity between surfaces, and conserved structural motifs that lock the domains in place. Environmental factors may influence the assembly process, such as pH conditions, temperature, ionic strength, presence of molecular chaperones, oxidizing/ reducing conditions, and/ or protein concentration.

Specifically, this assembly process is primarily characteristic of a natural homodimeric CH3 assembly. However, heterodimeric CH3 assemblies described herein may as well be formed according to such process.

Specifically, the CH3 assemblies described herein are heterodimeric, in particular comprising a first and second CH3 domains which are engineered to form heterodimers preferentially over forming homodimers.

Specifically, a CH3 assembly described herein comprises a first and a second CH3 domain, wherein the two CH3 domains are engineered CH3 domains such that the amino acid sequence of the CH3 domain differs from the amino acid sequence of the second CH3 domain. An assembly of such first and second CH3 domain is, thus, heterodimeric. CH3 domains of the heterodimeric CH3 assembly described herein are particularly engineered for an enhanced heterodimerization, in particular for preferred heterodimerization over homodimerization.

According to a specific aspect, the CH3 assembly (or Fc) as described herein comprises two differently mutated CH3 domains such as to favor heterodimerization over homodimerization. Specifically, heterodimerization of the CH3 domains of the heterodimeric CH3 assembly described herein is supported by engineering the dimerization sheets of the CH3 domains such as to incorporate alternating IgA and IgG segments.

Specifically, each of the CH3 domains comprises a dimerization sheet (in particular wherein the dimerization sheet is positioned at the assembly interface), each dimerization sheet being engineered to comprise alternating IgA and IgG segments, wherein the segments of the first CH3 domain are dimerized to the respective segments of the second CH3 domain to form the heterodimeric CH3 domains. In particular, structurally related sequences are exchanged within the CH3 domains. Alternating sequences from IgA and IgG in the CH3 domains generate two asymmetric but complementary domains, herein also referred to as designated AG and GA CH3 domains. This design allows efficient generation of AG/GA heterodimers, while disfavoring homodimerization of AG and GA CH3 domains.

Specifically, the CH3 mutations include an intermolecular beta-strand swap, *e.g.* wherein one or more segments or sequences within a beta-strand of a CH3 domain are mutated to incorporate segments or sequences of another CH3 domain which differ from those of the original CH3 domain, *e.g.* wherein the beta-strand swap is of CH3 domains of a different type or subtype. Specifically, the CH3 domains can be engineered by strand-exchange, wherein a CH3 domain of an IgG type incorporates one or more segments or sequences of a CH3 domain of an IgA type. If two strand-exchanged CH3 domains are mutated to form a cognate pair of CH3 domains which differ in their sequence, the IgA segments or sequences of each of the CH3 domains produce an interdomain contact surface (in particular an assembly interface) which is cognate, such that the mutated heterodimeric CH3 domains preferentially pair with each other over forming respective homodimers. Specific examples of such modifications of antibody domains to incorporate a segment swap may be strand-exchange engineered CH3 domains (SEED). Such modifications can be used to produce asymmetric or bispecific binding constructs (such as antibodies) by preferentially pairing the SEED modified CH3 domains.

The term "alternating IgA and IgG segments" as described herein refers to complementary beta-strand segments derived from IgA and IgG CH3 domains. According to a specific example, at least two IgA segments and at least two IgG segments are used, wherein each IgA segment is followed by an IgG segment or each IgG segment is followed by an IgA segment. An exemplary alternating pattern follows an A1-G2-A3-G4 or G1-A2-G3-A4 arrangement, where A is an IgA-derived segment and G is an IgG-derived segment. Segments are preferably chosen which comprise at least one residue in the respective junctions between the IgA and IgG segments, which is conserved in both CH3 domains.

Preferred IgA and IgG segments are of human immunoglobulin origin.

The heterodimeric CH3 assembly described herein specifically comprises strand-exchange engineered domain (SEED) CH3 domains and respective strand-exchange engineered domain (SEED) CH3 dimerization sheets.

According to a specific aspect, the CH3 domains of a heterodimeric CH3 assembly described herein may be further engineered to preferentially form heterodimers over homodimers. In order to favor proper pairing of antibody chains or domains, any further CH3 mutations may be employed, such as the knobs-into-holes technology, charge repulsion technology, disulfide linkage, or the cross-mAb technology.

For example, specific knob mutations can be introduced, which are understood as one or more amino acid substitutions to increase the contact surface between the two CH3 domains. In particular, one or more amino acids can be introduced which provide for an additional protuberance of a beta-strand structure, *e.g.* one or more of CH3 knob mutations selected from the group consisting of T366Y, T366W, T394W, F405A. A specific knob modification denotes the mutation T366W in the CH3 domain of an antibody (EU numbering). Knob mutations in a CH3 domain specifically provide a matching (cognate) surface to bind another CH3 domain *e.g.*, which is modified to incorporate hole mutations at corresponding positions.

Specific hole mutations are one or more amino acid substitutions to increase the contact surface between two domains by incorporating one or more amino acids which provide for an additional cave of a beta-strand structure, *e.g.* one or more of CH3 hole mutations selected from the group consisting T366S, L368A and Y407V (EU numbering). A specific hole-modification denotes any of the mutations T366S, L368A, Y407V, Y407T in the CH3 domain of an antibody (EU numbering). Hole mutations specifically provide a matching (cognate) surface to bind another antibody domain, e.g. which is modified to incorporate knob mutations.

Matching knob into hole mutations are, *e.g.* T366Y on one CH3 domain and the matching Y407'T on the second CH3 domain of the CH3 domain pair, herein referred to as T366Y/Y407'T. Further matching mutations are
T366Y/Y407'T,
F405A/T394'W,
T366Y:F405A/T394'W:Y407'T,
T366W/Y407'A, or
S354C:T366W/Y349'C:T366'S:L368'A:Y407'V,
(EU numbering).

The connection of antibody domains or LC/HC, or Fc chains may be further supported by intradomain or interdomain disulfide bridges. Disulfide bonds are usually formed from the oxidation of thiol groups of two cysteines, thereby linking the S-atoms to form a disulfide bridge between the two cysteine residues.

According to a specific aspect, antibody domains include mutations incorporating cysteine residues which are capable of forming disulfide bridges to stabilize an antibody domain by an additional intradomain disulfide bridge, or a pair of antibody domains by an additional interdomain disulfide bridge. Specifically, cysteine may be inserted (by an additional amino acid or an amino acid substitution) in the C-terminal region or at the C-terminus of a CH3 domain. A pair of CH3 that bears an additional cysteine modification can be stabilized by disulfide bond formation between the CH3 pair.

The term "beta-sheet" or "beta strand" of an antibody domain is herein understood in the following way. An antibody domain typically consists of at least two beta strands connected laterally by at least two or three backbone hydrogen bonds, forming a generally twisted, pleated sheet. A beta strand is a single continuous stretch of amino acids of typically 3 to 10 amino acids length adopting such an extended conformation and involved in backbone hydrogen bonds to at least one other strand, so that they form a beta sheet. In the beta sheet, the majority of beta strands are arranged adjacent to other strands and form an extensive hydrogen bond network with their neighbors in which the N-H groups in the backbone of one strand establish hydrogen bonds with the C=O groups in the backbone of the adjacent strands.

The structure of antibody constant domains, such as CH2 or CH3 domains, is similar to that of variable domains, consisting of beta-strands connected by loops, some of which contain short alpha-helical stretches. The framework is mostly rigid and the loops are comparatively more flexible, as can be seen from the b-factors of various Fc crystal structures. An antibody CH2 or CH3 domain typically has seven beta strands forming a beta-sheet (A-B-C-D-E-F-G), wherein the beta strands are linked via loops, three loops being located at the N-terminal tip of the domain (A-B, C-D, E-F), and further three loops being located at the N-terminal tip of the domain (B-C, D-E, F-G). A "loop region" of a domain refers to the portion of the protein located between regions of beta strands (for example, a CH3 domain comprises seven beta sheets, A to G, oriented from the N- to C-terminus).

An "assembly", "dimer" or pair", of antibody domains is herein understood as a set of two antibody domains, where one has an area on its surface or in a cavity that specifically binds to, and is therefore complementary to, an area on the other one. Antibody domains may associate and assemble to form a pair of antibody domains through contact of a beta-sheet region. Such domain pair is also referred to as a dimer, which is e.g. associated by electrostatic interaction, recombinant fusion or covalent linkage, placing two domains in direct physical association. In a pair of antibody domains, the antibody domains are also referred to as "counterpart" domains. In an exemplary antibody described herein, the following domains are considered counterparts suitably forming a pair of antibody domains (counterparts separated by a slash (/)):
VL/VH;
CL (Clambda or Ckappa)/CH1;
CH2/CH2;
CH3/CH3.

The term "assembly interface" as used herein with respect to a CH3 assembly such as described herein shall refer to the region where two CH3 domains interact to form a stable CH3 assembly, wherein such region comprises the contact surfaces (in whole or in part) of the two CH3 domains. The assembly interface specifically encompasses amino acid residues from both assembled CH3 domains that are within contact distance from each other and participate in an inter-domain interaction. Specifically, the assembly interface may further comprise one or more regions adjacent to the contact surface, that influence the dynamics, stability, or affinity of heterodimer formation during assembly.

An assembly interface typically comprises one or more beta-strands, and optionally other structural elements of an antibody domain, which form a complementary contact surface between the first and second CH3 domain.

Specifically, an assembly interface of the CH3 assembly described herein may comprise or consist of a dimerization sheet.

Specifically, the assembly interface comprises or consists of beta-sheets of both CH3 domains that are in contact with each other when the CH3 domains are assembled.

In a CH3 assembly described herein there is a dimerization sheet in each of the first and second CH3 domains. Specifically, the dimerization sheets form an assembly interface of the first and second CH3 domains. Specifically, the CH3 assembly described herein is a heterodimeric CH3 assembly, wherein the dimerization sheets of the first and second CH3 domains are engineered in a way to favor heterodimer formation. Specifically, either of the dimerization sheets comprises segments of different immunoglobulin origin, specifically IgA and IgG, that create complementary contact surfaces between the first and second CH3 domains of the CH3 assembly. Specifically, part of the assembly interface consists of IgA-derived sequences while another part consists of IgG-derived sequences.

The term "binding construct" (herein also referred to as "binding molecule") as used herein shall mean a compound comprising a binding moiety and a CH3 assembly, such as a heterodimeric CH3 assembly described herein, or an Fc, such as a heterodimeric Fc described herein. A binding construct may consist of one or more polypeptide chains or domains which may be bound or associated (or assembled) thereby forming a multichain or multidomain complex. An exemplary multichain binding construct is a binding construct comprising two domains or chains, herein also referred to as dimer or pair, which are dimerized.

A binding construct which comprises two dimerized domains or chains is herein also referred to as dimeric binding construct. A binding construct which comprises two non-identical dimerized domains or chains, is herein referred to as "heterodimeric". A binding construct which comprises two identical dimerized domains or chains, is herein referred to as "homodimeric".

A binding construct described herein can be provided as an isolated molecule or as a combination molecule, *e.g.* wherein the binding construct is combined with other molecular entities, in particular through recombination, fusion or conjugation techniques, which combination molecule can also be provided in the isolated form.

The term "binding construct" as used herein shall particularly include variants (e.g., variants which are derivatives), or functionally active variants of binding constructs further described herein.

Exemplary binding constructs comprise or consist of an antibody or antigen-binding fragment thereof.

The term "binding moiety" as used herein refers to molecules or molecular entities (*e.g.*, a peptide or polypeptide, such as an antibody domain) or an association of molecules or molecular entities (*e.g.*, an assembly or complex, such as a dimer or multimer of peptides or polypeptides, for example, an antigen-binding site of an antibody or an antibody Fv), which comprise a binding site that is capable of binding interactions with a target. Binding moieties can be used as such or integrated within a larger protein or protein complex (or assembly), thus, forming a specific region of such protein or protein complex with binding function.

Typically, a binding moiety of a binding construct described herein comprises a target-binding site.

Binding moieties can be derived from natural repertoires of binding polypeptides or proteins such as from immunoglobulins or antibodies. A variety of binding moieties can be produced by randomisation techniques, in particular by engineering a library of potential binding moieties and selecting those with a desired binding property. For example, antibody domains or loops of antibody domains, such as CDR loops of antibody domains, can be mutagenized to produce a respective library of binding moieties.

A binding moiety of a binding construct described herein, such as an antigen-binding moiety comprising an antibody variable region, may comprise an antigen-binding site of an antibody. Such antigen-binding site is typically formed by amino acid residues of the N-terminal variable ("V") regions of the heavy ("H") and/or light ("L") chains of an antibody, or by one or more antibody variable domains, in particular two antibody variable domains, a VH and a VL. Three highly divergent stretches within the V regions of the heavy and light chains, referred to as "hypervariable regions", are interposed between more conserved flanking stretches known as framework regions. The antigen-binding site provides for a surface that is complementary to the three-dimensional surface of a bound epitope or antigen, and the hypervariable regions are referred to as "complementarity-determining regions", or "CDRs." The antigen-binding site incorporated in the CDRs is herein also called "CDR binding site".

The term "derivative" as used herein with respect to a compound described herein, in particular a nucleic acid, a heterodimeric CH3 assembly, a heterodimeric Fc, or a binding construct (*e.g.*, an antibody), is understood as a molecule obtained by association or binding of the respective compound to other substances (*e.g.*, active substances) by a chemical reaction such as covalent coupling, electrostatic interaction, di-sulphide bonding *etc.* Substances bound to the compound can be selected from lipids, carbohydrates, nucleic acids, organic and inorganic molecules, or a combination of any of the foregoing (*e.g.* PEG, prodrugs or drugs). A derivative can also be a compound with the same amino acid sequence but made completely or partly from non-natural or chemically modified amino acids. Specifically, the compound can be a derivative comprising an additional tag allowing specific interaction with a biologically acceptable compound. There is not a specific limitation with respect to the tag usable, as far as it has no or tolerable negative impact on the binding of the antibody to its target. Examples of suitable tags include His-tag, Myc-tag, FLAG-tag, Strep-tag, Calmodulin-tag, GST-tag, MBP-tag, and S-tag. According to a specific example, the derivative is a compound comprising a label. The term "label" as used herein refers to a detectable compound or composition which is conjugated directly or indirectly to the compound so as to generate a "labelled" compound. The label may be detectable by itself, *e.g.* radioisotope labels or fluorescent labels, or, in the case of an enzymatic label, may catalyze chemical alteration of a substrate compound or composition which is detectable.

A derivative of a binding construct can be a variant of a parent binding construct and respective parent sequence, such as a parent antigen-binding (*e.g.* CDR) or framework (FR) sequence of an antibody *e.g.*, a mutant obtained by *in silico* or recombinant engineering or by site-directed mutagenesis or library techniques, or variants obtained by chemical derivatization or synthesis.

A derivative of a compound described herein, in particular a nucleic acid, a heterodimeric CH3 assembly, a heterodimeric Fc, or a binding construct (*e.g.*, an antibody), can be any combination of one or more of such compounds or a fusion protein in which any domain of a compound is fused (at any position) to one or more other molecular entities, such as to other compounds or active substances, in particular ligands, enzymes, drugs, toxins and the like.

Specifically, a derivative of an antibody can be any combination of one or more antibodies or a fusion protein in which any domain of an antibody is fused (at any position) to one or more other molecular entities, such as to other antibodies or antibody fragments, but also to ligands, enzymes, drugs, toxins and the like.

The term "dimerization sheet" as used herein with respect to a CH3 domain of a CH3 dimer (in particular a CH3 assembly) shall refer to the region of a CH3 domain comprising or consisting of one or more beta-strands or beta-sheets of a first CH3 antibody domain which are positioned to face and contact another dimerization sheet of a second CH3 domain in a CH3 dimer. The beta-strands may interact in an anti-parallel orientation and create an interaction surface (in particular an assembly interface). Specifically, the dimerization sheet in a CH3 domain comprises regions of the four key beta-strands (A, B, D, and E) which may play a role at the interface of a CH3 domain which faces the opposing CH3 domain for dimerization. In particular, the dimerization sheet comprises or consists of specific regions of beta-strands of a CH3 domain which are involved in an assembly interface formation, for example the dimerization sheet of a first CH3 domain is composed of the ABED sheet which interacts with the dimerization sheet (the ABED sheet) of a second CH3 domain, thereby forming a CH3 dimer or assembly.

Specifically, the dimerization sheet interactions are beta-sheet interactions, by conserved residues that form key contact points between the domains, and by precise alignment of the contacted beta-strands.

Specifically, the interactions may involve one or more of hydrogen bonding interactions, van der Waals interactions, hydrophobic interactions, electrostatic interactions and amino acid side-chain packing arrangements.

The first and second CH3 domains of a CH3 dimer can be assembled by an interaction of the respective dimerization sheets, thereby forming a CH3 assembly. Typically, the dimerization sheet of a first CH3 domain forms extensive interactions with the dimerization sheet of a second CH3 domain.

The term "expression" is understood in the following way. Nucleic acid molecules containing a desired coding sequence of an expression product such as *e.g.* a compound described herein, and control sequences such as *e.g.*, a promoter in operable linkage, may be used for expression purposes. Hosts transformed or transfected with these sequences are capable of producing the encoded proteins. In order to effect transformation, the expression system may be included in a vector; however, the relevant DNA may also be integrated into the host chromosome. Specifically, the term refers to a host cell and compatible vector under suitable conditions, *e.g.* for the expression of a protein coded for by foreign DNA carried by the vector and introduced to the host cell.

Coding DNA is a DNA sequence that encodes a particular amino acid sequence for a particular polypeptide or protein such as *e.g.*, a compound described herein. Promoter DNA is a DNA sequence which initiates, regulates, or otherwise mediates or controls the expression of the coding DNA. Promoter DNA and coding DNA may be from the same gene or from different genes, and may be from the same or different organisms. Recombinant cloning vectors will often include one or more replication systems for cloning or expression, one or more markers for selection in the host, *e.g.* antibiotic resistance, and one or more expression cassettes.

"Vectors" used herein are defined as DNA sequences that are required for the transcription of cloned recombinant nucleotide sequences, *i.e.* of recombinant genes and the translation of their mRNA in a suitable host organism.

An "expression cassette" refers to a DNA coding sequence or segment of DNA that code for an expression product that can be inserted into a vector at defined restriction sites. The cassette restriction sites are designed to ensure insertion of the cassette in the proper reading frame. Generally, foreign DNA is inserted at one or more restriction sites of the vector DNA, and then is carried by the vector into a host cell along with the transmissible vector DNA. A segment or sequence of DNA having inserted or added DNA, such as an expression vector, can also be called a "DNA construct".

Expression vectors comprise the expression cassette and additionally usually comprise an origin for autonomous replication in the host cells or a genome integration site, one or more selectable markers (*e.g.*, an amino acid synthesis gene or a gene conferring resistance to antibiotics such as zeocin, kanamycin, G418 or hygromycin), a number of restriction enzyme cleavage sites, a suitable promoter sequence and a transcription terminator, which components are operably linked together. The term "vector" as used herein includes autonomously replicating nucleotide sequences as well as genome integrating nucleotide sequences. A common type of vector is a "plasmid", which generally is a self-contained molecule of double-stranded DNA that can readily accept additional (foreign) DNA and which can readily be introduced into a suitable host cell. A plasmid vector often contains coding DNA and promoter DNA and has one or more restriction sites suitable for inserting foreign DNA. Specifically, the term "vector" or "plasmid" refers to a vehicle by which a DNA or RNA sequence (*e.g.* a foreign gene) can be introduced into a host cell, so as to transform the host and promote expression (*e.g.* transcription and translation) of the introduced sequence.

A recombinant expression product which is a compound described herein, in particular a nucleic acid, a heterodimeric CH3 assembly, a heterodimeric Fc, or a binding construct (*e.g.*, an antibody), can be produced using a recombinant host cell described herein, which comprises one or more expression cassettes that are encoding and expressing the respective compound. For expression, the host cell described herein can be cultured in an appropriate medium, and the expressed product can be isolated from the culture, and optionally further purified.

Specifically, an expression product can be harvested from the cell culture supernatant or a cellular fraction and optionally be purified to obtain the expression product at a higher degree of purity.

Methods for recovering and/or purifying an expression product such as a compound described herein are well-established in the art. Specifically, a physical or chemical or physical-chemical method is used. The physical or chemical or physical-chemical method can be a filtering method, a centrifugation method, an ultracentrifugation method, an extraction method, a lyophilization method, a precipitation method, a chromatography method or a combination of two or more of any such methods. Specifically, the chromatography method comprises one or more of size-exclusion chromatography (or gel filtration), ion exchange chromatography, e.g., anion or cation exchange chromatography, affinity chromatography, hydrophobic interaction chromatography, and/or multimodal chromatography.

There are several different approaches for the recombinant production of an expression product such as a compound described herein. An expression product may be expressed, processed and optionally secreted by transforming or transfecting a host cell with an expression vector harboring recombinant DNA encoding the expression product, preparing a culture of the transformed or transfected cell, growing the culture, inducing transcription and expression product production, and recovering the expression product.

The term "heterodimeric" as described herein shall refer to two non-identical domains or chains associating to form an asymmetric dimeric pair of domains or chains, respectively. In particular, a heterodimeric assembly is characterized by an asymmetric assembly (or an asymmetric interface formation) between non-identical domains or chains.

The term "homodimeric" as described herein shall refer to two identical domains or chains associating to form a symmetric dimeric pair of domains or chains, respectively. In particular, a homodimeric assembly is characterized by a symmetric assembly (or an asymmetric interface formation) between identical domains.

The term "host cell" as used herein shall particularly apply to any cell, which is suitably used for recombination purposes or as a recombinant host cell, to produce an expression product *e.g.*, a compound described herein. The term "host cell" shall particularly shall refer to primary subject cells transformed to produce a particular recombinant protein, such as a binding construct as described herein, and any progeny thereof. It should be understood that not a progeny may or may not be exactly identical to the parental cell (due to deliberate or inadvertent mutations or differences in environment), however, such altered progeny is included in these terms, so long as the progeny retains the same functionality as that of the originally transformed cell.

The term "host cell line" refers to a cell line of host cells as used for expressing a recombinant gene to produce recombinant polypeptides such as a recombinant compound described herein. The term "cell line" as used herein refers to an established clone of a particular cell type that has acquired the ability to proliferate over a prolonged period of time. Such host cell or host cell line may be maintained in cell culture and/or cultivated to produce a recombinant polypeptide.

It is well understood that the term "host cell" does not include human beings. Specifically, the host cells as described herein are recombinant host cells, which are artificial organisms or derivatives of native (wild-type) host cells. It is well understood that the host cells, methods and uses described herein, nucleic acid molecules described herein, expression cassettes expressing one or more nucleic acid molecules described herein, and transfected or transformed host cells and recombinant expression products described herein are non-naturally occurring, "man-made" or synthetic, and are therefore not considered as a result of "law of nature".

Host cells described herein may be cultured continuously or discontinuously; in a batch process, a fed-batch process or a repeated fed-batch process. According to a specific aspect, the cell culture process is a fed-batch process. For example, in a fed-batch process, a host cell transfected with one or more nucleic acid molecules encoding compound described herein can be first cultured in a growth phase and transitioned to a production phase in order to produce the compound as an expression product.

According to a specific aspect, host cells described herein can be cultured in a continuous mode *e.g.*, employing a chemostat. A continuous fermentation process is characterized by a defined, constant and continuous rate of feeding of fresh culture medium into a bioreactor, whereby culture broth is at the same time removed from the bioreactor at the same defined, constant and continuous removal rate. By keeping culture medium, feeding rate and removal rate at the same constant level, the cell culture parameters and conditions in the bioreactor remain constant.

According to a specific aspect, host cells described herein can be cultured in a perfusion mode e.g., culturing cells within a device while supplying fresh medium and removing the supernatant.

The term "isolated" or "isolation" as used herein with respect to a compound described herein, in particular a nucleic acid, a heterodimeric CH3 assembly, a heterodimeric Fc, or a binding construct (*e.g.*, an antibody), shall refer to such compound that has been sufficiently separated from the environment with which it would naturally be associated, so as to exist in "substantially pure" form. "Isolated" does not necessarily mean the exclusion of artificial or synthetic mixtures with other compounds or materials, or the presence of impurities that do not interfere with the fundamental activity, and that may be present, for example, due to incomplete purification. In particular, isolated nucleic acid molecules encoding a heterodimeric CH3 assembly, a heterodimeric Fc, or a binding construct, such as an antibody, as described herein are also meant to include codon-optimized variants of naturally occurring nucleic acid sequences to improve expression in a certain host cell, or those chemically synthesized.

With reference to nucleic acids or nucleic acid molecules described herein, it is well understood that these may be provided as isolated compounds. Such compounds are herein also referred to as "isolated nucleic acid". This term, when applied to DNA, refers to a DNA molecule that is separated from sequences with which it is immediately contiguous in the naturally occurring genome of the organism in which it originated. For example, an "isolated nucleic acid" may comprise a DNA molecule inserted into a vector, such as a plasmid or virus vector, or integrated into the genomic DNA of a prokaryotic or eukaryotic cell or host organism. When applied to RNA, the term "isolated nucleic acid" refers primarily to an RNA molecule encoded by an isolated DNA molecule as defined above. Alternatively, the term may refer to an RNA molecule that has been sufficiently separated from other nucleic acids with which it would be associated in its natural state (*i.e.,* in cells or tissues). An "isolated nucleic acid" (either DNA or RNA) may further represent a molecule produced directly by biological or synthetic means and separated from other components present during its production.

With reference to polypeptides or proteins as described herein, such as a heterodimeric CH3 assembly, a heterodimeric Fc, or a binding construct (*e.g.*, an antibody), it is well understood that these may be provided as isolated compounds. The term "isolated" shall specifically refer to compounds that are free or substantially free of material with which they are naturally associated such as other compounds with which they are found in their natural environment, or the environment in which they are prepared (e g., in a cell culture or cell culture fraction) *e.g.*, when such compounds are prepared by recombinant DNA technology, which can be practiced *in vitro* or *in vivo.* Isolated compounds can be formulated with diluents or adjuvants and still for practical purposes be isolated. For example, the polypeptides or polynucleotides can be mixed with pharmaceutically acceptable carriers or excipients such as for therapeutic or (*in vitro* or *in vivo*) diagnostic use.

The term "multivalent" with respect to a binding construct described herein shall refer to a molecule having at least two binding sites to bind the same target. The binding sites may recognize the same epitope, or may recognize different epitopes of such target. The term shall apply to a binding construct with two or more valencies to bind the target antigen, *e.g.* through at least 2, 3, 4 or even more binding sites. In particular, the term shall apply to a bivalent binding construct. For example, the binding construct may be a bivalent antibody which comprises two antigen-binding sites through two pairs of VH/VL domains, both binding the same target antigen or epitope.

The term "multispecific" with respect to a binding construct as described herein shall refer to a molecule having at least two binding sites specifically binding at least two different targets. The term shall apply to a binding construct with two or more specificities to bind at least two different targets, *e.g.* through at least 2, 3, 4 or even more binding sites. In particular, the term shall apply to a bispecific binding construct. For example, the binding construct may be a bispecific antibody which comprises two antigen-binding sites through two pairs of VH/VL domains, each binding a different target antigen or epitope. A bispecific antibody may bind one target antigen through a first pair of VH/VL domains (first Fv region), and another target antigen by a second pair of VH/VL domains (second Fv region). A bispecific antibody typically is composed of four different antibody chains, *i.e.* two HCs and two LCs, such that two different CDR binding sites are formed by heterodimerization (pairing) of a first HC with a first LC and a second HC with a second LC.

A "point mutation" is herein understood as the mutation that results from engineering an amino acid sequence or a polynucleotide to introduce a substitution or exchange, deletion or insertion of one or more single (non-consecutive) or doublets of amino acids for different amino acids. Specifically, the amino acid sequence engineered to comprise a point mutation differs from the non-engineered amino acid sequence by the respective mutation.

One or both of the CH3 domains of the CH3 assembly described herein comprises a number of point mutations to introduce a new CD89 binding site, as further described herein.

Besides such point mutations, a compound described herein, in particular a nucleic acid, a heterodimeric CH3 assembly, a heterodimeric Fc, or a binding construct (*e.g.*, an antibody), may further contain point mutations such as to introduce conservative substitutions *e.g.*, by an exchange of one or more amino acids of the same polarity and/or charge. In this regard, amino acids refer to 20 naturally-occurring amino acids encoded by sixty-four triplet codons. These 20 amino acids can be split into those that have neutral charges, positive charges, and negative charges:

The 20 naturally-occurring amino acids are shown in the table below along with their respective three-letter and single-letter code and polarity:

| **Amino-acid name** | **3-letter code** | **1-letter code** | **Properties** |
|---|---|---|---|
| Alanine | Ala | A | Non-polar; Hydrophobic |
| Arginine | Arg | R | Positively charged (basic amino acids; non-acidic amino acids); Polar; Hydrophilic; pK=12.5 |
| Asparagine | Asn | N | No charge (non-acidic amino acids); Polar; Hydrophilic |
| Aspartate | Asp | D | Negatively charged (acidic amino acids); Polar; Hydrophilic; pK=3.9 |
| Cysteine | Cys | C | No charge (non-acidic amino acids); Non-polar; Hydrophilic |
| Glutamate | Glu | E | Negatively charged (acidic amino acids); Polar; Hydrophilic; pK=4.2 |
| Glutamine | Gln | Q | No charge (non-acidic amino acids); Polar; Hydrophilic |
| Glycine | Gly | G | No charge (non-acidic amino acids); Non-polar; Hydrophilic |
| Histidine | His | H | Positively charged (basic amino acids; non-acidic amino acids); Polar; Hydrophilic; pK=6.0 |
| Isoleucine | Ile | I | Non-polar; Hydrophobic |
| Leucine | Leu | L | Non-polar; Hydrophobic |
| Lysine | Lys | K | Positively charged (basic amino acids; non-acidic amino acids); Polar; Hydrophilic; pK=10.5 |
| Methionine | Met | M | Non-polar; Hydrophobic |
| Phenylalanine | Phe | F | Non-polar; Hydrophobic |
| Proline | Pro | P | Non-polar; Hydrophobic |
| Serine | Ser | S | No charge (non-acidic amino acids); Polar; Hydrophilic |
| Threonine | Thr | T | No charge (non-acidic amino acids); Polar; Hydrophilic |
| Tryptophan | Trp | W | No charge; Non-polar; Hydrophobic |
| Tyrosine | Tyr | Y | No charge (non-acidic amino acids); Polar; Hydrophilic |
| Valine | Val | V | Non-polar; Hydrophobic |

"Percent (%) amino acid sequence identity" with respect to polypeptide sequences is defined as the percentage of amino acid residues in a candidate sequence that are identical with the amino acid residues in the specific polypeptide sequence, after aligning the sequence and introducing gaps, if necessary, to achieve the maximum percent sequence identity, and not considering any conservative substitutions as part of the sequence identity. Those skilled in the art can determine appropriate parameters for measuring alignment, including any algorithms needed to achieve maximal alignment over the full length of the sequences being compared.

The term "recombinant" as used herein shall mean "being prepared by or the result of genetic engineering". Alternatively, the term "engineered" is used. For example, compounds as described herein, such as a heterodimeric CH3 assembly, a heterodimeric Fc, a binding construct (*e.g*., an antibody), or respective coding nucleic acid molecules, may be engineered from a parent compound (*e.g*., a wild-type sequence) to introduce one or more modifications by recombinant means, such as by recombinant DNA technology, recombinant vectors or recombinant hosts or host cells.

A recombinant polypeptide or protein can be produced by expressing a respective recombinant nucleic acid in a host or host cell, herein also referred to as "recombinant host" or "recombinant host cell". A recombinant host may comprise an expression vector or cloning vector, or it may be genetically engineered to contain a recombinant nucleic acid sequence, in particular employing a nucleotide sequence foreign to the host.

The term "recombinant" with respect to compounds described herein shall particularly include those which are prepared, expressed, created or isolated by recombinant means, such as (a) antibodies isolated from an animal (*e.g*., a mouse) that is transgenic or transchromosomal for human immunoglobulin genes or a hybridoma prepared therefrom, (b) a compound isolated from a host cell transformed with a vector that expresses a respective expression product, *e.g*., from a transfectoma, (c) binding constructs isolated from a recombinant, combinatorial library, and (d) a compound prepared, expressed, created or isolated by any other means that involve splicing of immunoglobulin gene sequences to other DNA sequences. Such recombinant compounds may comprise binding constructs or antibodies, which are engineered to include rearrangements and mutations which occur, for example, during antibody maturation.

Once compounds with the desired structure are engineered, they can be produced by methods well-known in the art, including, for example, recombinant DNA technology, transgenic animals or hybridoma techniques. Specifically, binding constructs described herein can be produced using a suitable method that produces such binding constructs in a recombinant host cell culture.

According to a specific example, recombinant compound described herein, in particular binding constructs or monoclonal antibodies, can be produced by isolating the DNA encoding the polypeptide domains or chains required to assemble the binding construct *e.g.,* antibody domains or chains, transfecting a recombinant host cell with the coding sequences for expression, using well-known recombinant expression vectors, *e.g.* vectors, plasmids or expression cassette(s) comprising the respective coding nucleotide sequences, culturing the recombinant host cell in a host cell culture to express the compounds (*e.g*., the binding constructs or monoclonal antibodies), and isolating the compounds. Recombinant host cells can be selected from suitable prokaryotic and eukaryotic production host cells, as appropriate to produce a compound described herein as expression product.

According to a specific aspect, a nucleotide sequence may be genetically engineered to humanize a a compound described herein, in particular a nucleic acid, a heterodimeric CH3 assembly, a heterodimeric Fc, or a binding construct (*e.g*., an antibody), or to improve its characteristics. For example, an antibody constant region may be engineered to more nearly resemble human constant regions to avoid an undesired immune response, if used in humans. It may also be desirable to genetically manipulate a binding construct sequence to obtain greater affinity to the target. It will be apparent to one of skill in the art that one or more polynucleotide changes can be made to a compound described herein such as a binding construct (or antibody) and still maintain its binding ability to the target.

The compounds described herein, in particular a heterodimeric CH3 assembly, a heterodimeric Fc, a binding construct (*e.g*., an antibody), or a respective coding nucleic acid, may be used as such or used in the preparation of a pharmaceutical for administration to treat a subject in need thereof. In particular the compounds described herein are provided for medical use to treat a subject or patient in need of prophylaxis or treatment of a disease condition.

The term "subject" as used herein shall refer to a warm-blooded mammalian, particularly a human being or a non-human animal. Thus, the term "subject" may also particularly refer to non-human animals such as dogs, cats, rabbits, horses, cattle, pigs and poultry.

The term "patient" includes human and non-human animal subjects that receive either prophylactic or therapeutic treatment. The term "treatment" is thus meant to include both prophylactic and therapeutic treatment.

Specifically, a compound described herein, in particular a heterodimeric CH3 assembly, a heterodimeric Fc, a binding construct (*e.g*., an antibody), or a respective coding nucleic acid, may be purified or is provided in pure or substantially pure form. The term "substantially pure" or "purified" as used herein shall refer to a preparation comprising a compound with a purity of at least 50% (w/w), preferably at least 60%, 70%, 80%, 90% or 95%. Purity can be measured by methods appropriate for the compound (*e.g*. chromatographic methods, polyacrylamide gel electrophoresis, HPLC analysis, and the like).

The term "target" as used herein shall in particular include all targets of a binding construct (herein also referred to as binding molecule), including without limitation target molecules or structures, which are capable of being recognised by a binding site of a binding molecule. A target can be specifically recognized by a binding construct. In particular, a target can be specifically recognized by a binding moiety comprised in a binding construct. The target binding can be a specific binding, meaning that the binding moiety of a binding construct has specificity to bind the target.

A target of a binding construct which is an antibody described herein, is typically understood as an "antigen" or "target antigen". An antibody specifically recognizes a target antigen, in particular an epitope of an antigen. An antibody is capable of binding the specific target by its antigen-binding site (also referred to as "paratope").

A target can be recognized as a whole target molecule or as a fragment of such molecule, especially substructures, *e.g.* a polypeptide or carbohydrate structure of targets, generally referred to as "epitopes" (*e.g*., B-cell epitopes, T-cell epitope), which are immunologically relevant, *i.e.,* are also recognisable by natural or monoclonal antibodies. The term "epitope" as used herein shall in particular refer to a molecular structure which may completely make up a specific binding partner or be part of a specific binding partner to a binding site of binding molecule described herein. The term epitope may also refer to haptens. Chemically, an epitope may either be composed of a carbohydrate, a peptide, a fatty acid, an organic, biochemical or inorganic substance or derivatives thereof and any combinations thereof. If an epitope is a polypeptide, it will usually include at least 3 amino acids, preferably 8 to 50 amino acids, and more preferably between about 10-20 amino acids in the peptide. There is no critical upper limit to the length of the peptide, which could comprise nearly the full length of a polypeptide sequence of a protein. Epitopes can be either linear or conformational epitopes. A linear epitope is comprised of a single segment of a primary sequence of a polypeptide or carbohydrate chain. Linear epitopes can be contiguous or overlapping. Conformational epitopes are comprised of amino acids or carbohydrates brought together by folding of the polypeptide to form a tertiary structure and the amino acids are not necessarily adjacent to one another in the linear sequence. Specifically, epitopes are at least part of diagnostically relevant molecules, *i.e.* the absence or presence of an epitope in a sample is qualitatively or quantitatively correlated to either a disease or to the health status of a patient or to a process status in manufacturing or to environmental and food status. Epitopes may also be at least part of therapeutically relevant molecules, *i.e.,* molecules which can be targeted by the specific binding domain which changes the course of the disease.

Specifically preferred targets of a binding molecule described herein are those targets, which have already been proven to be or are capable of being immunologically or therapeutically relevant, especially those, for which a clinical efficacy has been tested. The term "target" (or "antigen") as used herein shall in particular comprise molecules selected from the group consisting of (human or other animal) tumor associated receptors and soluble tumor associated antigens, which are self-antigens, such as receptors located on the surface of tumor cells or cytokines or growth factors that are abundantly present in the circulation of cancer patients and associated with such tumor. Further targets (or antigens) may be of immune cells, cytokines or cytokine receptors, interferons or interferon receptors, or of pathogen origin, *e.g.* microbial (such as bacterial, viral or parasitic) pathogens.

Specific exemplary targets are naturally-occurring targets, antigens or epitopes, or synthetic (artificial) targets, antigens or epitopes. Artificial targets (or antigens or epitopes) which are derivatives of naturally-occurring ones may have the advantage of an increased antigenicity or stability, which is relevant for being recognized as a binding partner for the specific binding molecule.

The term "therapeutically effective amount", used herein interchangeably with any of the terms "effective amount" or "sufficient amount" of a compound described herein, in particular a heterodimeric CH3 assembly, a heterodimeric Fc, a binding construct (*e.g*., an antibody), or a respective coding nucleic acid, is a quantity or activity sufficient to, when administered to a subject, effect beneficial or desired results, including clinical results, and, as such, an effective amount or synonym thereof depends upon the context in which it is being applied.

An effective amount is intended to mean that amount of a compound that is sufficient to treat, prevent or inhibit such diseases or disorder. In the context of disease, therapeutically effective amounts of a compound described herein are specifically used to treat, modulate, attenuate, reverse, or affect a disease, disorder or disease condition. Specifically, therapeutically effective amounts of a binding construct (*e.g*., an antibody) described herein are specifically used to treat, modulate, attenuate, reverse, or affect a disease, disorder or disease condition that benefits from the interaction of the binding construct with its target.

The amount of the compound that will correspond to such an effective amount will vary depending on various factors, such as the nature of the compound, the pharmaceutical formulation, the route of administration, the type of disease, disorder or disease condition, the identity of the subject or host being treated, and the like, but can nevertheless be routinely determined by one skilled in the art.

Compounds described herein, in particular a heterodimeric CH3 assembly, a heterodimeric Fc, a binding construct (*e.g*., an antibody), or a respective coding nucleic acid, may specifically be used in a pharmaceutical composition. Therefore, a pharmaceutical composition is provided which comprise a compound as described herein and a pharmaceutically acceptable carrier or excipient. Specifically, the pharmaceutical composition is an artificial one *e.g.,* comprising an artificial carrier or excipient which does not naturally occur together with such compound in a body fluid, or which naturally occurs together with such compound, yet is provided in a preparation containing the carrier or excipient in a different amount or ratio.

Pharmaceutical carriers suitable for facilitating a specific mode of administration can be used as well-known in the art. State of the art pharmaceutically acceptable carriers are *e.g.,* described in REMINGTON'S PHARMACEUTICAL SCIENCES.

Pharmaceutically acceptable carriers generally include any and all suitable solid or liquid substances, solvents, dispersion media, coatings, isotonic and absorption delaying agents, and the like that are physiologically compatible with a compound described herein. Further examples of pharmaceutically acceptable carriers include sterile water, saline, phosphate buffered saline, dextrose, glycerol, ethanol, and the like, as well as combinations of any of the forgoing.

In one such aspect, a compound described herein can be combined with one or more carriers appropriate for a desired route of administration. For example, a compound described herein may be, *e.g.* admixed with any one or more of lactose, sucrose, starch, cellulose esters of alkanoic acids, stearic acid, talc, magnesium stearate, magnesium oxide, sodium and calcium salts of phosphoric and sulphuric acids, acacia, gelatin, sodium alginate, polyvinylpyrrolidine, polyvinyl alcohol, and optionally further tableted or encapsulated for conventional administration. Alternatively, a compound described herein may be dissolved in saline, water, polyethylene glycol, propylene glycol, carboxymethyl cellulose colloidal solutions, ethanol, corn oil, peanut oil, cotton-seed oil, sesame oil, tragacanth gum, and/or various buffers. Other carriers, adjuvants, and modes of administration are well-known in the pharmaceutical arts. A carrier may include a controlled release material or time delay material, such as glyceryl monostearate or glyceryl distearate alone or with a wax, or other materials well known-in the art that are suitably used in a retard formulation.

Liquid formulations can be solutions, emulsions or suspensions and can include excipients such as suspending agents, solubilizers, surfactants, preservatives, and chelating agents. Compounds disclosed herein may also be formulated as immunoliposomes, and/or entrapped in microcapsules.

Stable formulations are prepared for storage by mixing compounds with a desired degree of purity with pharmaceutically acceptable carriers, excipients or stabilizers *e.g.,* in the form of lyophilized formulations or aqueous solutions.

Pharmaceutical compositions are contemplated wherein a compound described herein, in particular a heterodimeric CH3 assembly, a heterodimeric Fc, a binding construct (*e.g*., an antibody), or a respective coding nucleic acid, are formulated with one or more therapeutically active agents.

Formulations used for *in vivo* administration are specifically sterile, preferably in the form of a sterile aqueous solution. This is readily accomplished by filtration through sterile filtration membranes or other methods of sterilization.

Administration of a pharmaceutical composition comprising a compound described herein, may be done in a variety of ways, including orally, subcutaneously, intravenously, intranasally, intraotically, transdermally, mucosal, topically, *e.g.,* gels, salves, lotions, creams, *etc.,* intraperitoneally, intramuscularly, intrapulmonary, vaginally, parenterally, rectally, or intraocularly.

According to a preferred aspect, a pharmaceutical composition described herein is administered parenterally *e.g.,* as a bolus injection, infusion or by continuous infusion. Exemplary formulations as used for parenteral administration include those suitable for subcutaneous, intramuscular or intravenous injection, such as, for example, a solution, emulsion or suspension.

As used herein, the term "specificity" or "specific binding" refers to a binding reaction which is determinative of the cognate ligand of interest in a heterogeneous population of molecules. Thus, under designated conditions (*e.g*. immunoassay conditions), the binding molecule described herein binds to its particular target and does not bind in a significant amount to other molecules present in a sample. The specific binding means that binding is selective in terms of target identity, high, medium or low binding affinity or avidity, as selected. Selective binding is usually achieved if the binding constant or binding dynamics is at least 10-fold different, preferably the difference is at least 100-fold, and more preferred at least 1000-fold.

The term "variant" as used herein shall specifically include any "mutant", "homolog", or "derivative". It is particularly understood that that the term variant shall also include derivatives. The term "variant" shall specifically encompass functionally active variants which are characterized by a certain functionality.

The term "variant" of a a compound described herein, in particular a nucleic acid, a heterodimeric CH3 assembly, a heterodimeric Fc, or a binding construct (*e.g*., an antibody), shall particularly refer mutants or fragments of a compound, *e.g.* obtained by mutagenesis methods, in particular to delete, exchange, introduce one or more amino acids into a specific amino acid sequence, such as to modulate target binding properties, thermostability, effector function, half-life, heterodimerization, *etc.* Any of the known mutagenesis methods may be employed, including point mutations at desired positions *e.g.,* obtained by site-directed mutagenesis or randomization techniques. In some cases, positions are chosen randomly, *e.g.* with either any of the possible amino acids or a selection of preferred amino acids to randomize the antibody sequences. The term "mutagenesis" refers to any art recognized technique for altering a polynucleotide or polypeptide sequence. Preferred types of mutagenesis include error prone PCR mutagenesis, saturation mutagenesis, or other site directed mutagenesis.

Specific variants of a compound described herein such as antibodies are mutants which comprise mutations in the constant domains *e.g.,* to engineer the antibody stability, effector function or half-life, or in the variable domains to improve antigen-binding properties *e.g.,* by affinity maturation techniques available in the art.

The term "functional variant" or "functionally active variant" as used herein for a compound described herein, in particular a nucleic acid, a heterodimeric CH3 assembly, a heterodimeric Fc, or a binding construct (*e.g*., an antibody), is understood as a variant comprises a modification which does not affect, in particular impair, a certain functionality of the binding construct.

The functionality of a binding construct described herein is particularly characterized by a certain target binding and CD89 binding property, as further described herein. Specifically, a functionally active binding construct comprises an effector function mediated by FcαR binding, in particular ADCC using neutrophils as effector cells.

According to specific examples, a functional variant of a binding construct described herein can be a variant comprising a modification of the binding moiety and/or of the Fc described herein. In the case of a modification of the binding moiety having specificity to bind a selected target, the functionally active variant of a binding construct would still have the predetermined binding specificity, though this could be changed *e.g.,* to change the fine specificity to a specific epitope, the affinity, the avidity, the Kon or Koff rate, *etc.* For example, an affinity matured antibody is specifically understood as a functionally active variant of an antibody (of a parent antibody). In case of a modification of the Fc comprising a certain Fc effector function, the functionally active variant of a binding construct would still have the predetermined FcαR effector function that is mediated by CD89 binding, though this could be changed *e.g.,* to increase or reduce the FcαR effector function. A functionally active variant comprising an Fc modification may have FcγR effector function or modulated FcγR effector function, such as to increase or to reduce ADCC, or ADCP. A functionally active variant comprising an Fc modification may have FcRn-mediated function or modulated FcRn-mediated function, such as to increase or to reduce the half-life of the binding construct.

The functional activity of a binding construct described herein can be determined by suitable standard assays to determine the structure and function of a variant comprising a modification as compared to a parent molecule. For example, functionality can be determined by comparing the specificity of binding a target and/or an Fc effector function of a binding construct with or without the modification. The functional activity of a binding construct or an antibody in terms of target (or antigen)-binding is typically determined in an ELISA assay, BIAcore assay, Octet BLI assay, or FACS based assay when the target (or antigen) is expressed on cell surface.

Functionally active variants of a binding construct described herein may be obtained, *e.g.* by changing the sequence of a parent of a binding construct *e.g.,* a monoclonal antibody having a specific native structure of an antibody, such as an IgG1 structure, to obtain a variant having the same specificity in recognizing a target antigen, but having a structure which differs from the parent structure *e.g.,* to modify any of the antibody domains to introduce specific mutations, to produce bispecific constructs, or to produce a fragment of the parent molecule.

According to a specific example, a parent binding construct or sequence thereof is modified to produce variants (in particular, functional variants) which incorporate mutations at a certain sequence position or region besides the target-binding site, or within the target-binding site, that do not impair the specificity of target binding, and preferably would result in a biological activity similar to the parent binding construct, including the ability to bind the target *e.g.,* with substantially the same biological activity, as determined by a specific binding assay or a respective functional test. Similarly, mutations at a certain sequence position or region besides the CD89-binding site or within the CD89-binding site can be introduced, which do not impair the CD89 binding, and preferably would have a biological activity similar to the parent binding construct, including the ability to bind CD89 *e.g.,* with substantially the same biological activity, as determined by a FcαR effector function assay.

The term "substantially the same biological activity" as used herein refers to the activity as indicated by substantially the same activity being at least 20%, at least 50%, at least 75%, at least 90%, *e.g.* at least 100%, or at least 125%, or at least 150%, or at least 175%, or *e.g.* up to 200% of the activity as determined for the comparable or parent binding construct.

According to specific examples, variants of a binding construct described herein are functionally active with regard to the specificity of target binding, preferably which have a potency to specifically bind the target, and not significantly bind to other targets *e.g.,* with a KD value difference of at least 2 logs, preferably at least 3 logs. The affinity of target binding by a functionally active variant is typically not impaired, corresponding to about substantially the same binding affinity as the parent binding construct *e.g.,* with a KD value difference of less than 2 logs, preferably less than 3 logs, however, it is possible that the affinity of a functionally active variant is even improved, *e.g.* with a KD value difference of at least 1 log, preferably at least 2 logs or at least 3 logs.

Specific examples of a functionally active variant of a parent binding construct can be any of the following:
a) a biologically active fragment of the binding construct, the fragment comprising at least 50% of the sequence of the molecule, preferably at least 60%, at least 70%, at least 80%, at least 90%, or at least 95% and most preferably at least 97%, 98% or 99%;
b) a mutant of the binding construct by at least one amino acid substitution, addition and/or deletion, wherein the functionally active variant has a sequence identity to the molecule or part of it, such as an antibody of at least 50% sequence identity, preferably at least 60%, more preferably at least 70%, more preferably at least 80%, still more preferably at least 90%, even more preferably at least 95% and most preferably at least 97%, 98% or 99%; and/or
c) a fusion of the binding construct with at least one amino acid or nucleotide that is heterologous to the binding construct;

According to a specific example, a functionally active variant of the binding construct described herein may comprise a modification to incorporate a homologous sequence of a different binding construct (*e.g*., antibody, antibody type, or antibody subtype), or originating from a different species, such as to produce a chimeric or humanized binding construct.

A a compound described herein, in particular a heterodimeric CH3 assembly, a heterodimeric Fc, or a binding construct (*e.g*., an antibody), may be glycosylated or unglycosylated. The term "variant" as used herein shall also apply to binding constructs with a certain glycosylation pattern, *e.g.* produced by glycoengineering, which are functional and may serve as functional equivalents, *e.g.* specifically binding to the same targets and with functional properties as described herein. For example, a compound described herein may be expressed in an appropriate mammalian cell to allow a specific glycosylation of the compound which is determined by the host cell expressing the binding construct.

The term "functionally active variant" also includes naturally occurring allelic variants, as well as mutants or any other non-naturally occurring variants. As is known in the art, an allelic variant is an alternate form of a (poly) peptide that is characterized as having a substitution, deletion, or addition of one or more amino acids that does essentially not alter the biological function of the polypeptide.

Variants or functionally active variants can be obtained by sequence alterations in the polypeptide or the nucleotide sequence *e.g.,* by one or more point mutations. The sequence alterations may retain or improve a function of the unaltered polypeptide or the nucleotide sequence. Such sequence alterations can include substitutions, additions, deletions, insertions, or other types of mutations, or a combination of any of the foregoing, which sequence alterations result in one or more point mutations. Specific sequence alterations can include can include or consist of conservative substitutions, additions, deletions, or insertions, or combinations of any of the foregoing, which sequence alterations result in one or more point mutations.

Conservative substitutions are those that take place within a family of amino acids that are related in their side chains and chemical properties. Examples of such families are amino acids with basic side chains, with acidic side chains, with non-polar aliphatic side chains, with non-polar aromatic side chains, with uncharged polar side chains, with small side chains, with large side chains *etc.*

The present invention is based on the finding that bispecific heterodimeric IgA-based antibodies (as well as IgG-based antibodies) could be engineered using the strand-exchanged engineered domain (SEED) technology, wherein each of the CH3 domains comprises corresponding intertwined segments of IgA and IgG. SEED heterodimers have so far been described in antibodies incorporating IgG-CH1 and CH2 domains including the hinge region.

It was surprisingly possible to engineer such antibodies to introduce two *de novo* binding sites (in particular, one per CH3 domain) which enable the interaction of the antibody Fc with the myeloid cell-activating receptor CD89 (FcαR). It was shown that SEED-type heterodimeric CH3 domains could be expressed as a part of IgA scaffold and such molecules could be further engineered to incorporate a CD89 binding sites in each of the CH3 domains, thereby inciting neutrophil-mediated effector functions. These antibodies exhibited good biophysical properties and thermostability.

When using the engineered bispecific antibodies which specifically bind to a target antigen on the surface of target antigen-positive cells, a significant interaction between the cells and a CD89-positive cell line has been demonstrated. According to a specific example, SEED-IgA antibodies specifically binding to the tumor antigens epidermal growth factor receptor (EGFR) and receptor tyrosine kinase like orphan receptor 1 (ROR1, could be engineered to incorporate two CD89 binding sites in the CH3 domains and has induced neutrophil-mediated target cell killing which is mediated by CD89 binding. Binding capacities to the target antigens recognized by variable domains (according to this specific example, EGFR and ROR1), was not impaired, and in contrast to the SEED-IgA antibodies without such engineering to incorporate the CD89 binding sites, antibodies engineered as described herein could bind to CD89-expressing cells, mediate tumor cell line-effector cell clustering and induce neutrophil-mediated specific lysis of tumor cells.

It has been proven that the heterodimeric CH3 assembly described herein can be successfully incorporated into various SEED antibody scaffolds, even in the IgA scaffold, such as the SEED-IgA1 and -IgA2 scaffolds. Mutants expressed in human cells *e.g*., HEK-expressed IgA-SEED mutants comprising the heterodimeric CH3 assembly described herein, were shown to comprise a CH2-linked N-glycan pattern more similar to wild-type IgA, with reduced core fucosylation in comparison with IgA-SEED comprising unmodified IgA-CH3 domains. When using the heterodimeric CH3 assembly described herein in larger binding molecules or constructs, mobilization of CD89-positive effector cells can be combined with the flexibility of incorporating target binding specificities, which is a promising basis for biochemically stable multispecific binding molecules with high therapeutic potential.

The invention specifically provides for exemplified compounds, such as exemplary heterodimeric CH3 assemblies, heterodimeric Fcs, binding constructs (in particular bispecific antibodies), or respective coding nucleic acids, as used in the examples provided herein. Further compounds are feasible *e.g.,* including functional variants of the exemplified compounds *e.g.,* where the Fc is further engineered to improve the structure and function of the binding constructs (the antibodies), or where antibodies with other antigen-binding sites are produced, which specifically recognize one or more antigens (or epitopes) of choice.

The invention if further described by one or more of the following items.
1. A heterodimeric CH3 assembly of a first and second CH3 domain of the IgG-type, wherein each of the CH3 domains comprises a dimerization sheet, each dimerization sheet being engineered to comprise alternating IgA and IgG segments, wherein the segments of the first CH3 domain are dimerized to the respective segments of the second CH3 domain, wherein either one or both of the CH3 domains is further engineered to incorporate a CD89 binding site.
2. The CH3 assembly of item 1, wherein both of the first and second CH3 domains comprise the CD89 binding site.
3. The CH3 assembly of item 1 or 2, wherein the first and second CH3 domains form heterodimers preferentially over forming homodimers.
4. The CH3 assembly of any one of items 1 to 3, wherein the CH3 domains are of human IgG3, IgG1, IgG2, or IgG4.
5. The CH3 assembly of any one of items 1 to 4, wherein the alternating IgA and IgG segments originate from corresponding segments of naturally-occurring dimerization sheets of respective IgA and IgG CH3 domains.
6. The CH3 assembly of any one of items 1 to 5, wherein
   a) the first CH3 domain comprises or consists of SEQ ID NO:37 or SEQ ID NO:39, which is engineered to comprise the point mutations
      i) E40R, G45S, P47E, S89M, M91G, and
      ii) optionally E42L, and/or one or both of I37V and A38L; and
      iii) optionally H96P, N97L, H98A, Y99F;
         and
   b) the second CH3 domain comprises or consists of SEQ ID NO:38 or SEQ ID NO:40, which is engineered to comprise the point mutations
      i) E40R, S89M, M91G, and
      ii) optionally one or both of I37V and A38L; and
      iii) optionally H96P, N97L, H98A, Y99F.
7. The CH3 assembly of any one of items 1 to 6, wherein
   a) the first CH3 domain comprises or consists of SEQ ID NO:37, which is engineered to comprise the point mutations
      i) E40R, G45S, P47E, S89M, M91G, and
      ii) optionally E42L, and/or one or both of I37V and A38L; and
      iii) optionally H96P, N97L, H98A, Y99F;
         and
   b) the second CH3 domain comprises or consists of SEQ ID NO:38, which is engineered to comprise the point mutations
      i) E40R, S89M, M91G, and
      ii) optionally one or both of I37V and A38L; and
      iii) optionally H96P, N97L, H98A, Y99F.
8. The CH3 assembly of any one of items 1 to 6, wherein
   a) the first CH3 domain comprises or consists of SEQ ID NO:39, which is engineered to comprise the point mutations
      i) E40R, G45S, P47E, S89M, M91G, and
      ii) optionally E42L, and/or one or both of I37V and A38L; and
      iii) optionally H96P, N97L, H98A, Y99F;
         and
   b) the second CH3 domain comprises or consists of SEQ ID NO:40, which is engineered to comprise the point mutations
      i) E40R, S89M, M91G, and
      ii) optionally one or both of I37V and A38L; and
      iii) optionally H96P, N97L, H98A, Y99F.
9. The CH3 assembly of any one of items 1 to 5, wherein
   a) the first CH3 domain comprises or consists of SEQ ID NO:37 or SEQ ID NO:39, which is engineered to comprise the point mutations
      i) E40R, G45S, P47E, S89M, M91G, and
      ii) optionally E42L, and/or one or both of I37V and A38L; and
      iii) optionally H96P, N97L, H98A, Y99F;
         and
   b) the second CH3 domain comprises or consists of SEQ ID NO:38 or SEQ ID NO:40.
10. The CH3 assembly of any one of items 1 to 5 or 9, wherein
   a) the first CH3 domain comprises or consists of SEQ ID NO:37, which is engineered to comprise the point mutations
      i) E40R, G45S, P47E, S89M, M91G, and
      ii) optionally E42L, and/or one or both of I37V and A38L; and
      iii) optionally H96P, N97L, H98A, Y99F;
         and
   b) the second CH3 domain comprises or consists of SEQ ID NO:38.
11. The CH3 assembly of any one of items 1 to 5 or 9, wherein
   a) the first CH3 domain comprises or consists of SEQ ID NO:39, which is engineered to comprise the point mutations
      i) E40R, G45S, P47E, S89M, M91G, and
      ii) optionally E42L, and/or one or both of I37V and A38L; and
      iii) optionally H96P, N97L, H98A, Y99F;
         and
   b) the second CH3 domain comprises or consists of SEQ ID NO:40.
12. The CH3 assembly of any one of items 1 to 5, wherein
   a) the first CH3 domain comprises or consists of SEQ ID NO:37 or SEQ ID NO:39; and
   b) the second CH3 domain comprises or consists of SEQ ID NO:38 or SEQ ID NO:40, which is engineered to comprise the point mutations
      i) E40R, S89M, M91G, and
      ii) optionally one or both of I37V and A38L; and
      iii) optionally H96P, N97L, H98A, Y99F.
13. The CH3 assembly of any one of items 1 to 5 or 12, wherein
   a) the first CH3 domain comprises or consists of SEQ ID NO:37;
      and
   b) the second CH3 domain comprises or consists of SEQ ID NO:38, which is engineered to comprise the point mutations
      i) E40R, S89M, M91G, and
      ii) optionally one or both of I37V and A38L; and
      iii) optionally H96P, N97L, H98A, Y99F.
14. The CH3 assembly of any one of items 1 to 5 or 12, wherein
   a) the first CH3 domain comprises or consists of SEQ ID NO:39;
      and
   b) the second CH3 domain comprises or consists of SEQ ID NO:40, which is engineered to comprise the point mutations
      i) E40R, S89M, M91G, and
      ii) optionally one or both of I37V and A38L; and
      iii) optionally H96P, N97L, H98A, Y99F.
15. The CH3 assembly of any one of items 6 to 14, wherein said engineering is to comprise the I37V and A38L point mutations.
16. The CH3 assembly of any one of items 6 to 15, wherein said engineering is to comprise the H96P, N97L, H98A, Y99F point mutations.
17. The CH3 assembly of any one of items 6 to 15, wherein only one of the first and second CH3 domains comprises the H96P, N97L, H98A, Y99F point mutations.
18. The CH3 assembly of any one of items 1 to 6, wherein the first CH3 domain comprises the amino acid sequence of any one of SEQ ID NO:1 to 24, and the second CH3 domain comprises the amino acid sequence of SEQ ID NO:25 to 36.
19. The CH3 assembly of any one of items 1 to 6 or 18, which comprises a combination of the first and second CH3 domains, selected from any one of the following:
   a) the first CH3 domain comprises or consists of SEQ ID NO:2; and the second CH3 domain comprises or consists of SEQ ID NO: 26;
   b) the first CH3 domain comprises or consists of SEQ ID NO:14; and the second CH3 domain comprises or consists of SEQ ID NO:32;
   c) the first CH3 domain comprises or consists of SEQ ID NO:4; and the second CH3 domain comprises or consists of SEQ ID NO: 26;
   d) the first CH3 domain comprises or consists of SEQ ID NO:16; and the second CH3 domain comprises or consists of SEQ ID NO: 32;
   e) the first CH3 domain comprises or consists of SEQ ID NO:6; and the second CH3 domain comprises or consists of SEQ ID NO: 28;
   f) the first CH3 domain comprises or consists of SEQ ID NO:18; and the second CH3 domain comprises or consists of SEQ ID NO: 34;
   g) the first CH3 domain comprises or consists of SEQ ID NO:6; and the second CH3 domain comprises or consists of SEQ ID NO: 28;
   h) the first CH3 domain comprises or consists of SEQ ID NO:18; and the second CH3 domain comprises or consists of SEQ ID NO: 36;
   i) the first CH3 domain comprises or consists of SEQ ID NO:10; and the second CH3 domain comprises or consists of SEQ ID NO: 28;
   j) the first CH3 domain comprises or consists of SEQ ID NO:22; and the second CH3 domain comprises or consists of SEQ ID NO: 34;
   k) the first CH3 domain comprises or consists of SEQ ID NO:12; and the second CH3 domain comprises or consists of SEQ ID NO: 28;
   l) the first CH3 domain comprises or consists of SEQ ID NO:24; and the second CH3 domain comprises or consists of SEQ ID NO: 34;
   m) the first CH3 domain comprises or consists of SEQ ID NO:8; and the second CH3 domain comprises or consists of SEQ ID NO: 30;
   n) the first CH3 domain comprises or consists of SEQ ID NO:20; and the second CH3 domain comprises or consists of SEQ ID NO: 36.
20. A heterodimeric Fc comprising dimerized Fc chains of CH2 and CH3 domains, wherein the CH3 domains comprise the CH3 assembly of any one of items 1 to 19.
21. The Fc of item 20, wherein the CH2 domains are of the IgA type, preferably IgA2 or IgA1, of the IgG type, preferably IgG3, IgG1, or IgG2, or of the IgE type, preferably wherein the CH2 domains comprise an amino acid sequence independently selected from an amino acid sequence comprising at least 90% sequence identity to any one of SEQ ID NO:43 to 48.
22. The Fc of item 20 or 21, which comprises a first Fc chain comprising any one of SEQ ID NO:49-53; and a second Fc chain comprising any one of SEQ ID NO:54-56.
23. The Fc of any one of items 20 to 22, comprising a combination of a first and second Fc chain, which is selected from any one of the following:
   a) the first Fc chain comprises or consists of SEQ ID NO:49; and the second Fc chain comprises or consists of SEQ ID NO: 54;
   b) the first Fc chain comprises or consists of SEQ ID NO:49; and the second Fc Fc chain comprises or consists of SEQ ID NO: 55;
   c) the first Fc chain comprises or consists of SEQ ID NO:50; and the second Fc chain comprises or consists of SEQ ID NO: 54;
   d) the first Fc chain comprises or consists of SEQ ID NO:52; and the second Fc chain comprises or consists of SEQ ID NO: 54;
   e) the first Fc chain comprises or consists of SEQ ID NO:51; and the second Fc chain comprises or consists of SEQ ID NO: 55.
24. A binding construct comprising one or more binding moieties and an Fc of any one of items 20 to 23, preferably comprising at least two binding moieties with different binding specificities.
25. The construct of 24, which comprises at least two binding moieties, wherein a first binding moiety is bound to a one Fc chain, and a second binding moiety is bound to the other Fc chain.
26. The construct of item 24 or 25, comprising at least one hinge or linker, which links a binding moiety to the N-terminus of an Fc chain, preferably wherein the hinge originates from an IgA or IgG antibody.
27. The construct of any one of items 24 to 26, wherein the binding moiety comprises an antigen-binding moiety, an enzyme, a substrate, a cytokine, a cytokine-binding moiety, a receptor, or a ligand, preferably wherein the antigen-binding moiety comprises an antibody variable region, which comprises or consists of one or more antibody domains including at least one antibody variable domains, preferably any one or more of a VL domain, a VH domain, a VHH domain, Fv, scFv, diabody, Fab, a scFab, Fab', Fab₂, Fab₃, F(ab')₂; sdAb, diabody, triabody, tetrabody, minibody, nanobody, maxibody, tandab, DVD, BiTe, TandAb, or a combination of any of the foregoing.
28. The construct of any one of items 24 to 27, which is a multispecific antibody, preferably a bispecific, trispecific, or tetraspecific antibody, or a one-armed antibody.
29. The construct of any one of items 24 to 28, which has antibody-dependent cytotoxicity (ADCC) using neutrophils as effector cells.
30. Nucleic acid molecules encoding the CH3 assembly of any one of items 1 to 19, or the Fc of any one of items 20 to 23, or the construct of any one of items 24 to 29.

The foregoing description will be more fully understood with reference to the following examples. Such examples are, however, merely representative of methods of practicing one or more embodiments of the present invention and should not be read as limiting the scope of invention.

### EXAMPLES

### Example 1: IgA-SEED variants

### a) Molecular design

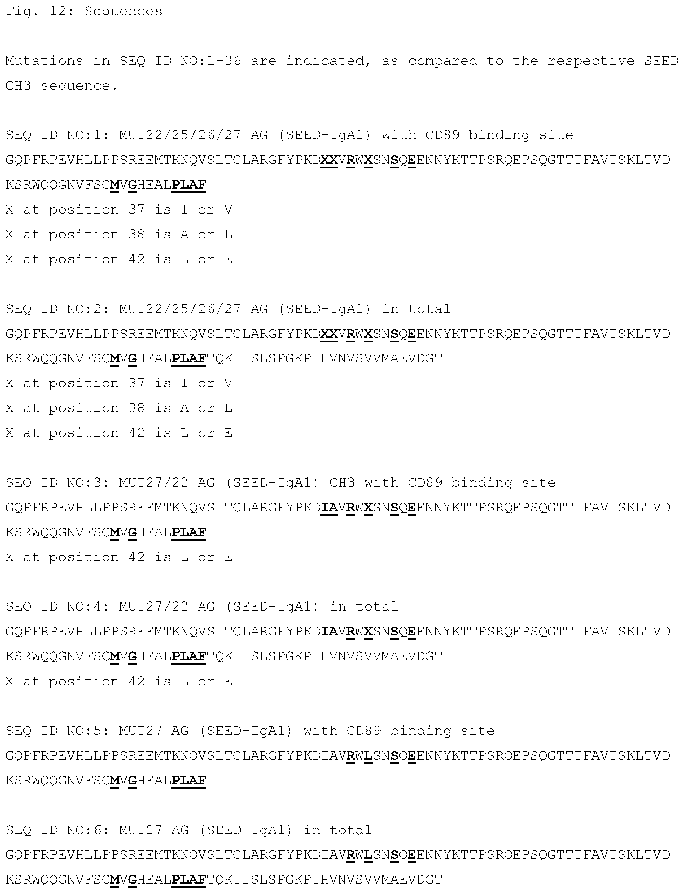

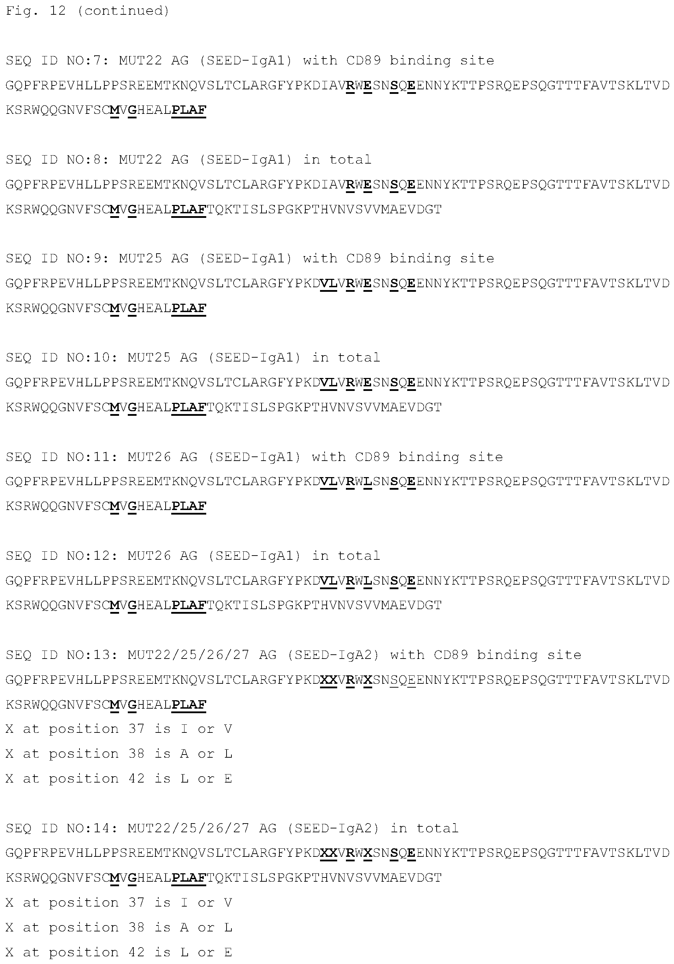

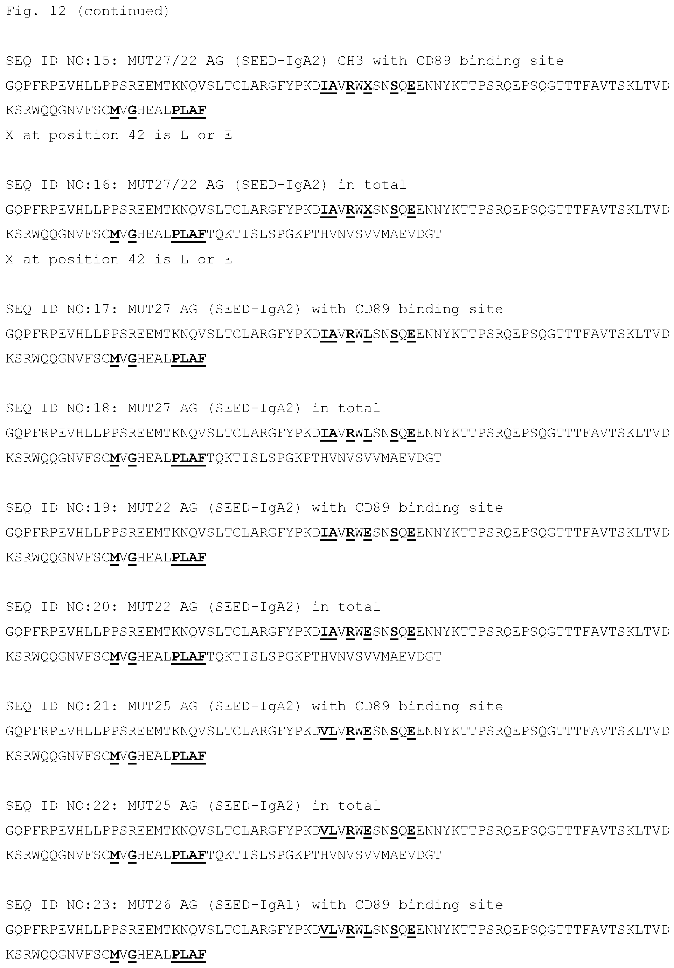

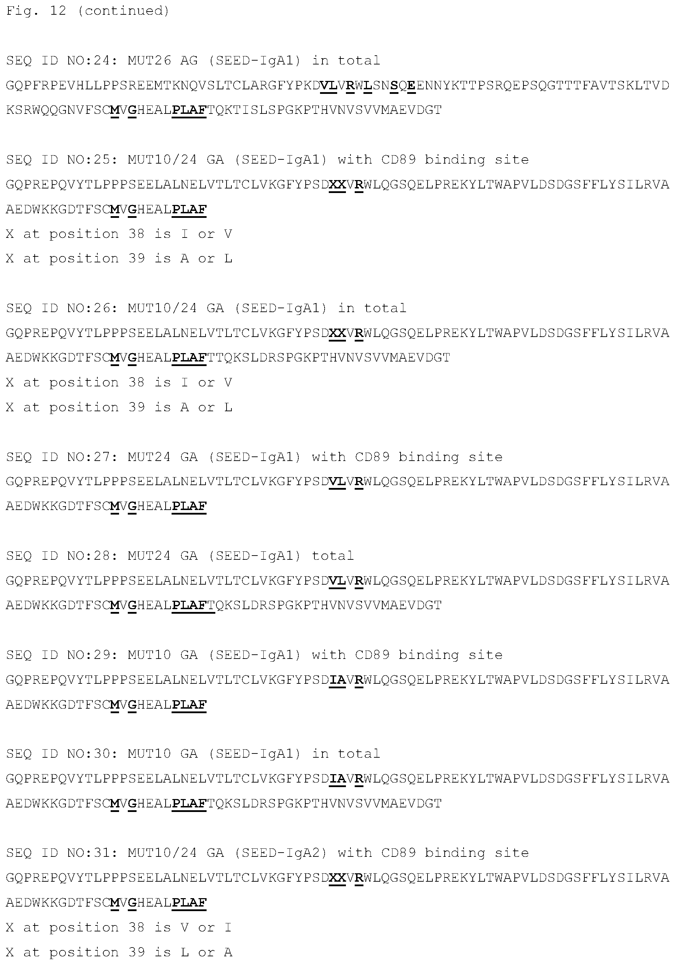

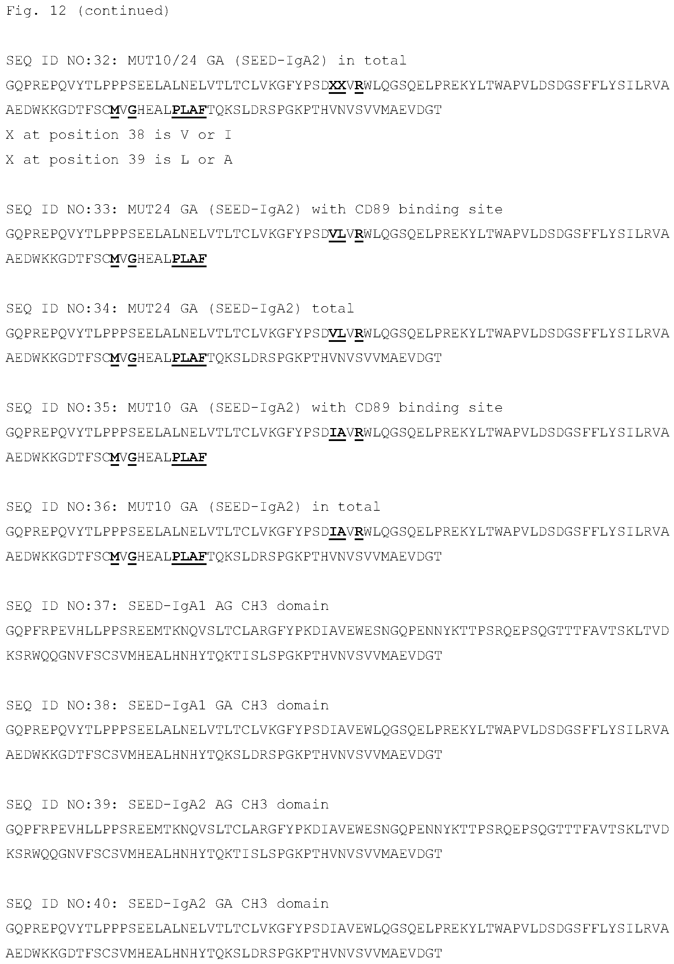

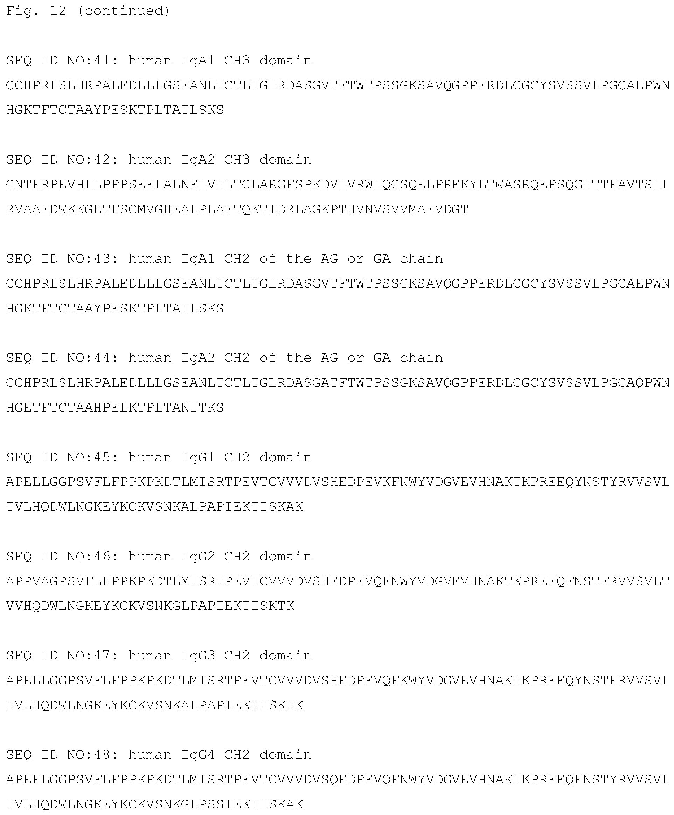

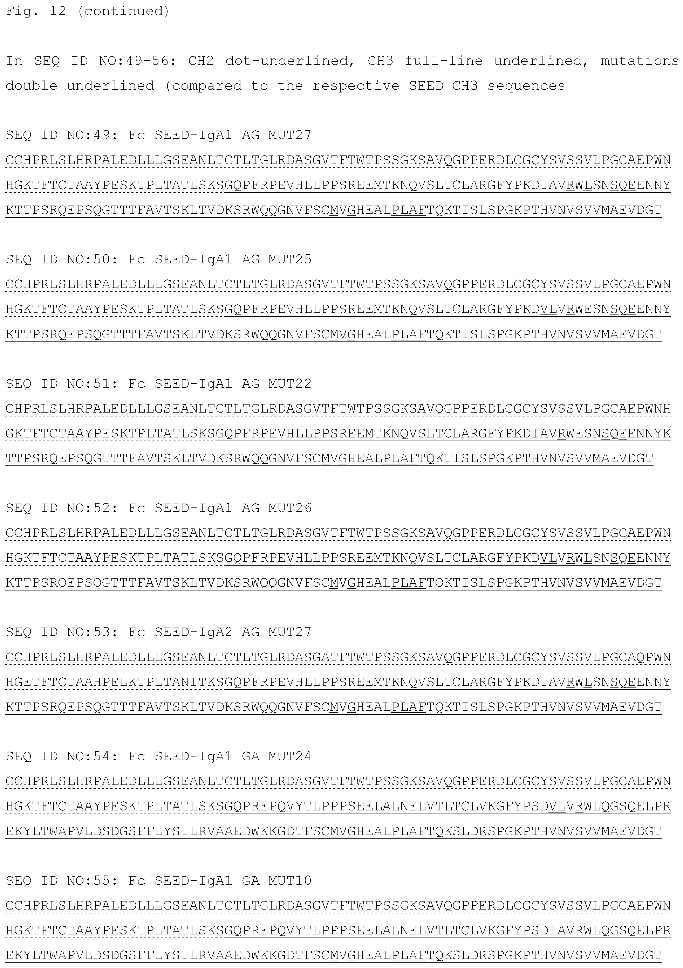

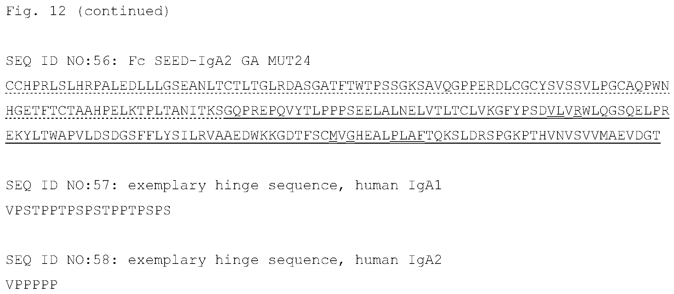

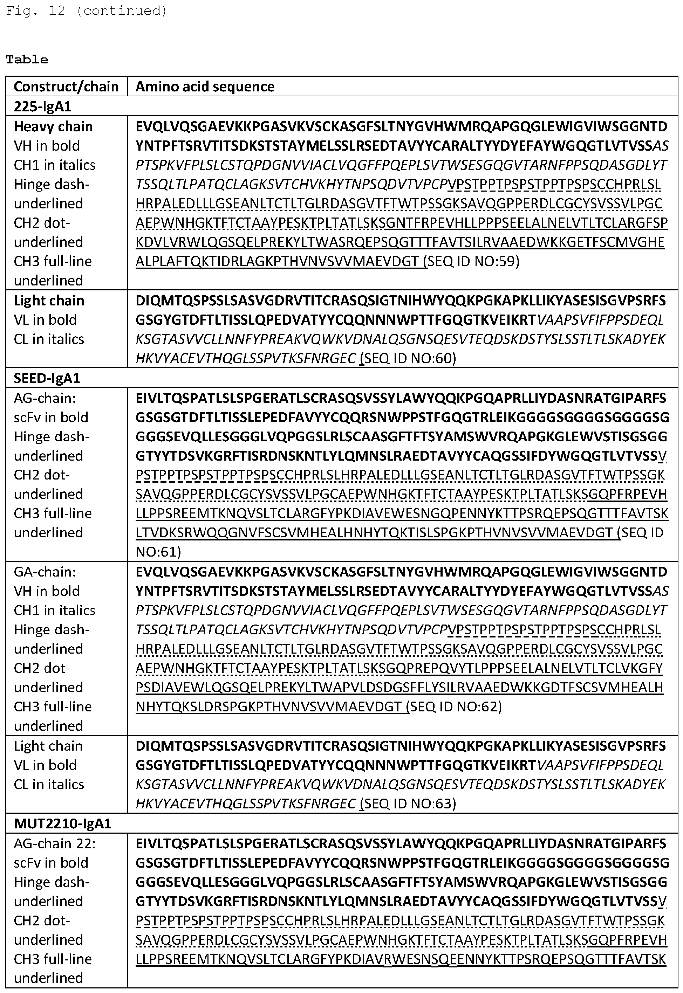

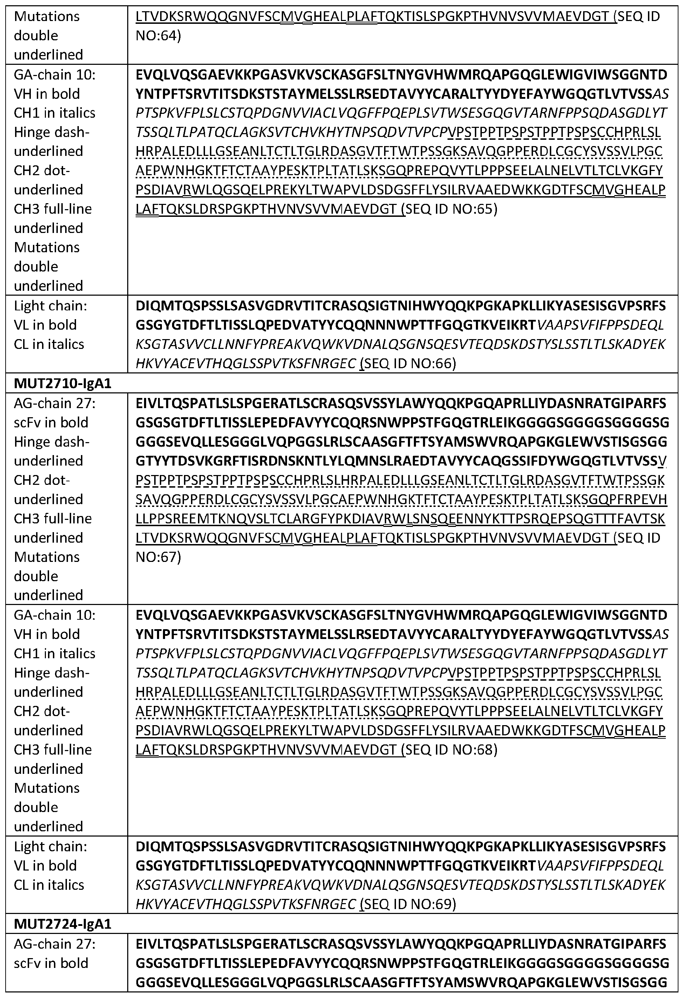

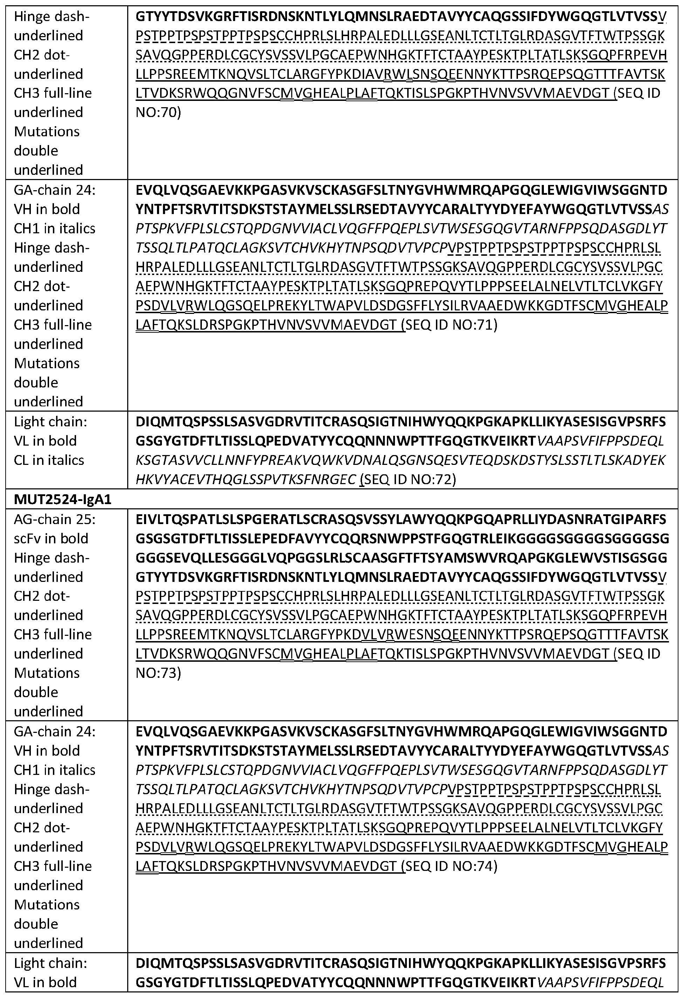

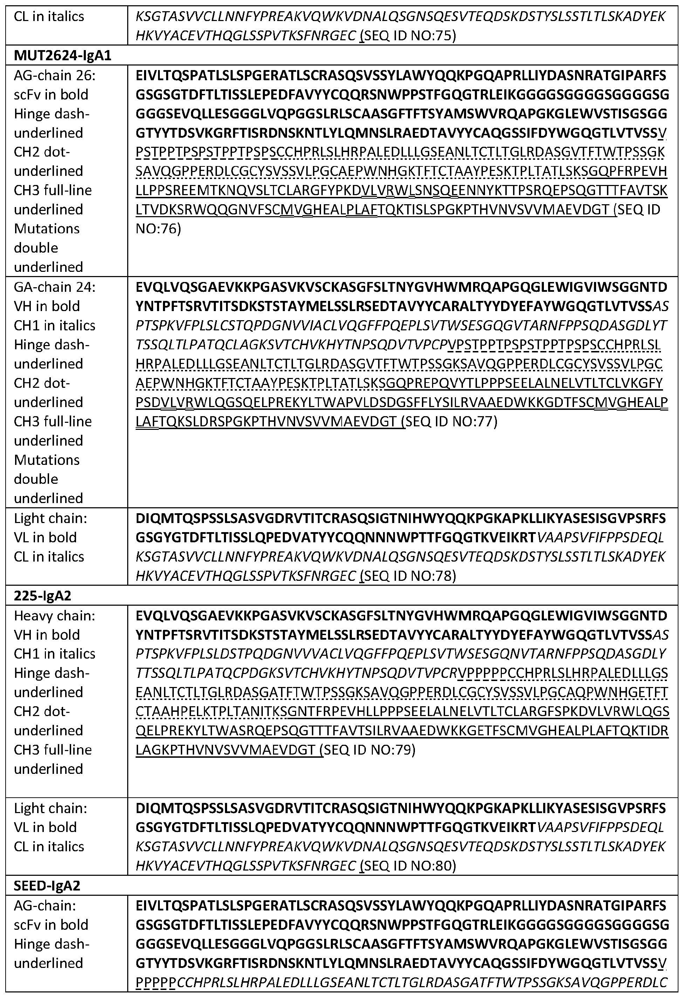

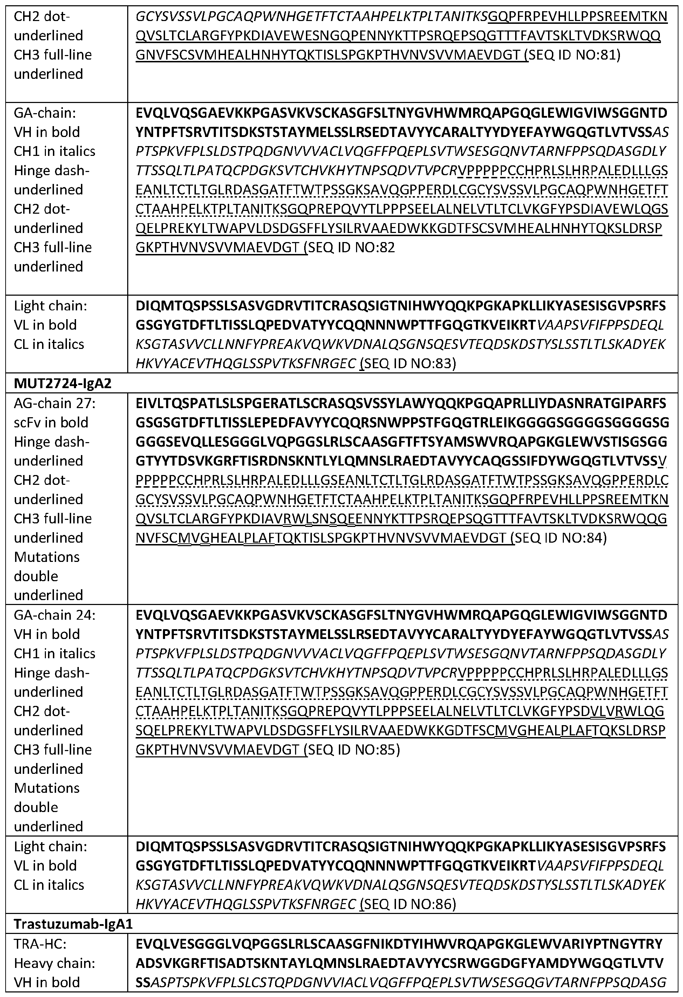

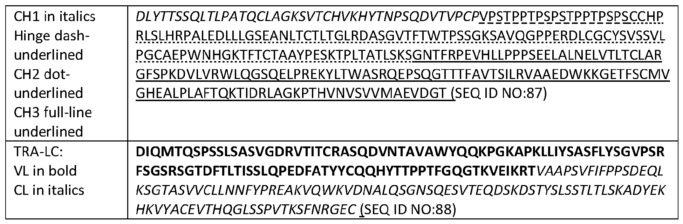

In the initial constructs, anti-epidermal growth factor receptor (EGFR)-Fab 225 and anti-ROR1 scFv H082 were expressed fused with IgACH1, hinge, and CH2 domain, and SEED GA- and AG-domain, respectively (sequences of all constructs in Figure 12). EGFR is a validated tumor target, addressed by several blockbuster therapeutic antibodies such as cetuximab and panitumomab. The expression of ROR1 (receptor tyrosine kinase-like orphan receptor 1) correlates positively with the progression of several blood and solid malignancies, and potentiates EGF-induced EGFR-signaling. Although the heterodimeric CH3 domains contain contigs corresponding to IgA sequence, the residues discovered to be important for CD89 interaction are modified. The prominent interaction contig P93_L94_A95_F96 (P433_L434_A435_F436, EU numbering) is replaced for HNHY (SEQ ID NO:90) in both SEED chains (Davis et al, 2010) with the purpose of retaining the neonatal Fc receptor (FcRn) binding (numbering of the CH3 domains are as proposed in Davis et al 2010, and in Figure 1a). Further, in IgA complexed with CD89, the CH3-domain residues E90, L94, F96, M86, E47, S45 and R40 (E430, L434, F436, M428, E387, S385 and R380, EU numbering) have been shown to play an important role for the interaction (Posgai et al. 2018, Proc Natl Acad Sci U S A;115:E8882-91). The residues whose mutagenesis could lead to an improved interaction of SEED CH3-domains with CD89 were initially targeted, using homology modeling in an overlay of the structures PDB:1OW0 (Herr et al. Nature 2003;423:614-20) and PDB:1OQ0 and amino acids interacting with CD89 within a distance of 4 Å were searched to be reversely mutated.

In the SEED GA-chain, the stretch of residues H93_N94_H95_Y96 has been altered to P93_L94_A95_F96 (Figure 1b) (P433_L434_A435_F436, EU numbering). S86 (S426, EU numbering) has been exchanged for M as the mutagenesis of this residue to A has been reported to render the IgA about 100-fold less active in binding to CD89. Also, M88 (M428, EU numbering) has been altered to G, as it could otherwise significantly impact the mobility of L257 and L258 in the CH2 domain (numbering for the IgA sequence is according to the myeloma IgA1 protein (Bur) scheme) (Putnam et al. J Biol Chem 1979;254:2865-74). E40 (E380, EU numbering) has been mutated to R to counter charge reversal and restore the stabilizing contacts with a part of the N263-linked glycan that can be expected judging from the crystal structure.

In the SEED AG-chain, the same changes have been introduced. Additionally, G45 (G385, EU numbering) was changed to S and P47 (P387, EU numbering) to E as both are positioned within 4 Å from L54 in CD89. Specifically, E47 forms a polar contact with a galactose residue on the N263-linked glycan. At this point, an antibody containing all these mutations (MUT2210) was produced and indeed a weak interaction with CD89 could be detected in a biolayer interferometry experiment (Figure 7).

Moreover, the AG-chain was modified in that the E42 (E382, EU numbering) was changed to L, as this residue is also within 4 Å from the residues L54 and K55 in the CD89 (residue numbering of CD89 as in 10W0).

Finally, the residues I37 (I377, EU numbering) and A38 (A378, EU numbering) were targeted for mutagenesis in both chains, as it has been shown previously that changes at these positions may influence the stability of the CH2 domain in the IgG context. While this mutagenesis in the GA-chain caused an increase in affinity for CD89, it was not beneficial for the CD89 binding when included in the CD89-binding variant of the AG-chain (Figure 7).

To enable efficient expression and avoid multimerization, C-terminal residues C and Y were removed from the coding sequence in all constructs.

In the IgA2 scaffold, the light chains are not covalently linked with the heavy chains, and therefore the interaction of light and heavy chain interaction was stabilized by introducing the P221R mutation for all expressed mutants.

### b) Expression and purification of IgA-SEED variants.

225-IgA1 and SEED-IgA1 variants were expressed at a yield of 60 mg/L after Protein L purification and contained about 72% monomeric species (Figure 2a). This percentage was notably higher for both, IgA1 and SEED-IgA1, than when those were expressed with a full-length C-terminus harboring end C and Y residues (41% and 38%). The SEED-IgA1 constructs were of high thermostability, showing first thermal transition at about 65°C, associated with denaturation of modified CH3 domains, and a second at about 75°C, corresponding to overlapping denaturation of Fab and CH2 domains (Figure 2b). Importantly, mutagenesis required for the introduction of a CD89-binding site did not negatively impact thermostability properties (Figure 7). Consequently, the SEED variant with the highest affinity for CD89 named MUT2724 was chosen for further characterization. In SDS-PAGE analysis, all three proteins exhibited a single main band (Figure 2c). SEED-IgA1 additionally displayed a band at about 75 kDa, which probably corresponds to a single GA-heavy chain and light chain, and another one at 60 kDa, probably corresponding to a single AG-heavy chain, and interestingly these were less present in MUT2724-IgA1, indicating its higher chemical stability.

### c) Glycan analysis.

The glycosylation sites for both IgA1 and IgA2 were labeled with I-IV according to their sequence homology (Figure 3a). In all IgA1-based antibodies, the glycosite containing N263 (Site II) could be analyzed (Figure 3b). Interestingly, the unmodified SEED variants contained a markedly higher proportion of fucosylated glycans (71.24%) than wild-type 225-IgA1 (1.81%) and MUT2724 (6.86%). Glycan composition at the glycosite at N459 (Site IV) was more similar between the SEED and MUT2724, for both GA and AG-chain. While this site was not utilized in about 50% of the AG-chain and in about 40% in the corresponding fragment of the wild-type IgA1, only 15-20% of the GA-chain pertaining fragment were not glycosylated.

### d) Analysis of antigen- and cell-binding properties.

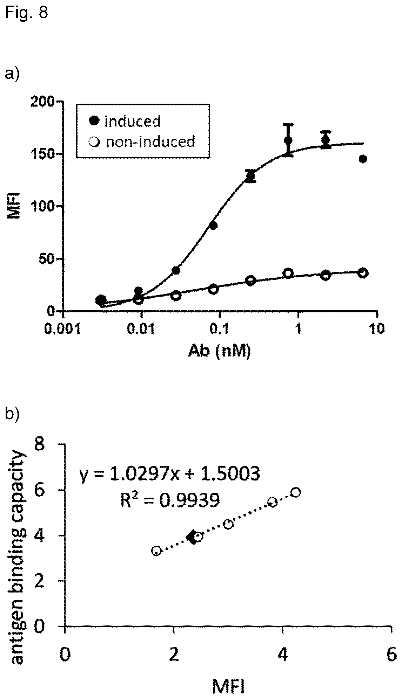

In contrast to SEED-IgA1, MUT2724-IgA1 could bind to recombinant CD89 with 57 nM affinity, in comparison with 27 nM determined for 225-IgA1 (Figure 4a). Importantly, the novel mutant could bind simultaneously to both tumor-associated antigens (Figure 4a). The mutagenesis applied to promote CD89 binding did not impair the binding properties to any of the cognate antigens of bispecific SEED antibody and antigen-positive cell binding remained the same, with an EC50 of about 2 nM for both molecules (Figure 4b). Internalization of MUT2724-IgA1 proceeded faster than of homodimeric 225-IgA1, to 42% vs. 32% after 4-h-incubation and 68% vs. 57% after overnight incubation, while SEED-IgA1 was 31% after 4 hours and 71% after overnight incubation (Figure 4c). For analysis of binding to cell-expressed CD89 antigen, induced HL-60 cells were used. The number of the CD89 copies was determined to be 8000 per cell, which corresponds well to the value reported for neutrophils (Figure 8). While SEED-IgA1 was not reactive with the induced cells, MUT2724 bound to the cell surface as well as the positive control 225-IgA1 (Figure 4d). The interaction was dependent on the CD89 expression as only background reactivity was measured with non-induced HL-60 cells.

### e) Cell-cell interaction assay and neutrophil activation.

The ability of MUT2724-IgA1 to mediate strong intercellular contacts was examined in a cell-cell interaction assay. Upon co-incubation of target and effector cell types, where induced HL-60 cells were used as effector cell type and MDA-MB-468 as target cell type with 20 nM antibody, about 30% of the measured events were complexes of both cell types, in comparison with 35% observed with 225-IgA1 (Figure 5a). The presence of SEED-IgA1 did not change the percentage of associated cells from that formed without any antibody (15% vs. 13%). For further evidence of specificity of this assay, trastuzumab (TRA)-IgA1 was produced. This antibody mediated association of 16% of the cells, which was not above background for the Her2-negative cell line used. None of the antibodies had any effect when HEK293-6E cells were used as a target cell line. The ability to mediate cell-cell association was dose dependent in the concentration range from 0.1-10 nM.

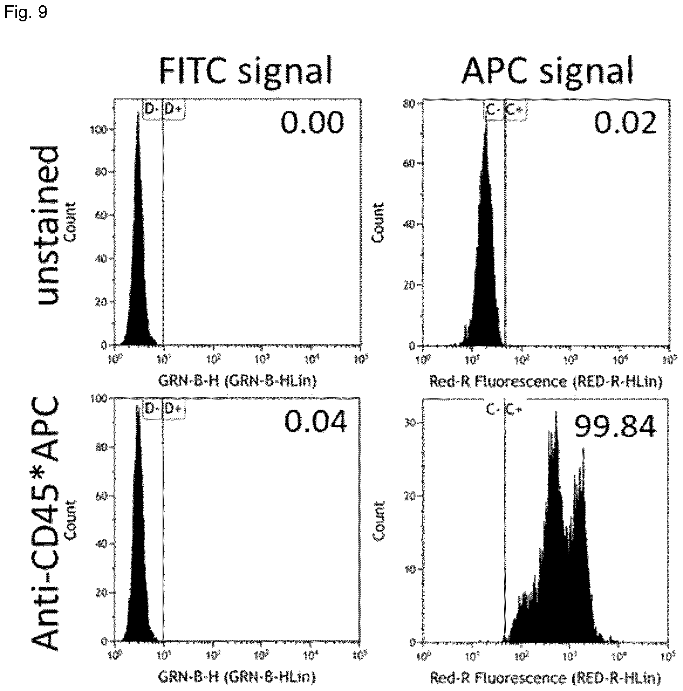

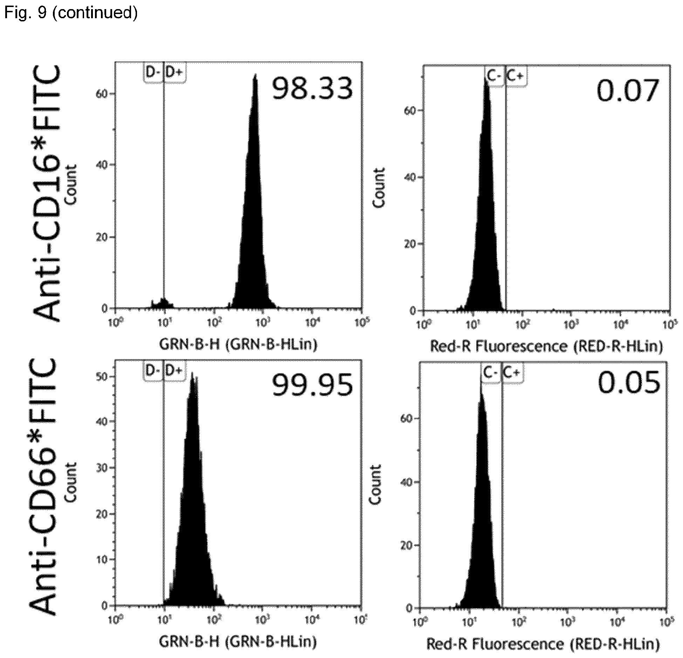

Finally, it was investigated whether the novel mutant can indeed induce neutrophil-mediated killing. Neutrophils were isolated at a high purity (Figure 9). In contrast to SEED-IgA1, which was inactive in this assay, MUT2724-IgA1 killed 21% of the target cells, while 225-IgA1 was more potent with 37% killed cells at 20 nM concentration (Figure 5b). There was no effect when antigen-negative HEK293-6E cells were used as a target cell line. The mere addition of the proteins to the culture medium had no effect on the MDA-MB-468 target cell line (Figure 5c).

### f) Neutrophil-engaging SEED antibody in IgA2 format

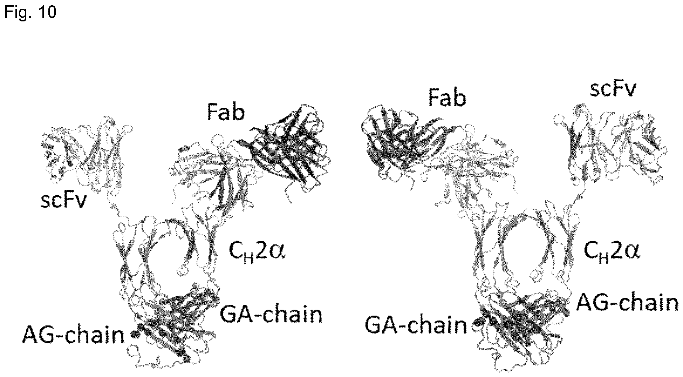

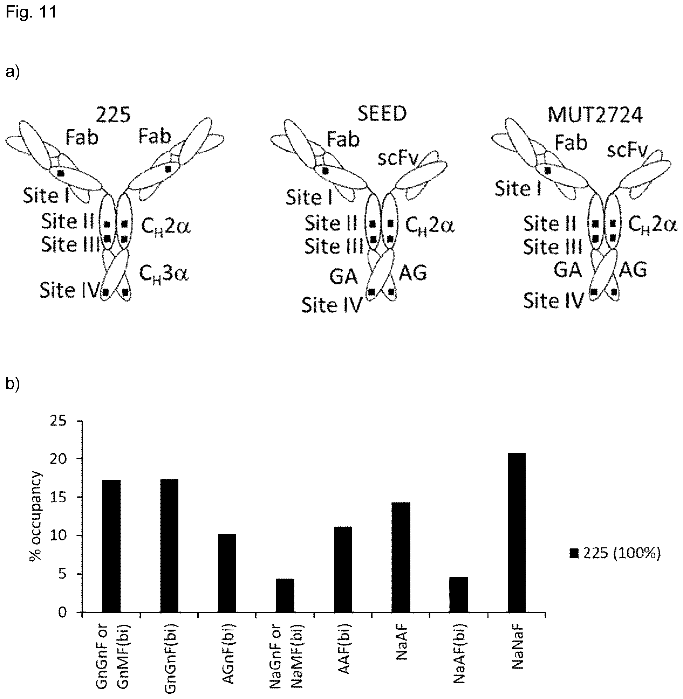

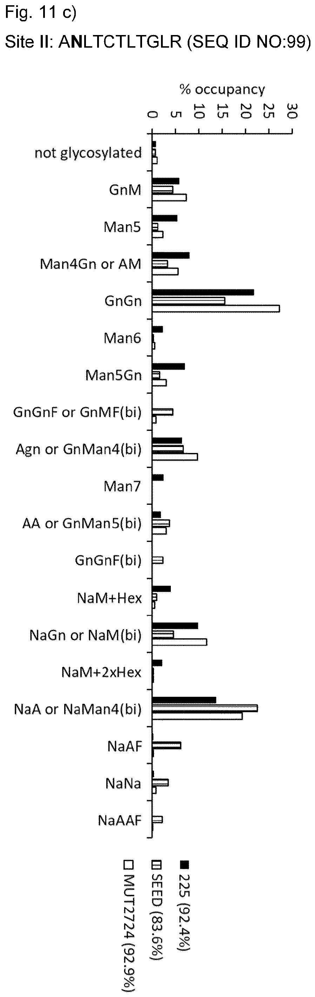

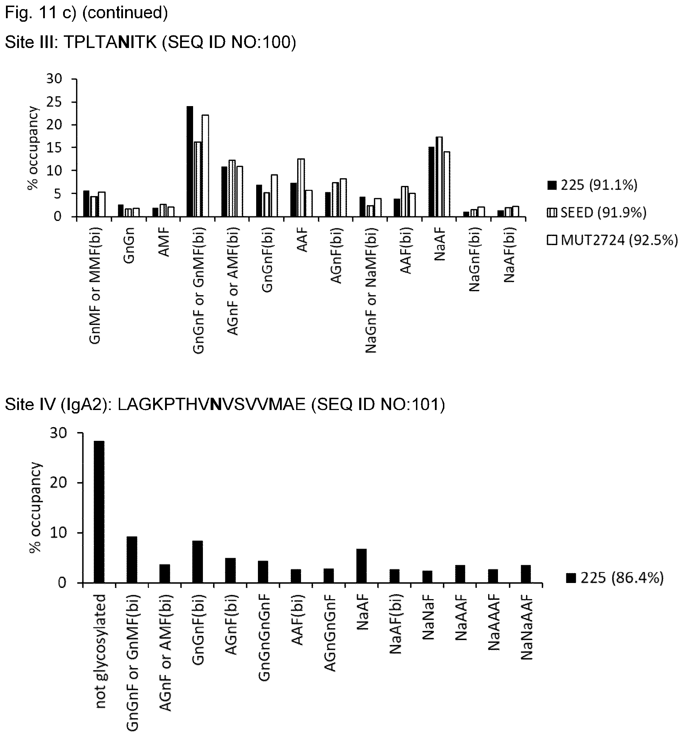

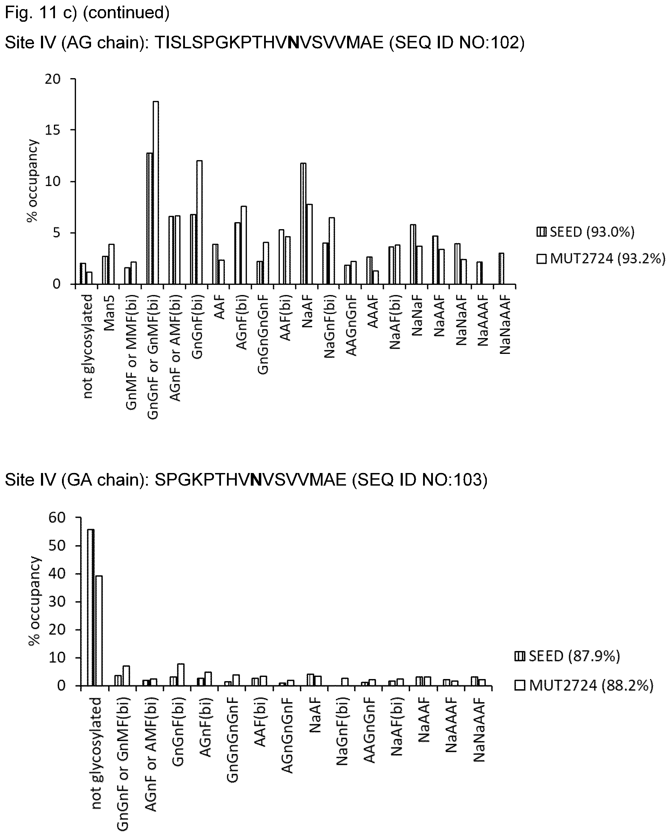

Compared with IgA1, IgA2 format is believed to be better suited for potential clinical development due to certain advantageous features, such as shorter hinge region, which hinders digestion by microbial proteases, and lacks several O-linked glycosylation motifs present in IgA1. The mutations required for CD89 engagement were introduced into a SEED-IgA2m(1) scaffold with the same antigen specificities as described above (Figure 10) and examined the features of the CD89-binding SEED-based mutant (MUT2724-IgA2) also in this molecular context. The antibodies 225-IgA2, SEED-IgA2 and MUT2724-IgA2 could be purified at 60 mg/L supernatant with Protein L-based affinity chromatography, and monomer amounted to 43% for SEED and 84.5% for Mut2724 (Figure 6a). After gel filtration, 17 mg of monomeric antibody per liter supernatant were obtained. The thermostability of the antibodies was high, with deconvolutions measured at 76.3 and 80°C for 225-IgA2, 64.9 and 78.8 °C for SEED, and 65.8 and 78.6°C for MUT2724 (Figure 6a) The produced proteins appeared as one major band in the SDS-PAGE analysis, and again the minor bands of lower molecular weight were less pronounced for MUT2724 than for SEED. As in the IgA1 background, the fucosylation of core N263-glycan (Site II) in SEED was more common than for 225-IgA2 and MUT2724-IgA2 (14.9% vs. 0.32% and 1.04%, respectively) (Figure 11). Site III (including N337) was occupied with similar structures in all three mutants. Site IV was similarly as in the IgA1-based species not utilized in about 50% of the AG-chain, as seen also in the corresponding fragment of the wild-type IgA2, but in contrast only 15-20% of the GA-chain pertaining fragment were not glycosylated.

In an IgA2 scaffold, bispecific MUT2724 could bind to CD89 with an affinity of 48 nM, whereas 31 nM were measured for 225-IgA2 construct (Figure 6b).

Target antigens EGFR and ROR1 were bound with an EC50 of 1.1 nM and 10.0 nM by SEED vs. 1.1 nM and 7.2 nM by MUT2724, and binding to MDA-MB-468 cells proceeded with an EC50 of 4.4 nM for SEED and 2.9 nM for MUT2724 (Figure 6c): interestingly, here the maximal level of binding was lower for MUT2724 than for SEED, which could be attributed to the lower mobility of the Fab arms and the scFv moiety as observed in IgA1 format. In contrast to SEED, MUT2724 could react with the induced HL-60 cell line (Figure 6d) and mediate strong target-effector cell association causing about 23% of the cells to cluster, similarly to 225-IgA2 (Figure 6e), while there was no effect on the control HEK293-6E cell line in this experiment. When the target MDA-MB-468 cells were incubated overnight together with antibodies and neutrophils, MUT2724-IgA2 could kill about 20% of the cells, 225-IgA2 killed 40% and the SEED-IgA2 was not active at all (Figure 6f). None of the mutants had an effect on HEK293-6E cells (Figure 6f) used as a control cell line and there was also no direct toxicity (Figure 6g).

### g) Discussion

Bispecific IgA antibodies were constructed where heterodimerization is achieved via the SEED architecture of CH3 domains, that was modified to enable the interaction with CD89. The molecules were well expressed in HEK293-6E cells in standard laboratory conditions and could be purified using Protein L affinity chromatography followed by preparative SEC. While the original SEED-based constructs were not interacting with CD89, mutagenesis of 9 residues in the SEED GA-chain and 10 residues in AG-chain appreciably delivered molecules that could bind to this important ligand. Resulting antibodies could also mediate formation of target and effector cell clusters and induce neutrophil-mediated specific target cell killing. As such, the proposed format therefore represents an important addition to the spectrum of IgA-based anti-cancer reagents, whose potential was only recognized in the last decades. Only recently, it has been reported that the expression of CD89 is increased in nearby all myeloid cell subsets in tumor-bearing mice transgenic for human CD89, and that this ligand is expressed on tumor-infiltrating myeloid cells, which underlines the promise of CD89-reactive antibodies for cancer immunotherapy.

With the mutagenesis strategy used here, the model anti-EGFR/anti-ROR1 IgA-SEED antibody did not lose the ability to recognize its original antigens. As the CD89 interaction is based on the recognition motifs in the Fc regions, this method can be considered applicable to incorporate antigen specificities of choice. For example, an element blocking immunomodulatory CD47-SIRPα signaling could prove valuable to further augment the activation of myeloid cells to kill target cancer cells. Apart from cancer targeting, IgA antibodies have gained attention as therapeutically meaningful anti-microbial entities and antiviral agents, and here the use of bispecific antibodies could broaden the targeting spectrum by enhancing strain coverage. It is also worth mentioning that for both unmodified SEED-IgA and CD89-engaging MUT2724-IgA with our chosen specificities of variable domains a higher level of internalization was determined than for 225-IgA for both IgA-subclasses. Apart from the lower affinity this could also have an adverse effect on the potency in the killing assays.

In the presented format, surface-exposed amino acid residues were modified in both heterodimer chains, to enable multivalent engagement of CD89 using monomeric IgA-SEED antibodies. The avidity effect derived from complexing of these monomers by J-chain driven multimerization would have been expected to even increase the level of clustering of the ligand on the cell surface.

One important property that could contribute to establishment of IgA-based reagents in therapeutic settings is the extended plasma half-life, which is much lower than 2-3 weeks, characteristic for IgG1,2 and 4-class antibodies owing the recycling mediated by FcRn. An engineered IgA2-based format (IgA 2.0), which in mice exhibits multiple times higher serum levels comparing with unmodified IgA2, has been successfully designed (Lohse et al. 2016, Cancer Res. doi: 10.1158/0008-5472.CAN-15-1232). While IgG-SEED format retains its FcRn binding activity (Davis, et al 2010), this cannot be expected for the CD89-engaging variant where the critical interaction residues H435 in both heterodimer chains have been altered as the interaction sites of CD89 and FcRn receptors with IgG-based molecules are largely overlapping. The pharmacokinetic properties of IgA format have been improved using fusion with an albumin-binding domain, hence exploiting the pH-dependent interaction of albumin with FcRn, which would be a viable strategy also for the newly designed molecules described here.

In IgA2.0 format, altered glycosylation pattern featuring a larger ratio of terminally sialylated N-glycans renders the antibody less likely to be captured and cleared by asialoglycoprotein receptor, which has an additional positive effect on the half-life in vivo. Altered glycosylation of CD89-engaging SEED-IgA1 antibody were also observed, most prominently featuring less fucosylation of N-terminal core than SEED-IgA, especially at the glycosite containing N263, which is proximal to CD89 binding site. This phenomenon is interesting because it reflects the activity of FUT8 enzyme, which is considered to depend on the accessibility and nature of the N-glycan and much less on the underlaying peptide sequence. As no changes have been introduced in the CH2 domain, it appears that solely its conformation, dependent on the status of SEED-CH3 domain mutagenesis, influenced the level of FUT8-mediated fucosylation. Glycosylation at the N263 site itself is considered important for the interaction with CD89 (Gomes et al. 2008, Biochemistry;47:11285-99), and moreover, aberrant N-linked glycosylation of IgA has been implicated in pathological conditions such as IgA nephropathy (Amore et al. 2001, J Am Soc Nephrol12:1862-71).

To sum up, a heterodimeric IgA-SEED based antibody was constructed using rational mutagenesis, which can additionally incite neutrophil-mediated killing and displays a comparable stability to the parental SEED-IgA molecule. Due to the flexibility in choice of antigens targeted with its Fab-arms, this scaffold can be considered a platform for the design of multispecific IgA-based therapeutic molecules.

### Example 2: Materials and Methods.

### a) Expression and purification of IgA-SEED variants

Heavy and light chains constructs were cloned into pTT-based vectors (Canadian National Research Council (CNRC)). Mutations in the SEED domain were introduced by Site Directed Mutagenesis using the QuikChange lightning mutagenesis Kit (Agilent). IgA1, IgA2 and the corresponding IgA-SEED variants and derived mutants were expressed in HEK293-6E cells using heavy to light chain ratio of 1:1 for homodimeric or 2:1:2 (AG-chain: GA-chain: light chain) for heterodimeric antibodies. Expression constructs have been introduced to the cells at a density of 1.5-2×10⁶/mL using PEI-mediated transfection, with mass ratio of DNA:PEI of 1:2. Cells were kept on orbital shaker at 125 rpm in hydrated atmosphere with 5% CO₂ at 37°C and cultured for 5 days with the addition of 0.5% TN-1 on day 2 after transfection. Supernatant was harvested with centrifugation at 2500 g, 15 min at 4°C, buffered with 0.02 M sodium phosphate/150 mM NaCl, pH 7.0, and cleared through a 0.45-µm-filter before the application to Protein L column equilibrated with the same buffer. After loading was completed, the bound protein was eluted with 0.1 M glycine, pH 3.0, and protein containing fractions were neutralized immediately with the addition of 19 µL 2 M Tris, pH 9.0, and dialyzed against at least 100-fold volume of PBS, pH 7.4 at 4°C overnight. Monomeric IgA fraction was separated from oligomers and free light chain on a preparative gel-filtration column Superdex 200 Increase 10/300 GL in PBS/200 mM NaCl, at 0.75 mL/min flow rate, and stored at -80°C.

### b) Biophysical characterization.

### i. Differential Scanning Calorimetry

About 5 µM protein solution, diluted in PBS, was heated in Automated MicroCal PEAQ-DSC system (Malvern Panalytical, Malvern, United Kingdom) from 20°C to 100°C at a rate of 1°C/min, with repeated scans run at the same conditions to obtain the baselines for subtraction from the first scan. Data was fitted with Origin 7.0 for DSC software and the non-two-state transition mechanism was applied for scan deconvolution and determination of midpoint temperatures of transitions (T_{M}s).

### ii. Glycan analysis

The samples were digested in-solution. The proteins were S-alkylated with iodoacetamide and digested with Trypsin and GluC (Promega). The digested samples were loaded on a nanoEase C18 column (nanoEase M/Z HSS T3 Column, 100Å, 1.8 µm, 300 µm × 150 mm, Waters) using 0.1% formic acid as the aqueous solvent. A gradient from 1% B (B: 80% acetonitrile, 0.1% formic acid) to 40% B in 50 min was applied, followed by a 10 min gradient from 40% B to 95% B that facilitates elution of large peptides, at a flow rate of 6 µL/min. Detection was performed with an Orbitrap MS (Exploris 480, Thermo) equipped with the standard H-ESI source in positive ion, DDA mode (switching to MSMS mode for eluting peaks). MS-scans were recorded (range: 350-1200 Da) and the 20 highest peaks were selected for fragmentation. Instrument calibration was performed using Pierce FlexMix Calibration Solution (Thermo Scientific). The possible glycopeptides were identified as sets of peaks consisting of the peptide moiety and the attached N-glycan. The theoretical masses of these glycopeptides were determined with a spreadsheet using the monoisotopic masses for amino acids and monosaccharides.

Manual glycopeptide searches were made using FreeStyle 1.8 (Thermo). For the quantification of the different glycoforms the peak intensities of deconvoluted spectra were compared. All glycoforms present in over 2% were taken into the calculation.

### c) Antigen- and cell-binding properties

### i. ELISA

Biotinylated extracellular domain of ROR1 and Fc-tagged extracellular domain of EGFR (both Sino Biological) were coated to the wells of Streptavidin Immobilizer plate (NUNC) or Maxisorp plate (NUNC) at 5 µg/mL and 10 µg/mL in 100 µL PBS for 1 h at RT. After washing 3 times with 200 µL PBS, wells were blocked with 200 µL 4% BSA-PBS for 1 h at RT. Antibodies were added in three-fold dilution series starting from 100 nM in 2% BSA-PBS and allowed to bind for 1 h at RT. After 3 washing steps with 200 µL PBS, their binding was detected for 30 min with 100 µL anti-human kappa-horseradish peroxidase conjugate (RRID: AB_258334, A-7164, Sigma-Aldrich), diluted 1:5000 in 2% BSA-PBS, and the color reaction was developed with 100 µL 3,3',5,5'-3,3,5,5-tetramethylbenzidin (TMB) (Sigma-Aldrich) and stopped with the addition of 100 µL 30% H2SO4. Absorbance at 450/620 nm was recorded using Tecan Spark reader.

### ii. Biolayer Interferometry

### Affinity to CD89

An Octet 96RED (ForteBio)-based assay was used for determination of binding kinetics. His-tagged extracellular domain (ECD) of CD89 (residues Q22-N227 (NP_001991.1) with C-terminal His6-Tag, expressed in HEK293-6E) was immobilized on HIS1K biosensors (Sartorius) equilibrated in assay buffer (PBS supplemented with Kinetics Buffer (ForteBio, Sartorius) at 10 µg/ml for 5 min. Antibodies diluted in assay buffer in two-fold steps starting at 1 µM were allowed to bind to the immobilized receptor for 10 min at 25°C with shaking at 1000 rpm. Dissociation into assay buffer was recorded for 10 min. Assay background was determined with a sensor immersed in assay buffer only and background binding with antibodies binding to uncoated sensors. After background measurement subtraction, data were fitted with the 2:1 binding model (ForteBio Evaluation Software, version 11.0).

### Simultaneous antigen binding

Streptavidin sensors were loaded with 10 µg/mL of biotinylated ROR1 (Sino Biological) and 100 nM antibody dilutions in assay buffer were allowed to bind for 10 min. Sensors were then immersed into 10 µg/mL solution of EGFR-Fc (Sino Biological) in assay buffer for 5 min, and finally into a solution of 100 µg/mL of His-tagged CD89 ECD in assay buffer for 15 minutes.

### iii. Binding to cell-bound antigen

### Cultivation methods

Streptavidin sensors were loaded with 10 µg/mL of biotinylated ROR1 (Sino Biological)

MDA-MB-468 cells (ATCC HTB-132^{™}) were cultivated in DMEM medium with 10% FCS and penicillin/streptomycin, and passaged twice a week. For detachment, the cells grown in a 75-cm²-T-flask were rinsed twice with 12 mL PBS, treated with 3 mL 0.5% Trypsin/EDTA solution (Sigma-Aldrich) for 5 min at 37°C, and pelleted at 300 g, 5 min at room temperature. They were then resuspended in 5 mL culture medium, counted and either resuspended at the density indicated below in the culture medium for further propagation, labeling, internalization and cell killing experiments, or in 2% BSA-PBS for cell staining and cell-cell interaction assay. HL-60 cells (ATCC CCL-240^{™}) were propagated in RPMI medium with 10% FCS and penicillin/streptomycin and maintained at a density of 1-9×10⁵ cells/mL.

### Cell surface binding assays

### Expression of CD89 on HL-60 cells

The expression of CD89 on the surface of HL-60 cells was induced with 1.3% dimethyl sulfoxide (DMSO) and the peak expression was determined using staining with an anti-CD89 antibody (RRID: AB_447122, ab22520, Abcam) after 5 days. Briefly, 100,000 induced cells were blocked with 100 µL 2% BSA-PBS for 30 min on ice and then incubated with 3-fold dilution series of anti-CD89, starting from 10 nM, in 2% BSA-PBS for 30 min on ice. After centrifugation at 300 g for 5 min at 4°C, binding was detected using anti-mouse-phycoerythrin antibody (RRID: AB_315010, 405307, Bio-Legend), diluted 1: 500 in 2% BSA-PBS, for 30 min on ice. Finally, cells were resuspended in 200 µL ice-cold PBS and 5000 events were analyzed using Guava easyCyte flow cytometer (Luminex). The number of copies of CD89 was determined using QIFlkit (Agilent) exactly according to the manufacturer's instructions, with the same primary anti-CD89 antibody.

### i. The interaction of bispecific antibodies with target cells

Surface target expressing cells were diluted to 10⁶ cells/mL in 2% BSA-PBS for blocking for 30 min on ice. 100-µL-aliquots were distributed into the wells of a 96-U-well plate and pelleted at 300 g for 5 min at 4°C before the incubation with graded concentrations of antibodies, starting with 1000 nM for CD89 interaction on HL-60 cells, and 100 nM for the determination of EC₅₀ for binding to the surface of MDA-MB-468 cells. After 30 minutes, cells were collected by centrifugation at 300 g, 5 minutes, 4 °C and resuspended in 100 µL of Anti-human-kappa-light chain-FITC conjugated antibody (RRID: AB_259557, F-3761, Sigma Aldrich), diluted at 1:100 in 2% BSA/PBS. Cells were incubated for 30 minutes on ice and after a final centrifugation at 300 g, 5 minutes, 4 °C, they were resuspended with ice-cold PBS and analyzed with a Guava easyCyte Flow Cytometer Instrument (Luminex).

### Cell-cell interaction assay

Target MDA-MB-468 and negative control cells (HEK293-6E) were stained with Vybrant^{™} DiD Cell-Labeling Solution and the CD89-expressing cells (HL-60) with Vybrant^{™} Dil Cell-Labeling Solution (both Fisher Scientific), for 20 min at 37 °C. 100,000 cells of each type were mixed and incubated in 100 µL of antibody dilution of graded concentrations, starting with 20 nM, in 2% BSA-PBS in 96-U-well plates for 30 min on ice. After centrifugation at 300 g, 5 min at 4 °C, cells were resuspended in 200 µL ice-cold PBS and analyzed with a Guava easyCyte flow cytometer (Luminex).

### Internalization experiments

Bispecific antibodies were labeled with Alexa Fluor 488 (A10235, Fisher Scientific) following manufacturer's instructions. Target cells were harvested and blocked as described above. 100,000 MDA-MB-468 cells/mL were incubated with 20 nM antibodies at 37°C for 4 h or overnight. After the incubation, the cells were detached at 300 g for 5 min at 4°C, rinsed once with 200 µL ice-cold PBS, and one parallel sample was incubated with 20 µg/mL anti-Alexa Fluor 488 antibody (RRID: AB 221544, A11094, Fisher Scientific), diluted in 2% BSA-PBS for 30 min on ice. After centrifugation, the cells were resuspended in 200 µL ice-cold PBS and analyzed with a Guava easyCyte flow cytometer (Luminex).

### d) Neutrophil activation

### i. Neutrophil isolation

Full blood was drawn into heparin tubes. 15 mL of blood were transferred to 50-ml-conical tube, mixed 10 times by repeated inversion with an equal volume of 3% Dextran and the mixture was allowed to set for 20 min at RT. Leukocyte-rich upper layer was aspirated with a sterile plastic pipette and transferred to a fresh conical tube. Leukocytes were pelleted by centrifugation at 500 g for 10 min at 4 °C with brake off and the supernatant was discarded. Pellets were resuspended in 10 mL PBS and pellets were pooled into a single 50-mL-conical tube to achieve a final volume of 35 mL and the suspension was underlayed with 15 mL Ficoll-Hypaque. After centrifugation at 400 g for 30 min at RT, the polymorphonuclear cells-erythrocyte layer was isolated and resuspended in 10 mL sterile water for 28 s, an equal volume of 1.8% saline was added and cells were pelleted at 500 g for 5 min at RT. Finally, cells were resuspended in 5 mL PBS and their concentration was determined using a hemocytometer. Finally, neutrophils were resuspended in assay media (DMEM medium with 10% FCS and penicillin/streptomycin) at a final density of 8×10⁶/mL.

### ii. Neutrophil characterization

Isolated neutrophils were characterized using staining with anti-CD45, anti-CD16 and anti-CD66b antibodies (Stemcell 60018AZ, 60041FI.1, 60086FI.1). Cells were blocked at a density of 1×10⁶/mL with 10% FBS for 20 min at 4 °C. Aliquots of 100,000 cells were delivered into each well, centrifuged at 300 g, 5 min at 4°C, and incubated with 1 µL antibody, diluted in 100 µL 0.5% FBS for 20 min at 4°C. Cells were centrifuged and resuspended in 200 µL ice-cold PBS and 5000 events were measured with Guava easyCyte (Luminex).

### iii. Cytotoxicity assay

Target MDA-MB-468 cells at a density of 10,000 cells/well were allowed to adhere overnight. Medium was removed and antibodies at 20 nM concentration, as well as the neutrophils at an effector to target ratio of 40:1 were added and incubated overnight in humidified atmosphere at 37°C with 5% CO₂. Plates were then inverted and blotted dry, and 100 µL of complete culture medium containing CellTiter-Blue^{®} Cell Viability Assay Reagent (Promega), diluted 1:5, were added per well. Fluorescence at 560/590 nm was measured after 1.5-h-incubation using Tecan Spark microplate reader (Tecan Instruments) after shaking the plate for 10 s. Quadruplicate samples were measured and for HEK293-6E, used as control cells, triplicate samples were measured. Wells without any cells were considered background, and the cells without any antibody were considered not to experience any specific killing. Complete lysis was assessed in wells where 11 µL Lysis Solution (Promega) were added to the medium 45 min previously to the completion of the incubation. Additional controls, where no neutrophils were added, were performed to exclude the effect of direct toxicity of the compounds on the cells (in 6 parallels). Percent specific killing of target cells was calculated according to the following equation: 100 - ((relative fluorescence units (RFU) of test wells-RFU of background wells) - (RFU of wells without antibody-RFU of background wells)/(RFU of wells without antibody-RFU of background wells)) and background were the wells with medium and substrate only.

### REFERENCES

Borrok, M. J., Luheshi, N. M., Beyaz, N., Davies, G. C., Legg, J. W., Wu, H., ... & Tsui, P. (2015, July). Enhancement of antibody-dependent cell-mediated cytotoxicity by endowing IgG with FcαRI (CD89) binding. In MAbs (Vol. 7, No. 4, pp. 743-751). Taylor & Francis.
Davis, J. H., Aperlo, C., Li, Y., Kurosawa, E., Lan, Y., Lo, K. M., & Huston, J. S. (2010). SEEDbodies: fusion proteins based on strand-exchange engineered domain (SEED) CH3 heterodimers in an Fc analogue platform for asymmetric binders or immunofusions and bispecific antibodies. Protein Engineering, Design & Selection, 23(4), 195-202.
Heinkel, F., Verstraete, M. M., Cao, S., Li, J., Farber, P., Stangle, E., ... & Escobar-Cabrera, E. (2022, December). Engineering a pure and stable heterodimeric IgA for the development of multispecific therapeutics. In Mabs (Vol. 14, No. 1, p. 2141637). Taylor & Francis.
Kelton, W., Mehta, N., Charab, W., Lee, J., Lee, C. H., Kojima, T., ... & Georgiou, G. (2014). IgGA: a "cross-isotype" engineered human Fc antibody domain that displays both IgG-like and IgA-like effector functions. Chemistry & biology, 21(12), 1603-1609.
Kelton, C., Wesolowski, J. S., Soloviev, M., Schweickhardt, R., Fischer, D., Kurosawa, E., ... & Gross, A. W. (2012). Anti-EGFR biparatopic-SEED antibody has enhanced combination-activity in a single molecule. Archives of biochemistry and biophysics, 526(2), 219-225.

## Claims

1. A heterodimeric CH3 assembly of a first and second CH3 domain of the IgG-type, wherein each of the CH3 domains comprises a dimerization sheet, each dimerization sheet being engineered to comprise alternating IgA and IgG segments, wherein the segments of the first CH3 domain are dimerized to the respective segments of the second CH3 domain, wherein either one or both of the CH3 domains is further engineered to incorporate a CD89 binding site.

2. The CH3 assembly of claim 1, wherein the first and second CH3 domains form heterodimers preferentially over forming homodimers.

3. The CH3 assembly of claim 1 or 2, wherein the CH3 domains are of human IgG3, IgG1, IgG2, or IgG4.

4. The CH3 assembly of any one of claims 1 to 3, wherein the alternating IgA and IgG segments originate from corresponding segments of naturally-occurring dimerization sheets of respective IgA and IgG CH3 domains.

5. The CH3 assembly of any one of claims 1 to 4, wherein the first CH3 domain comprises the amino acid sequence of any one of SEQ ID NO: 1 to 24, and the second CH3 domain comprises the amino acid sequence of SEQ ID NO:25 to 36.

6. A heterodimeric Fc comprising dimerized Fc chains of CH2 and CH3 domains, wherein the CH3 domains comprise the CH3 assembly of any one of claims 1 to 5.

7. The Fc of claim 6, wherein the CH2 domains are of the IgA type, preferably IgA2 or IgA1, of the IgG type, preferably IgG3, IgG1, or IgG2, or of the IgE type, preferably wherein the CH2 domains comprise an amino acid sequence independently selected from an amino acid sequence comprising at least 90% sequence identity to any one of SEQ ID NO:43 to 48.

8. The Fc of claim 6 or 7, which comprises a first Fc chain comprising any one of SEQ ID NO:49-53; and a second Fc chain comprising any one of SEQ ID NO:54-56.

9. A binding construct comprising one or more binding moieties and an Fc of any one of claims 6 to 8, preferably comprising at least two binding moieties with different binding specificities.

10. The construct of 9, which comprises at least two binding moieties, wherein a first binding moiety is bound to a one Fc chain, and a second binding moiety is bound to the other Fc chain.

11. The construct of claim 9 or 10, comprising at least one hinge or linker, which links a binding moiety to the N-terminus of an Fc chain, preferably wherein the hinge originates from an IgA or IgG antibody.

12. The construct of any one of claims 9 to 11, wherein the binding moiety comprises an antigen-binding moiety, an enzyme, a substrate, a cytokine, a cytokine-binding moiety, a receptor, or a ligand, preferably wherein the antigen-binding moiety comprises an antibody variable region, which comprises or consists of one or more antibody domains including at least one antibody variable domains, preferably any one or more of a VL domain, a VH domain, a VHH domain, Fv, scFv, diabody, Fab, a scFab, Fab', Fab₂, Fab₃, F(ab')₂; sdAb, diabody, triabody, tetrabody, minibody, nanobody, maxibody, tandab, DVD, BiTe, TandAb, or a combination of any of the foregoing.

13. The construct of any one of claims 9 to 12, which is a multispecific antibody, preferably a bispecific, trispecific, or tetraspecific antibody, or a one-armed antibody.

14. The construct of any one of claims 9 to 13, which has antibody-dependent cytotoxicity (ADCC) using neutrophils as effector cells.

15. Nucleic acid molecules encoding the CH3 assembly of any one of claims 1 to 5, or the Fc of any one of claims 6 to 8, or the construct of any one of claims 9 to 14.
